(19) Europäisches Patentamt
European Patent Office
Office européen des brevets

(11) EP 0 737 670 B1

(12) FASCICULE DE BREVET EUROPEEN

(45) Date de publication et mention de la délivrance du brevet: **21.07.1999 Bulletin 1999/29**

(21) Numéro de dépôt: **96400777.7**

(22) Date de dépôt: **11.04.1996**

(51) Int. Cl.$^6$: **C07C 233/06**, C07C 233/23, C07C 233/60, C07C 233/74, C07C 275/06, C07C 327/42, C07C 335/14, A61K 31/16, A61K 31/17, C07D 209/90, C07D 307/92, C07D 333/74, A61K 31/335, A61K 31/38, A61K 31/395

# (54) Amides tricycliques, leurs procédés de préparation et les compositions pharmaceutiques qui les contiennent

Tricyclische Amidverbindungen, Verfahren zu deren Herstellung und sie enthaltende pharmazeutische Zusammensetzungen

Tricyclic amides, processes for their preparation, and pharmaceutical compositions containing them

(84) Etats contractants désignés:
**AT BE CH DE DK ES FI FR GB GR IE IT LI LU NL PT SE**

(30) Priorité: **14.04.1995 FR 9504503**

(43) Date de publication de la demande:
**16.10.1996 Bulletin 1996/42**

(73) Titulaire: **ADIR ET COMPAGNIE**
**92415 Courbevoie Cédex (FR)**

(72) Inventeurs:
- **Langlois, Michel**
  **92330 Sceaux (FR)**
- **Mathe-Allainmat, Monique**
  **91300 Massy (FR)**
- **Delagrange, Philippe**
  **92130 Issy-Les-Moulineaux (FR)**
- **Renard, Pierre**
  **78000 Versailles (FR)**
- **Guardiola, Béatrice**
  **92210 Saint Cloud (FR)**

(56) Documents cités:
**EP-A- 0 091 328**
**EP-A- 0 153 083**
**EP-A- 0 162 695**
**EP-A- 0 420 064**
**WO-A-87/04153**
**WO-A-95/29173**
**US-A- 3 732 299**

- **HETEROCYCLES, vol. 34, no. 12, 1992, pages 2269-2275, XP000196125 NAKAGAWA K. ET AL.: "A simple four step synthesis and optical resolution of 4-nitro-1, 3, 4, 5 - tetrahydrobenz(cd)indole,..."**
- **JOURNAL OF THE CHEMICAL SOCIETY, vol. 9, 1971, LETCHWORTH GB, pages 1607-1609, XP002005306 C. EVANS ET AL.: "Reactions of 2,3-Dihydro-4-methylphenalen-1-one"**
- **CHIMICA THERAPEUTICA, vol. IV, no. 2, 1969, pages 95-102, XP002005307 ROBERT VIOLLAND ET AL.: "Psychotropes potentiels.."**
- **PATENT ABSTRACTS OF JAPAN vol. 12, no. 421 (C-541), 8 Novembre 1988 & JP-A-63 154660 (KISSEI PHARMACEUT CO LTD), 27 Juin 1988,**

EP 0 737 670 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

L'invention concerne de nouveaux amides tricycliques, leurs procédés de préparation et les compositions pharmaceutiques qui les contiennent.

L'invention décrit de nouveaux amides tricycliques qui s'avèrent être de puissants ligands des récepteurs mélatoninergiques.

Le N-(4-méthyl-2,3-dihydro-1H-phénalèn-2-yl)acétamide est déjà décrit dans la littérature (Waite D. et al. J. Chem. Soc. 1971, (9), pp 1607-1609) mais aucune activité pharmacologique n'est enseignée à son sujet.

Certains dérivés amides tricycliques de type benzo[cd]indole sont connus (EP 153 083 et EP 162 695) mais uniquement comme intermédiaires de synthèse de dérivés amines à vocation sérotoninergique.

De nombreuses études ont mis en évidence ces dix dernières années, le rôle capital de la mélatonine (5-méthoxy N-acétyl tryptamine) dans le contrôle du rythme circadien et des fonctions endocrines, et les récepteurs de la mélatonine ont été caractérisés et localisés.

Outre leur action bénéfique sur les troubles du rythme circadien (J. Neurosurg 1985, 63, pp 321-341) et du sommeil (Psychopharmacology, 1990, 100, pp 222-226), les ligands du système mélatoninergique possèdent d'intéressantes propriétés pharmacologiques sur le système nerveux central, notamment anxiolytiques et antipsychotiques (Neuropharmacology of Pineal Secretions, 1990, 8 (3-4), pp 264-272) et analgésiques (Pharmacopsychiat., 1987, 20, pp 222-223) ainsi que pour le traitement de la maladie de Parkinson (J. Neurosurg 1985, 63, pp 321-341) et d'Alzheimer (Brain Research, 1990, 528, pp 170-174). De même, ces composés ont montré une activité sur certains cancers (Melatonin - clinical Perspectives, Oxford University Press, 1988, page 164-165), sur l'ovulation (Science 1987, 227, pp 714-720) et sur le diabète (Clinical endocrinology, 1986, 24, pp 359-364).

Des composés permettant d'agir sur le système mélatoninergique sont donc pour le clinicien d'excellents médicaments pour le traitement des pathologies liées au système mélatoninergique et notamment celles mentionnées précédemment.

L'invention concerne les composés de formule (I) :

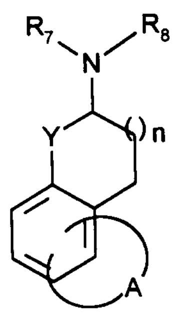

(I)

dans laquelle A forme avec le groupement auquel il est lié un groupement tricyclique choisi parmi ($A_1$) à ($A_4$) :

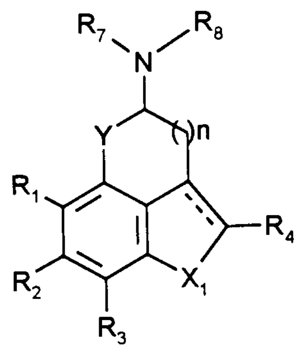

**($A_1$)**

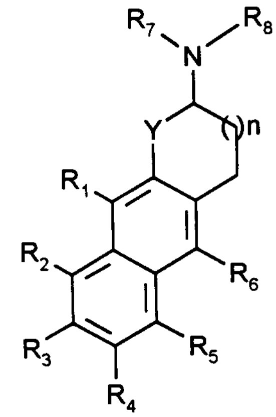

**($A_2$)**

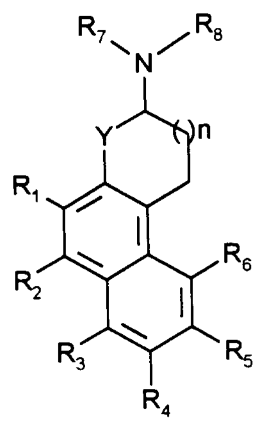

**($A_3$)**

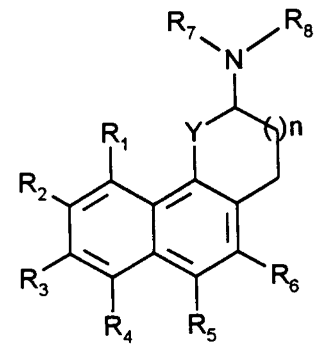

**($A_4$)**

dans lesquels :

- $R_1$, $R_2$, $R_3$, $R_4$, $R_5$ et $R_6$ représentent chacun indépendamment l'un de l'autre un hydrogène ou un radical choisi parmi halogène, hydroxy, Ra et -O-Ra ; avec Ra représentant un radical choisi parmi alkyl, alkyl substitué par un ou plusieurs halogènes, alcényl, alcynyl, cycloalkyl, cycloalkylalkyl, aryl, aryl substitué, arylalkyl, et arylalkyl substitué ;
- $X_1$ représente un groupement choisi parmi soufre, oxygène, $-C(R_5)=C(R_6)-$, $-C(R_5)(R_5')-C(R_6)(R_6')-$, $-C(R_5)(R_5')-C(R_6)=$, $-C(R_5)(R_5')-$ et $-N(R_{14})-$ où $R_5$ et $R_6$ sont tels que définis précédemment et $R_5'$ et $R_6'$ sont choisis parmi les mêmes significations que celles de $R_1$, $R_2$, $R_3$, $R_4$, $R_5$ et $R_6$, telles que définies précédemment, et $R_{14}$ représente un hydrogène ou un radical choisi parmi alkyl, aryl, arylalkyl, arylsubstitué et arylalkylsubstitué,
- la liaison ⋯ signifie que cette liaison peut être simple ou double,
- Y représente un groupement $-C(R_9)(R_{10})-$ dans lequel $R_9$ et $R_{10}$ représentent, chacun indépendamment l'un de l'autre un hydrogène, un alkyl ou un alkoxy,
- n représente un nombre entier de 1 à 3 ;
- $R_7$ représente un hydrogène ou un radical $R_7'$ choisi parmi alkyl, aryl, arylalkyl, aryl substitué et arylalkyl substitué
- et $R_8$ représente :

  . un groupement de formule $-CO-R_{11}$ dans laquelle $R_{11}$ représente un radical $R_{12}$ avec $R_{12}$ représentant un hydrogène ou un radical choisi parmi alkyle, alkyle substitué par un ou plusieurs halogènes, alcényle, alcynyle, cycloalkyl, cycloalkylalkyl, aryl, aryl substitué, arylalkyl, arylalkyl substitué, ou $R_{11}$ représente un radical $-NH-R_{13}$, avec $R_{13}$ représentant un atome d'hydrogène ou un radical choisi parmi alkyl, alkyl substitué par un ou plusieurs halogènes, alcényl, alcynyl, cycloalkyl, cycloalkylalkyl, aryl, aryl substitué, arylalkyl et arylalkyl substitué ;

. ou un groupement de formule $-CS-R_{11}$ dans lequel $R_{11}$ est tel que défini précédemment

avec la réserve que le composé de formule (I) ne peut représenter le N-(4-méthyl-1H-2,3-dihydro-phénalèn-2-yl)acétamide,
et que si $X_1$ représente un groupement -NH- ou $-N(CH_3)-$ alors $R_1$ ne peut pas représenter un hydrogène, un halogène ou un groupement méthoxy,

leurs énantiomères et diastéréoisomères
et leurs sels d'addition à une base pharmaceutiquement acceptable,
étant entendu que :

- les termes "alkyl" et "alkoxy" désignent des radicaux linéaires ou ramifiés de 1 à 6 atomes de carbone,
- les termes "alcényl" et "alcynyl" représentent des radicaux insaturés, linéaires ou ramifiés, de 2 à 6 atomes de carbone,
- le terme "cycloalkyl" désigne un groupement cyclique de 3 à 8 atomes de carbone,
- le terme "aryl" désigne un naphtyl, un phényl ou un pyridyl,
- l'expression "substitué" associée aux termes "aryl" et "arylalkyl" signifie que ces groupements sont substitués par un ou plusieurs radicaux choisis parmi halogène, alkyl, alkyl substitué par un ou plusieurs halogènes, alkoxy et hydroxy.

L'invention concerne particulièrement les composés de formule (I) dans laquelle, pris séparément ou ensemble le cas échéant,

- A représente un groupement $A_1$,
- A représente un groupement $A_2$,
- A représente un groupement $A_3$,
- A représente un groupement $A_4$,
- $X_1$ représente un soufre,
- $X_1$ représente un oxygène,
- $X_1$ représente un groupement $-N(R_{14})-$,
- $X_1$ représente un groupement $-C(R_5)=C(R_6)-$
- $X_1$ représente un groupement $-C(R_5)(R_5')-C(R_6)(R_6')-$
- $R_1$, $R_2$, $R_3$, $R_4$, et $R_5$, $R_5'$, $R_6$ et $R_6'$ le cas échéant, représentent simultanément des hydrogènes,
- un des radicaux $R_1$, $R_2$, $R_3$, $R_4$, et $R_5$, $R_5'$, $R_6$ et $R_6'$ le cas échéant, est un radical choisi parmi halogène, alkyle et alkoxy, par exemple alkoxy et les autres représentent des hydrogènes,
- Y représente un groupement méthylène,
- n est égal à 1,
- $R_7$ représente un hydrogène,
- $R_7$ représente un alkyl,
- $R_8$ représente un groupement $-CO-R_{11}$,
- $R_8$ représente un groupement $-CS-R_{11}$,
- $R_{11}$ représente un alkyl,
- $R_{11}$ représente un cycloalkyle,
- $R_{11}$ représente un groupement $-NH-R_{13}$,
- $R_{13}$ représente un alkyl,
- et $R_{13}$ représente un cycloalkyl.

Par exemple, l'invention concerne les composés particuliers de formule (I) correspondants aux formules (1) à (21) :

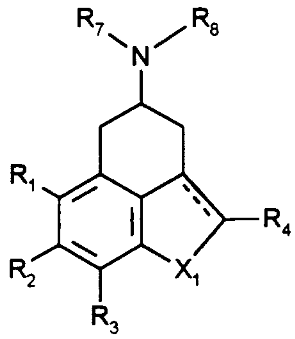

(1)

,

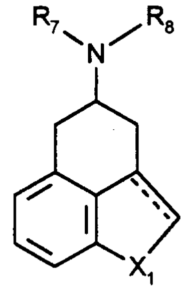

(2)

,

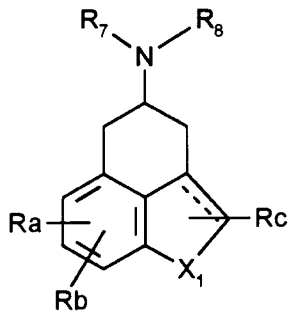

(3)

,

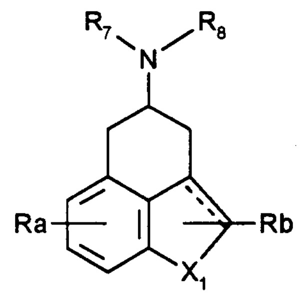

(4)

,

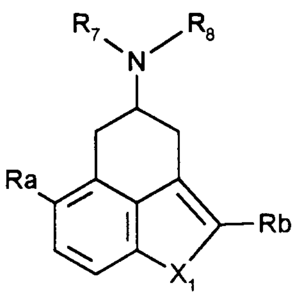

(5)

,

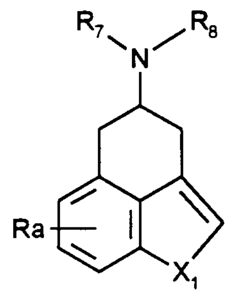

(6)

,

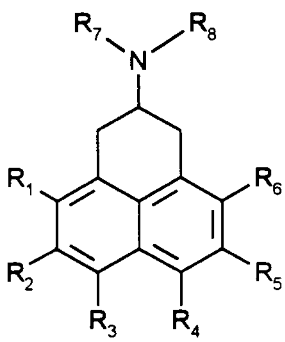

(7)

,

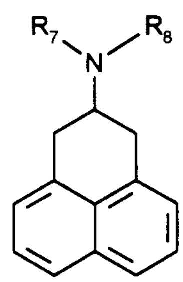

(8)

,

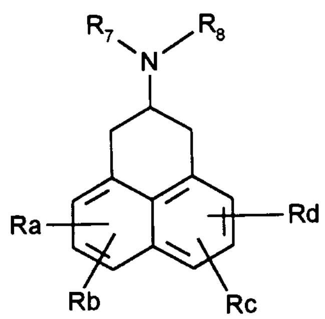

,

(9)

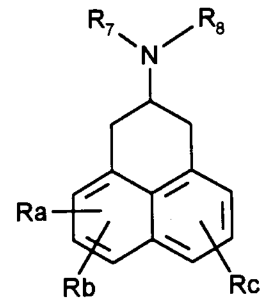

,

(10)

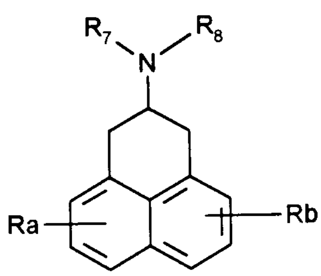

,

(11)

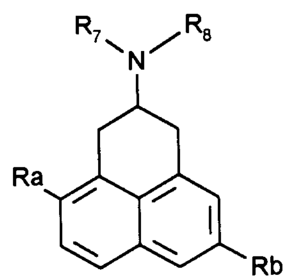

,

(12)

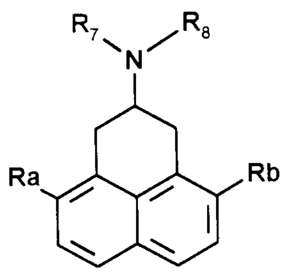

(13)

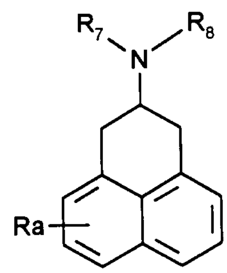

(14)

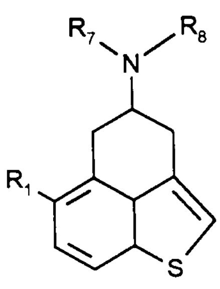

,

(15)

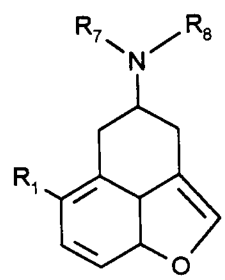

,

(16)

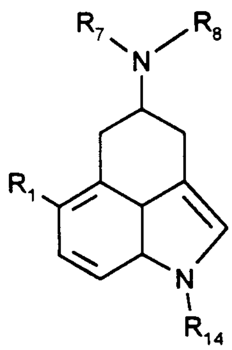

,

(17)

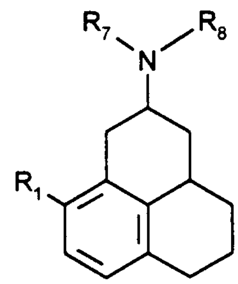

,

(18)

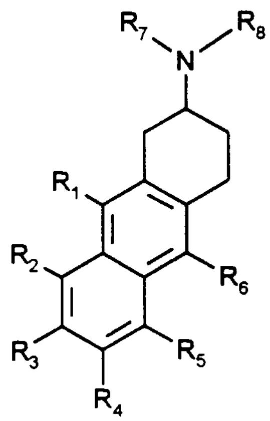

,

(19)

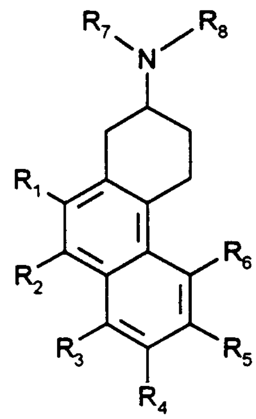

,

(20)

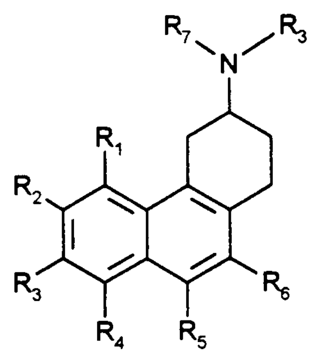

(21)

dans lesquelles $X_1$, $R_1$, $R_2$, $R_3$, $R_4$, $R_5$, $R_6$, $R_7$ et $R_8$ sont tels que définis dans la formule (I) et Ra, Rb, Rc et Rd peuvent être choisis parmi les mêmes valeurs que celles définies pour $R_1$, $R_2$, $R_3$, $R_4$, $R_5$, et $R_6$ à l'exception de l'hydrogène.

Par exemple Ra, Rb, Rc et Rd sont choisis parmi halogène, alkyl et alkoxy.

Par exemple, $X_1$ représente un groupement choisi parmi soufre, oxygène, $-C(R_5)=C(R_6)-$, $-C(R_5)(R_5')-C(R_6)(R_6')-$, $-C(R_5)(R_5')-C(R_6)=$ et $-C(R_5)(R_5')-$ dans lesquels $R_5$, $R_5'$, $R_6$ et $R_6'$ sont tels que définis précédemment.

De façon particulière, les radicaux alkyls présents dans la formule (I) peuvent être choisis parmi méthyl, éthyl, n-propyl, isopropyl, n-butyl, isobutyl, sec-butyl, tert-butyl, pentyl, et hexyl ainsi que les isomères de squelette des radicaux pentyl et hexyl.

Les radicaux alkoxy présents dans la formule (I) peuvent être choisis parmi méthoxy, éthoxy, n-propoxy, isopropoxy, n-butoxy, iso-butoxy, sec-butoxy, tert-butoxy, pentyloxy, et hexyloxy.

Les halogènes présents dans la formule (I) peuvent être choisis parmi le brome, le chlore, le fluor, et l'iode.

Les cycloalkyles présents dans la formule (I) peuvent être choisis parmi cyclopropyl, cyclobutyl, cyclopentyl, cyclohexyl, cycloheptyl et cyclooctyl.

Les alcényls présents dans la formule (I) peuvent être choisis parmi vinyl, allyl, propènyl, butènyl, pentènyl et hexènyl,

et les alcynyls présents dans la formule (I) peuvent être choisis parmi éthynyl, propyn-1-yl et propyn-2-yl, butynyle, pentynyl et hexynyl,
ainsi que les différents isomères de ces radicaux selon la position de la double ou triple liaison.

Parmi les bases pharmaceutiquement acceptables que l'on peut utiliser pour former un sel d'addition avec les composés de l'invention, on peut citer à titre d'exemples et de façon non limitative, les hydroxydes de sodium, de potassium, de calcium, ou d'aluminium, les carbonates de métaux alcalins ou alcalinoterreux, et les bases organiques comme la triéthylamine, la benzylamine, la diéthanolamine, la tert-butylamine, la dicyclohexylamine, et l'arginine.

L'invention concerne également le procédé de préparation des composés de formule ($A_1$) telle que définie dans la formule (I) caractérisé en ce que l'on procède à la cyclisation d'un composé de formule (II) :

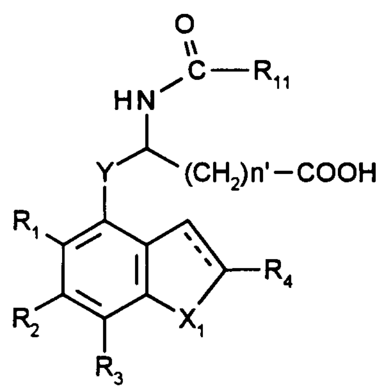

**(II)**

dans laquelle $R_1$, $R_2$, $R_3$, $R_4$, $R_{11}$, $X_1$ et Y sont tels que définis dans la formule (I), et n' représente 0, 1 ou 2, pour obtenir un composé de formule (III) :

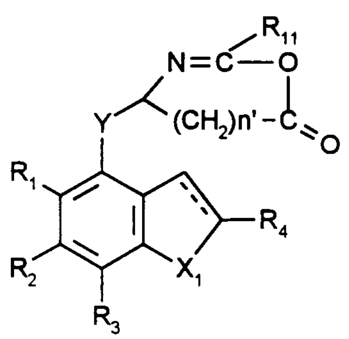

**(III)**

dans laquelle $R_1$, $R_2$, $R_3$, $R_4$, $R_{11}$, n', $X_1$ et Y sont tels que définis précédemment, qui est ensuite mis en réaction avec un acide de Lewis pour obtenir un composé de formule (IV) :

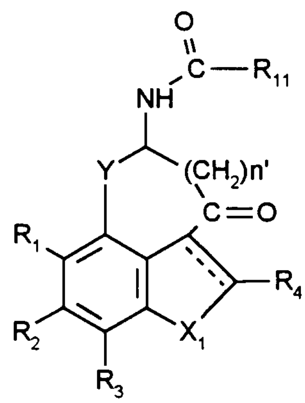

(IV)

dans laquelle $R_1$, $R_2$, $R_3$, $R_4$, $R_{11}$, n', $X_1$ et Y sont tels que définis précédemment, qui est alors réduit pour obtenir le composé de formule (I/a) :

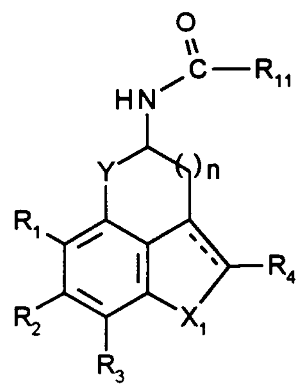

(I/a)

dans laquelle $R_1$, $R_2$, $R_3$, $R_4$, $R_{11}$, n, $X_1$ et Y sont tels que définis précédemment et n est tel que défini dans la formule (I),
composé de formule (I/a) qui est soumis à un agent de thionation, tel que le réactif de Lawesson, pour obtenir le composé de formule (I/b) :

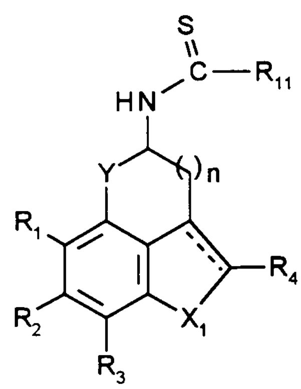

(I/b)

dans laquelle $R_1$, $R_2$, $R_3$, $R_4$, $R_{11}$, n, $X_1$ et Y sont tels que définis précédemment,
le composé de formule (I/a) ou (I/b) pouvant être mis en réaction avec un composé de formule (V) :

$$R_7'—Hal \quad (V)$$

dans laquelle $R_7'$ est tel que défini dans la formule (I) et Hal représente un atome d'halogène,

pour obtenir le composé correspondant de formule (I/c) :

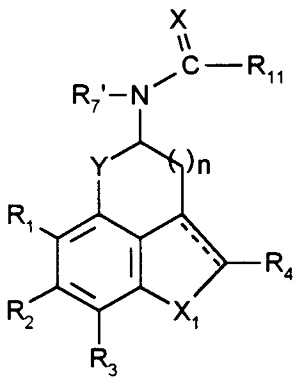

**(I/c)**

dans laquelle $R_1$, $R_2$, $R_3$, $R_4$, $R_{11}$, $R_7'$, n, $X_1$ et Y sont tels que définis précédemment, et X représente un oxygène ou un soufre,
les composés de formule (I) pouvant être :

- purifiés suivant une ou plusieurs méthodes de purification choisies parmi la cristallisation, la chromatographie, l'extraction, la filtration, et le passage sur charbon ou résine,
- séparés, sous forme pure ou sous forme de mélange, en leurs éventuels énantiomères ou diastéréoisomères,
- et salifiés par une base pharmaceutiquement acceptable.

L'invention concerne ainsi le procédé de préparation des composés de formule (I-1) :

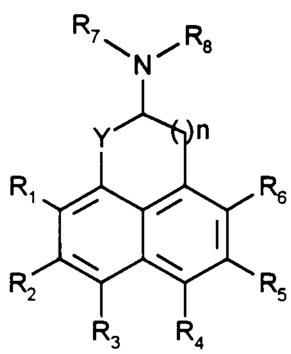

**(I-1)**

dans laquelle $R_1$, $R_2$, $R_3$, $R_4$, $R_5$, $R_6$, $R_7$, $R_8$, n et Y sont tels que définis dans la formule (I)
caractérisé en ce que on procède à la cyclisation d'un composé de formule (II-1) :

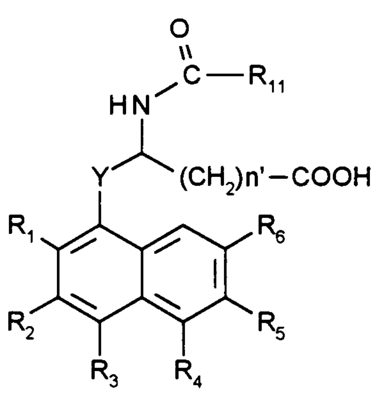

**(II-1)**

dans laquelle $R_1$, $R_2$, $R_3$, $R_4$, $R_5$, $R_6$, $R_{11}$ et Y sont tels que définis dans la formule (I), et n' représente 0, 1 ou 2, pour obtenir un composé de formule (III-1) :

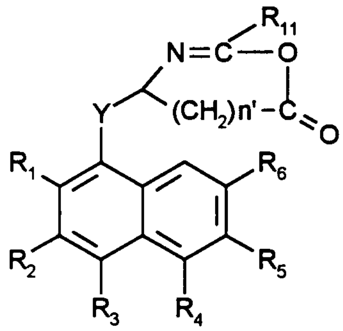

**(III-1)**

dans laquelle $R_1$, $R_2$, $R_3$, $R_4$, $R_5$, $R_6$, $R_{11}$, n' et Y sont tels que définis précédemment,
qui est ensuite mis en réaction avec un acide de Lewis pour obtenir un composé de formule (IV-1):

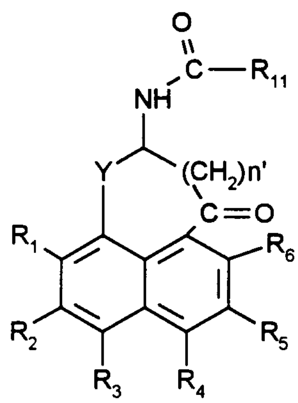

**(IV-1)**

dans laquelle $R_1$, $R_2$, $R_3$, $R_4$, $R_5$, $R_6$, $R_{11}$, n' et Y sont tels que définis précédemment,
qui est alors réduit pour obtenir le composé de formule (I/a-1) :

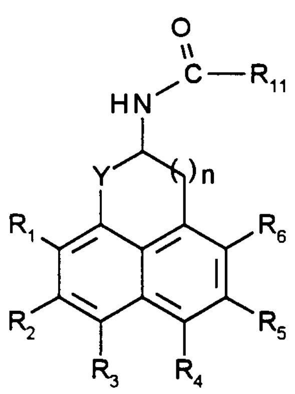

**(I/a-1)**

dans laquelle $R_1$, $R_2$, $R_3$, $R_4$, $R_5$, $R_6$, $R_{11}$, n et Y sont tels que définis précédemment et n est tel que défini dans la formule (I),
composé de formule (I/a-1) qui est soumis à un agent de thionation, tel que le réactif de Lawesson, pour obtenir le composé de formule (I/b-1) :

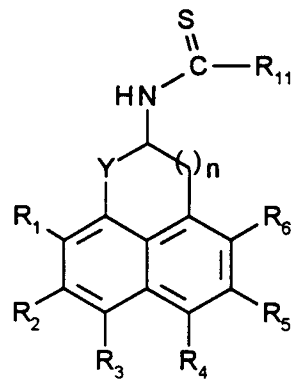

(I/b-1)

dans laquelle $R_1$, $R_2$, $R_3$, $R_4$, $R_5$, $R_6$, $R_{11}$, n et Y sont tels que définis précédemment, le composé de formule (I/a) ou (I/b) pouvant être mis en réaction avec un composé de formule (V) :

$$R_7'—Hal \qquad (V)$$

dans laquelle $R_7'$ est tel que défini dans la formule (I) et Hal représente un atome d'halogène, pour obtenir le composé correspondant de formule (I/c-1) :

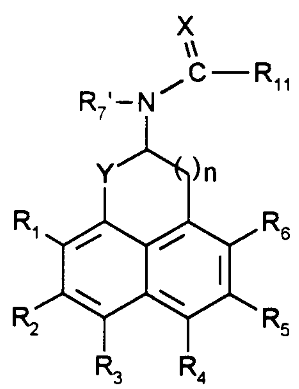

(I/c-1)

dans laquelle $R_1$, $R_2$, $R_3$, $R_4$, $R_5$, $R_6$, $R_{11}$, $R_7'$, n et Y sont tels que définis précédemment, et X représente un oxygène ou un soufre,
les composés de formule (I/a-1), (I/b-1), (I/c-1) formant l'ensemble des composés de formule (I-1),
les composés de formule (I-1) pouvant être :

- purifiés suivant une ou plusieurs méthodes de purification choisies parmi la cristallisation, la chromatographie, l'extraction, la filtration, et le passage sur charbon ou résine,
- séparés, sous forme pure ou sous forme de mélange, en leurs éventuels énantiomères ou diastéréoisomères,
- et salifiés par une base pharmaceutiquement acceptable.

Par exemple, l'invention concerne le procédé de préparation des composés de formule (I/d) :

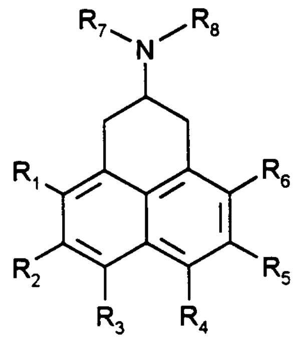

(I/d)

dans laquelle $R_1$, $R_2$, $R_3$, $R_4$, $R_5$, $R_6$, $R_7$ et $R_8$ sont tels que définis dans la formule (I), caractérisé en ce que on procéde à la cyclisation d'un composé de formule (II/d) :

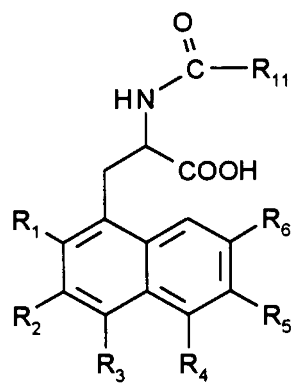

(II/d)

dans laquelle $R_1$, $R_2$, $R_3$, $R_4$, $R_5$, et $R_6$ sont tels que définis précédemment et $R_{11}$ est tel que défini dans la formule (I), pour obtenir un composé de formule (III/d) :

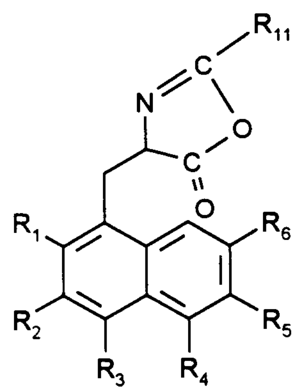

(III/d)

dans laquelle $R_1$, $R_2$, $R_3$, $R_4$, $R_5$, $R_6$, et $R_{11}$ sont tels que définis précédemment,
qui est ensuite mis en réaction avec un acide de Lewis pour obtenir un composé de formule (IV/d) :

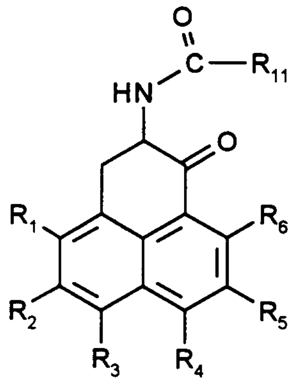

**(IV/d)**

dans laquelle $R_1$, $R_2$, $R_3$, $R_4$, $R_5$, $R_6$ et $R_{11}$ sont tels que définis précédemment, qui est alors réduit pour obtenir le composé de formule (I/d1) :

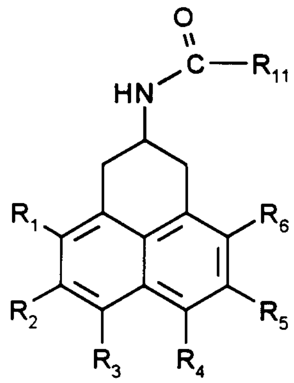

**(I/d1)**

dans laquelle $R_1$, $R_2$, $R_3$, $R_4$, $R_5$, $R_6$ et $R_{11}$ sont tels que définis précédemment, composé de formule (I/d1) qui est soumis à un réactif de thionation, tel que le réactif de Lawesson, pour obtenir un composé de formule (I/d2) :

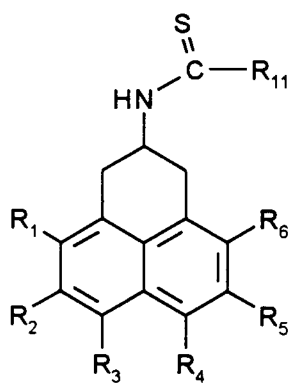

**(I/d2)**

dans laquelle $R_1$, $R_2$, $R_3$, $R_4$, $R_5$, $R_6$ et $R_{11}$ sont tels que définis précédemment, les composés de formules (I/d1) et (I/d2) pouvant être mis en réaction avec un composé de formule (V) :

$$R_7'\text{—Hal} \qquad (V)$$

dans laquelle $R_7'$ est tel que défini dans la formule (I) et Hal représente un atome d'halogène, pour obtenir un composé de formule (I/d3) :

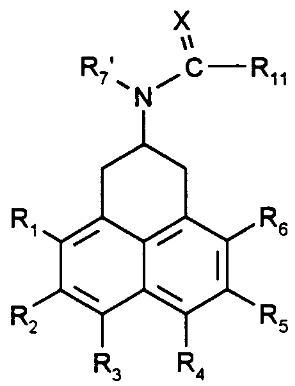

(I/d3)

dans laquelle $R_2$, $R_2$, $R_3$, $R_4$, $R_5$, $R_6$ $R_{11}$ et $R_7'$ sont tels que définis précédemment et X représente un oxygène ou un soufre,
les composés de formules (I/d1), (I/d2) et (I/d3) formant l'ensemble des composés de formule (I/d),
les composés de formule (I/d) pouvant être :

- purifiés suivant une ou plusieurs méthodes de purification choisies parmi la cristallisation, la chromatographie, l'extraction, la filtration, et le passage sur charbon ou résine,
- séparés, sous forme pure ou sous forme de mélange, en leurs éventuels énantiomères ou diastéréoisomères,
- et salifiés par une base pharmaceutiquement acceptable.

L'invention concerne également le procédé de préparation des composés de formule (I) caractérisé en ce que on fait réagir un composé de formule (g-VI) :

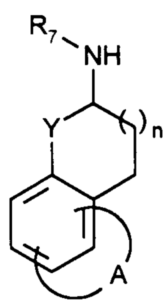

(g-VI)

dans laquelle A, $R_7$, n et Y sont tels que définis dans la formule (I),

- soit avec un composé de formule (VII) ou (VIIa) :

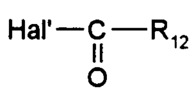

(VII)

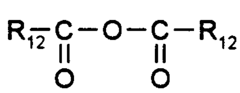

(VIIa)

dans laquelle $R_{12}$ est tel que défini dans la formule (I), l'anhydride de formule (VIIa) étant mixte ou symétrique, et Hal'

représente un halogène,
pour obtenir un composé de formule (g-I/e) :

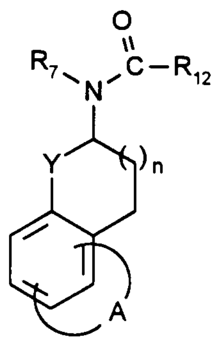

(g-I/e)

dans laquelle A, $R_7$, $R_{12}$, n et Y sont tels que définis précédemment,
qui est ensuite soumis à un réactif de thionation tel que le réactif de Lawesson pour obtenir un composé de formule (g-I/e') :

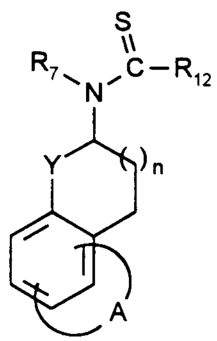

(g-I/e')

dans laquelle A, $R_7$, $R_{12}$, Y et n sont tels que définis précédemment,

- soit avec un composé de formule (VIII) :

$$X{=}C{=}N{-}R_{13} \qquad \text{(VIII)}$$

dans laquelle $R_{13}$ est tel que défini dans la formule (I) et X représente un oxygène ou un soufre,
pour obtenir un composé de formule (g-I/f) :

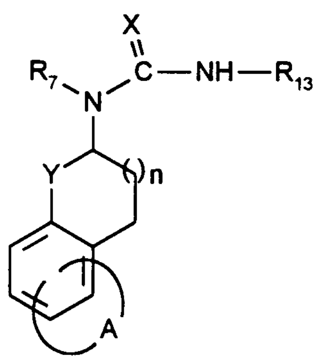

(g-I/f)

dans laquelle A, $R_7$, $R_{13}$, n, X et Y sont tels que définis précédemment,
les composés de formule (g-I/e), (g-I/e') et (g-I/f) formant l'ensemble des composés de formule (I),
les composés de formule (I) ainsi obtenus pouvant être :

- purifiés suivant une ou plusieurs méthodes de purification choisies parmi la cristallisation, la chromatographie, l'extraction, la filtration, et le passage sur charbon ou résine,
- séparés sous forme pure ou sous forme de mélange, en leurs éventuels énantiomères ou diastéréoisomères,
- et salifiés par une base pharmaceutiquement acceptable.

L'invention concerne en particulier le procédé de préparation des composés de formule (A/1) caractérisé en ce que on fait réagir un composé de formule (VI) :

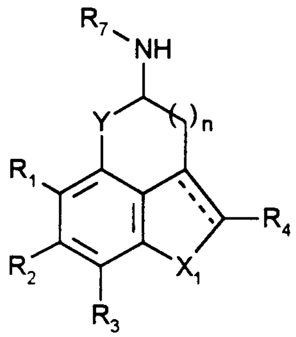

**(VI)**

dans laquelle $R_1$, $R_2$, $R_3$, $R_4$, $R_7$, n, $X_1$ et Y sont tels que définis dans la formule (I),

- soit avec un composé de formule (VII) ou (VIIa) :

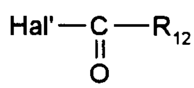

**(VII)**

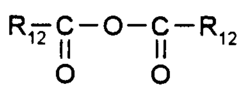

**(VIIa)**

dans laquelle $R_{12}$ est tel que défini dans la formule (I), l'anhydride de formule (VIIa) étant mixte ou symétrique, et Hal' représente un halogène,
pour obtenir un composé de formule (I/e) :

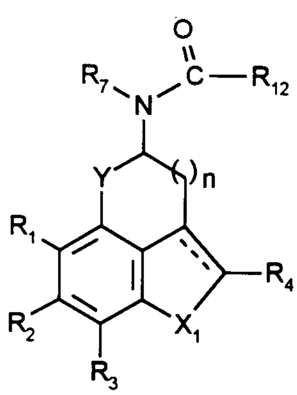

**(I/e)**

dans laquelle $R_1$, $R_2$, $R_3$, $R_4$, $R_7$, $R_{12}$, n, $X_1$ et Y sont tels que définis précédemment,
qui est ensuite soumis à un réactif de thionation, tel que le réactif de Lawesson, pour obtenir un composé de formule (I/e') :

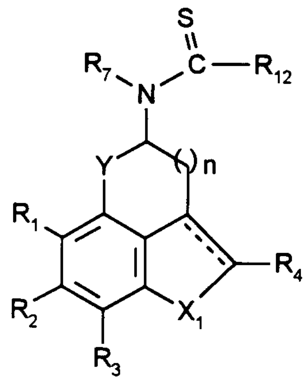

**(I/e')**

dans laquelle $R_1$, $R_2$, $R_3$, $R_4$, $R_7$, $R_{12}$, $X_1$, Y et n sont tels que définis précédemment,

- soit avec un composé de formule (VIII) :

$$X{=}C{=}N{-}R_{13} \quad \text{(VIII)}$$

dans laquelle $R_{13}$ est tel que défini dans la formule (I) et X représente un oxygène ou un soufre, pour obtenir un composé de formule (I/f) :

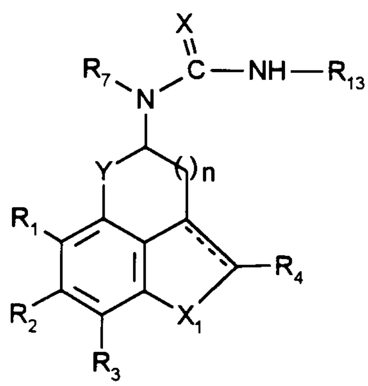

**(I/f)**

dans laquelle $R_1$, $R_2$, $R_3$, $R_4$, $R_7$, $R_{13}$, n, $X_1$, X et Y sont tels que définis précédemment, les composés de formule (I) ainsi obtenus pouvant être :

- purifiés suivant une ou plusieurs méthodes de purification choisies parmi la cristallisation, la chromatographie, l'extraction, la filtration, et le passage sur charbon ou résine,
- séparés, sous forme pure ou sous forme de mélange, en leurs éventuels énantiomères ou diastéréoisomères,
- et salifiés par une base pharmaceutiquement acceptable.

L'invention concerne ainsi le procédé de préparation des composés de formule (I-1) telle que définie précédemment caractérisé en ce que on fait réagir un composé de formule (VI-1) :

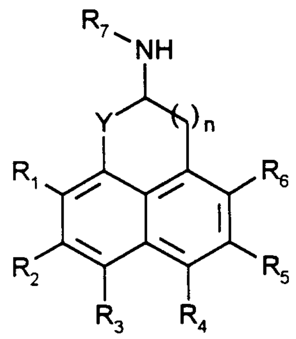

**(VI-1)**

dans laquelle $R_1$, $R_2$, $R_3$, $R_4$, $R_5$, $R_6$, $R_7$ , n et Y sont tels que définis dans la formule (I),

- soit avec un composé de formule (VII) ou (VIIa) :

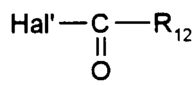

**(VII)**

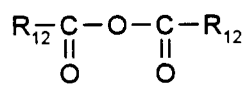

**(VIIa)**

dans laquelle $R_{12}$ est tel que défini dans la formule (I), l'anhydride de formule (VIIa) étant mixte ou symétrique, et Hal' représente un halogène,
pour obtenir un composé de formule (I/e-1) :

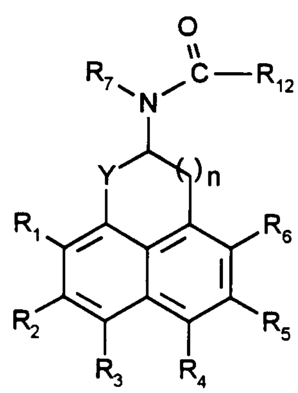

**(I/e-1)**

dans laquelle $R_1$, $R_2$, $R_3$, $R_4$, $R_5$, $R_6$, $R_7$, $R_{12}$, n et Y sont tels que définis précédemment,
qui est ensuite soumis à un réactif de thionation, tel que le réactif de Lawesson, pour obtenir un composé de formule (I/e'-1) :

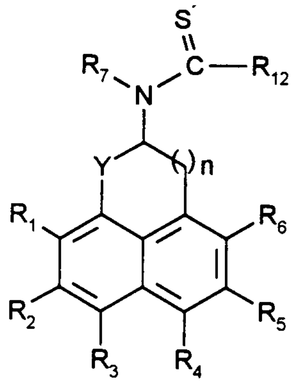

(I/e'-1)

dans laquelle $R_1$, $R_2$, $R_3$, $R_4$, $R_5$, $R_6$, $R_7$, $R_{12}$, Y et n sont tels que définis précédemment,

- soit avec un composé de formule (VIII) :

$$X{=}C{=}N{-}R_{13} \qquad (VIII)$$

dans laquelle $R_{13}$ est tel que défini dans la formule (I) et X représente un oxygène ou un soufre, pour obtenir un composé de formule (I/f-1) :

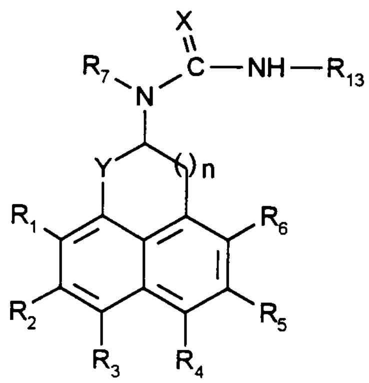

(I/f-1)

dans laquelle $R_1$, $R_2$, $R_3$, $R_4$, $R_5$, $R_6$, $R_7$, $R_{13}$, n , X et Y sont tels que définis précédemment, les composés de formules (I/e-1), (I/e'-1) et (I/f-1) formant l'ensemble des composés de formule (I-1), les composés de formule (I-1) ainsi obtenus pouvant être :

- purifiés suivant une ou plusieurs méthodes de purification choisies parmi la cristallisation, la chromatographie, l'extraction, la filtration, et le passage sur charbon ou résine,
- séparés, sous forme pure ou sous forme de mélange, en leurs éventuels énantiomères ou diastéréoisomères,
- et salifiés par une base pharmaceutiquement acceptable.

Par exemple, l'invention concerne le procédé de préparation des composés de formule (I/d) :

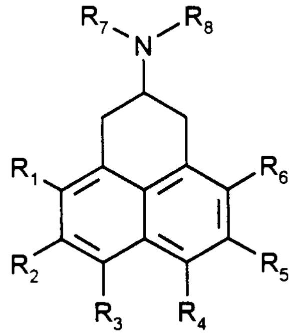

(I/d)

dans laquelle $R_1$, $R_2$, $R_3$, $R_4$, $R_5$, $R_6$, $R_7$, et $R_8$ sont tels que définis dans la formule (I) caractérisé en ce que
on fait réagir un composé de formule (VI/d) :

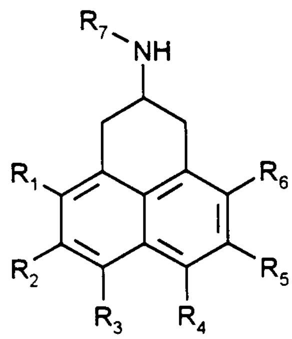

(VI/d)

dans laquelle $R_1$, $R_2$, $R_3$, $R_4$, $R_5$, $R_6$, et $R_7$, sont tels que définis précédemment

- soit avec un composé de formule (VII) ou (VIIa) :

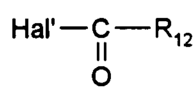

(VII)

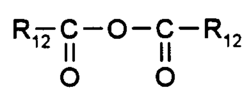

(VIIa)

dans laquelle $R_{12}$ est tel que défini dans la formule (I) l'anhydride de formule (VIIa) étant mixte ou symétrique, et Hal' représente un halogène,
pour obtenir un composé de formule (I/d4) :

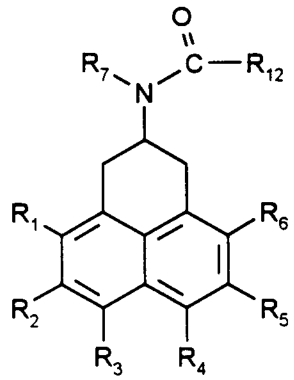

(I/d4)

dans laquelle $R_1$, $R_2$, $R_3$, $R_4$, $R_5$, $R_6$, $R_7$ et $R_{12}$ sont tels que définis précédemment, qui est ensuite soumis d'un réactif de thionation, tel que le réactif de Lawesson, pour obtenir un composé de formule (I/d4') :

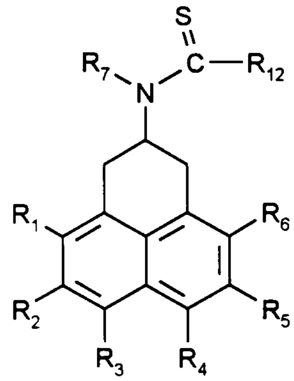

(I/d4')

dans laquelle $R_1$, $R_2$, $R_3$, $R_4$, $R_5$, $R_6$, $R_7$ et $R_{12}$ sont tels que définis précédemment,

- soit avec un composé de formule (VIII) :

$$X{=}C{=}N{-}R_{13} \quad \text{(VIII)}$$

dans laquelle $R_{13}$ est tel que défini dans la formule (I) et X représente un atome d'oxygène ou de soufre, pour obtenir un composé de formule (I/d5) :

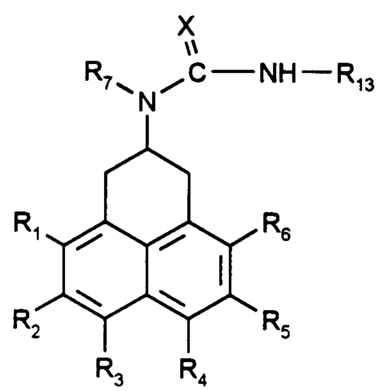

(I/d5)

dans laquelle $R_1$, $R_2$, $R_3$, $R_4$, $R_5$, $R_6$, $R_7$, $R_{13}$ et X sont tels que définis précédemment les composés de formule (I/d4),

(I/d4') et (I/d5) formant l'ensemble des composés de formule (I/d),
les composés de formule (I/d) ainsi obtenus pouvant être :

- purifiés suivant une ou plusieurs méthodes de purification choisies parmi la cristallisation, la chromatographie sur colonne de silice, l'extraction, la filtration, et le passage sur charbon ou résine,
- et séparés, sous forme pure ou sous forme de mélange, en leurs éventuels énantiomères ou diastéréoisomères,

De façon générale, les composés de formule (I) dans lesquels $R_7$ est différent d'un hydrogène peuvent être obtenus par réaction du composé de formule (I) correspondant dans lequel $R_7$ est un hydrogène avec un composé de formule (V) :

$$R_7'—Hal \qquad (V)$$

dans laquelle $R_7$' est tel que défini dans la formule (I) et Hal représente un atome d'halogène.

Plus particulièrement, l'invention concerne les procédés de préparation des composés de formule (I) dans laquelle $R_1$, $R_2$, $R_3$, et $R_4$, et $R_5$, $R_5$', $R_6$ et $R_6$' le cas échéant, représentent simultanément des hydrogènes ou dans laquelle l'un de ces substituants $R_1$, $R_2$, $R_3$, $R_4$, et $R_5$, $R_5$', $R_6$ et $R_6$' le cas échéant, est un radical choisi parmi halogène, alkyle et alkoxy, par exemple alkoxy, les autres substituants étant des atomes d'hydrogène.

Par exemple, l'invention concerne les procédés de préparation des composés de formule (I/d) dans laquelle $R_1$, $R_2$, $R_3$, et $R_4$, et $R_5$, $R_5$', $R_6$ et $R_6$' le cas échéant, représentent simultanément des hydrogènes ou dans laquelle l'un de ces substituants $R_1$, $R_2$, $R_3$, et $R_4$, et $R_5$, $R_5$', $R_6$ et $R_6$' le cas échéant, est un radical choisi parmi halogène, alkyle et alkoxy, par exemple alkoxy, les autres substituants étant des atomes d'hydrogène.

L'invention concerne également le procédé de préparation des composés de formule (I/i), cas particulier des composés de formule (I) :

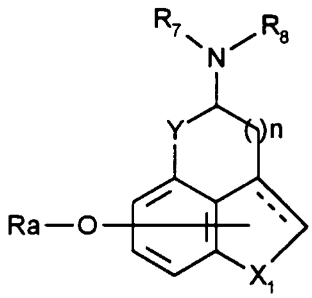

**(I/i)**

dans laquelle $R_7$, $R_8$, Ra, n, $X_1$ et Y sont tels que définis dans la formule (I) par greffage d'un radical Ra sur un composé de formule (I/j) :

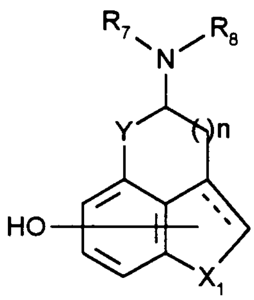

**(I/j)**

dans laquelle $R_7$, $R_8$, $X_1$, Y et n sont tels que définis précédemment,
les composés de formule (I/i) ainsi obtenus pouvant être :

- purifiés suivant une ou plusieurs méthodes de purification choisies parmi la cristallisation, la chromatographie sur colonne de silice, l'extraction, la filtration, et le passage sur charbon ou résine,
- et séparés, sous forme pure ou sous forme de mélange, en leurs éventuels énantiomères ou diastéréoisomères,

Par exemple, l'invention concerne également le procédé de préparation des composés de formule (I/i-1), cas particulier des composés de formule (I-1) telle que définie précédemment :

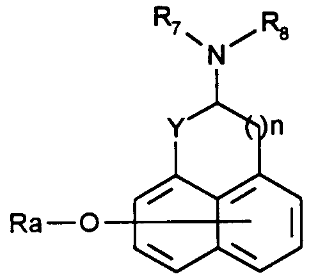

**(I/i-1)**

dans laquelle $R_7$, $R_8$, Ra, n et Y sont tels que définis dans la formule (I) par greffage d'un radical Ra sur un composé de formule (I/j-1) :

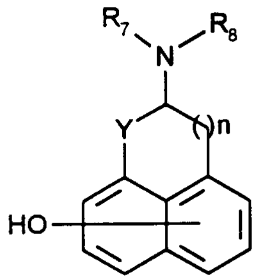

**(I/j-1)**

dans laquelle $R_7$, $R_8$, Y et n sont tels que définis précédemment,
les composés de formule (I/i) ainsi obtenus pouvant être :

- purifiés suivant une ou plusieurs méthodes de purification choisies parmi la cristallisation, la chromatographie sur colonne de silice, l'extraction, la filtration, et le passage sur charbon ou résine,
- et séparés, sous forme pure ou sous forme de mélange, en leurs éventuels énantiomères ou diastéréoisomères,

Par exemple, le greffage du radical Ra peut se faire par l'intermédiaire d'un composé de formule (IX) :

$$Ra—W \qquad (IX)$$

dans laquelle Ra est tel que défini dans la formule (I) et W représente un halogène ou un groupement partant.
Les composés de formule (I/j) :

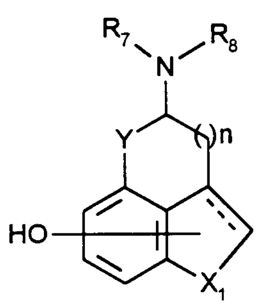

**(I/j)**

dans laquelle $R_7$, $R_8$, $X_1$, Y et n sont tels que définis dans la formule (I),
sont accessibles à l'homme du métier par déalkylation d'un composé de formule (I/k) :

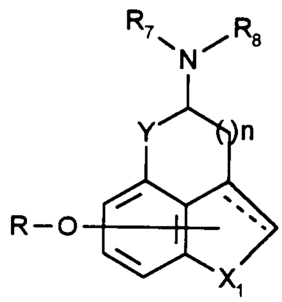

(I/k)

dans laquelle $R_7$, $R_8$, n, $X_1$ et Y sont tels que définis précédemment et R représente un radical ($C_1$-$C_6$)alkyl.

Par exemple, la déalkylation du composé (I/k) tel que défini ci-dessus peut être réalisée par l'intermédiaire du $BBr_3$ ou d'un mélange $AlX'_3$, R'-SH dans lequel X' représente un halogène et R' représente un radical ($C_1$-$C_6$)alkyl.

Les composés de formule (I/j-1) :

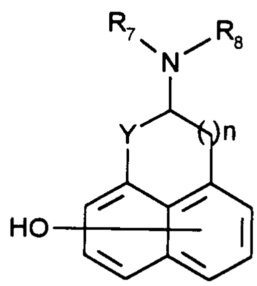

(I/j-1)

dans laquelle $R_7$, $R_8$, Y et n sont tels que définis dans la formule (I),
sont accessibles à l'homme du métier par déalkylation d'un composé de formule (I/k-1) :

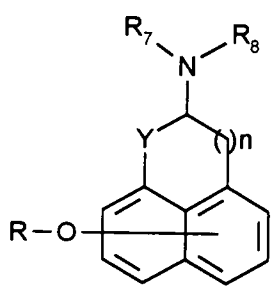

(I/k-1)

dans laquelle $R_7$, $R_8$, n et Y sont tels que définis précédemment et R représente un radical ($C_1$-$C_6$)alkyl.

Par exemple, la déalkylation du composé (I/k-1) tel que défini ci-dessus peut être réalisée par l'intermédiaire du $BBr_3$ ou d'un mélange $AlX'_3$, R'-SH dans lequel X' représente un halogène et R' représente un radical ($C_1$-$C_6$)alkyl.

Chaque matière première ou intermédiaire de synthèse utilisé est purifié, si nécessaire, grâce aux méthodes de purification classiques.

Les matières premières utilisées dans les procédés de préparation ci-dessus sont :

- soit commerciales,
- soit accessibles à l'homme du métier au vu de la littérature ou des exemples de préparations mentionnés ci-après.

Les composés de formule (II) sont ainsi accessibles par réaction d'un composé de formule (II/a) :

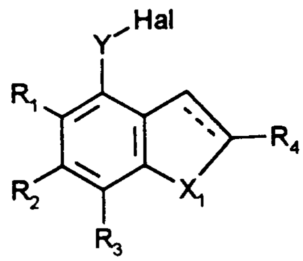

(II/a)

dans laquelle $R_1$, $R_2$, $R_3$, $R_4$, $X_1$ et Y sont tels que définis dans la formule (I) et Hal représente un atome d'halogène, avec un composé de formule (II/b) :

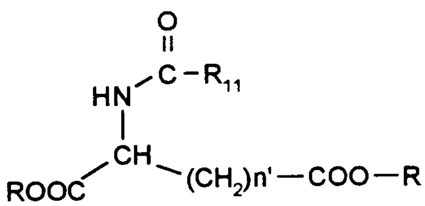

(II/b)

dans laquelle $R_{11}$ est tel que défini dans la formule (I), n' est 0, 1 ou 2, et R représente un ($C_1$-$C_6$) alkyl, pour obtenir un composé de formule (II/c) :

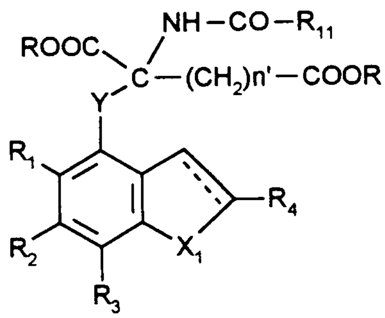

(II/c)

dans laquelle $R_1$, $R_2$, $R_3$, $R_4$, $R_{11}$, R, $X_1$, Y et n' sont tels que définis précédemment,
qui est ensuite traité par de la soude pour obtenir le composé de formule (II) : qui est ensuite traité par de la soude pour obtenir le composé de formule (II) :

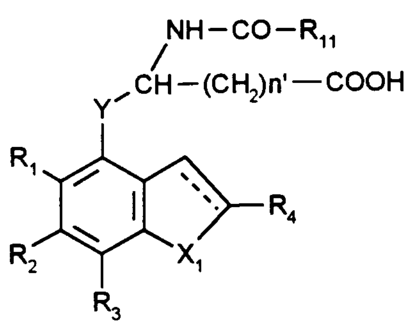

(II)

dans laquelle $R_1$, $R_2$, $R_3$, $R_4$, $R_{11}$, $X_1$, Y et n' sont tels que définis précédemment.

Par exemples, les composés de formule (II-1) sont ainsi accessibles par réaction d'un composé de formule (II/a-1) :

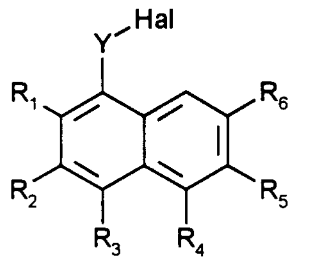

**(II/a-1)**

dans laquelle $R_1$, $R_2$, $R_3$, $R_4$, $R_5$, $R_6$ et Y sont tels que définis dans la formule (I) et Hal représente un atome d'halogène, avec un composé de formule (II/b-1) :

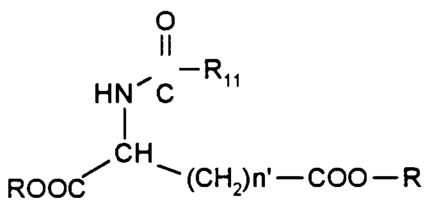

**(II/b-1)**

dans laquelle $R_{11}$ est tel que défini dans la formule (I), n' est 0, 1 ou 2, et R représente un ($C_1$-$C_6$) alkyl, pour obtenir un composé de formule (II/c-1) :

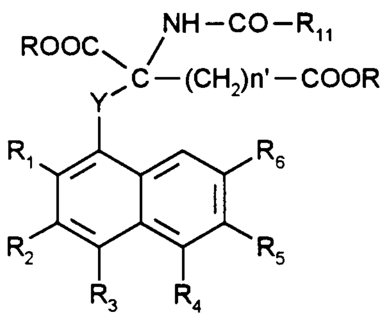

**(II/c-1)**

dans laquelle $R_1$, $R_2$, $R_3$, $R_4$, $R_5$, $R_6$, $R_{11}$, R, Y et n' sont tels que définis précédemment, qui est ensuite traité par de la soude pour obtenir le composé de formule (II-1) :

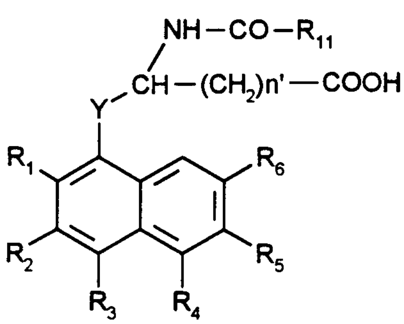

**(II-1)**

dans laquelle $R_1$, $R_2$, $R_3$, $R_4$, $R_5$, $R_6$, $R_{11}$, Y et n' sont tels que définis précédemment.

Par exemple, les composés de formule (II/d) sont ainsi accessibles par réaction d'un composé de formule (II/d1) :

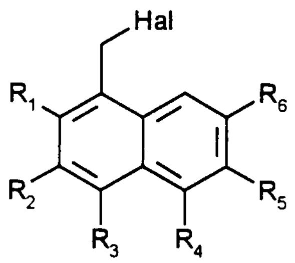

(II/d1)

dans laquelle $R_1$, $R_2$, $R_3$, $R_4$, $R_5$, et $R_6$ sont tels que définis dans la formule (I) et Hal représente un atome d'halogène, avec un composé de formule (II/d2) :

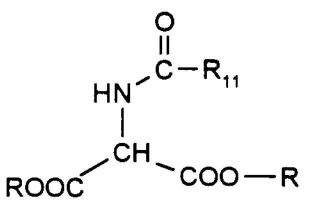

(II/d2)

dans laquelle $R_{11}$ est tel que défini dans la formule (I), et R représente un ($C_1$-$C_6$)alkyl pour obtenir un composé de formule (II/d3) :

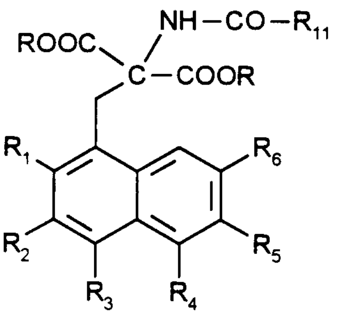

(II/d3)

dans laquelle $R_1$, $R_2$, $R_3$, $R_4$, $R_5$, $R_6$, $R_{11}$ et R sont tels que définis précédemment,
qui est ensuite traité par de la soude pour obtenir le composé de formule (II/d) :

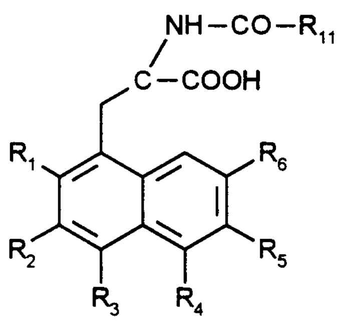

(II/d)

dans laquelle $R_1$, $R_2$, $R_3$, $R_4$, $R_5$, $R_6$ et $R_{11}$ sont tels que définis précédemment.

Les composés de formule (VI) tels que définis précédemment sont par exemple accessibles par déacylation d'un composé de formule (I/g), cas particulier des composés de formule (I) :

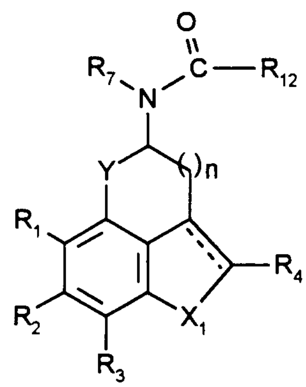

**(I/g)**

dans laquelle $R_1$, $R_2$, $R_3$, $R_4$, $R_7$, $R_{12}$, n, $X_1$ et Y sont tels que définis dans la formule (I).

Les composés de formule (VI-1) tels que définis précédemment sont par exemple accessibles par déacylation d'un composé de formule (I/g-1), cas particulier des composés de formule (I) :

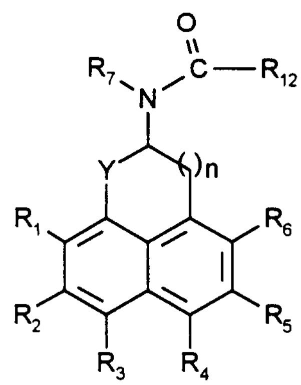

**(I/g-1)**

dans laquelle $R_1$, $R_2$, $R_3$, $R_4$, $R_5$, $R_6$, $R_7$, $R_{12}$, n et Y sont tels que définis dans la formule (I).
Par exemple, les composés de formule (VI) sont accessibles par déacétylation d'un composé de formule (I/h-1) :

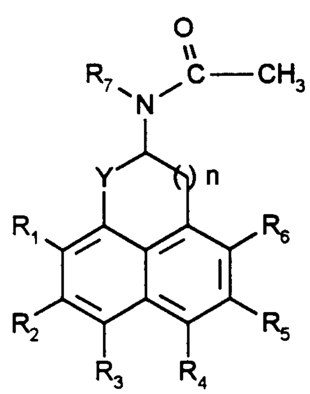

**(I/h-1)**

dans laquelle $R_1$, $R_2$, $R_3$, $R_4$, $R_5$, $R_6$, $R_7$, Y et n sont tels que définis dans la formule (I).

Les composés de formule (I) possèdent des propriétés pharmacologiques très intéressantes pour le clinicien.

Les composés de l'invention et les compositions pharmaceutiques les contenant s'avèrent être utiles pour le traitement des troubles du système mélatoninergique et des troubles liés au système mélatoninergique.

L'étude pharmacologique des dérivés de l'invention a en effet montré qu'ils n'étaient pas toxiques, doués d'une très haute affinité sélective pour les récepteurs de la mélatonine et possédaient d'importantes activités sur le système mélatoninergique en particulier, on a relevé des propriétés thérapeutiques sur les troubles du sommeil, des propriétés anxiolytiques, antipsychotiques, analgésiques ainsi que sur la microcirculation qui permettent d'établir que les produits

de l'invention sont utiles dans le traitement du stress, des troubles du sommeil, de l'anxiété, des dépressions saisonnières, des pathologies cardiovasculaires, des insomnies et fatigues dues aux décalages horaires, de la schizophrénie, des attaques de panique, de la mélancolie, des troubles de l'appétit, de l'obésité, du psoriasis, des troubles psychotiques, de l'épilepsie, de la maladie de Parkinson, de la démence sénile, des divers désordres liés au vieillissement normal ou pathologique, de la migraine, des pertes de mémoire, de la maladie d'Alzheimer, ainsi que les troubles de la circulation cérébrale. Dans un autre domaine d'activité, il apparaît que les produits de l'invention possèdent des propriétés d'inhibiteurs de l'ovulation, d'immunomodulateurs et qu'ils sont susceptibles d'être utilisés dans le traitement anticancéreux.

Les composés seront utilisés de préférence dans les traitements des dépressions saisonnières, des troubles du sommeil, des pathologies cardiovasculaires, des insomnies et fatigues dues aux décalages horaires, des troubles de l'appétit et de l'obésité.

Par exemple, les composés seront utilisés dans le traitement des dépressions saisonnières et des troubles du sommeil.

La présente invention a également pour objet les compositions pharmaceutiques contenant un composé de formule (I) en combinaison avec un ou plusieurs excipients pharmaceutiquement acceptables.

Parmi les compositions pharmaceutiques selon l'invention, on pourra citer, plus particulièrement celles qui conviennent pour l'administration orale, parentérale, nasale, per. ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques, et les ampoules buvables ou injectables.

La posologie varie selon le sexe, l'âge et le poids du patient, la voie d'administration, la nature de l'indication thérapeutique, ou des traitements éventuellement associés et s'échelonne entre 0,1 mg et 1 g par 24 heures, plus particulièrement entre 1 à 100 mg, par exemple entre 1 à 10 mg.

Les exemples suivants illustrent l'invention, mais ne la limitent en aucune façon.

**PREPARATION 1 : ACIDE 2-ACETAMIDO-3-(NAPHT-1-YL)-PROPANOIQUE**

**STADE A : 2-[(NAPHT-1-YL)-METHYL]-2-ACETAMIDO MALONATE DE DIETHYLE**

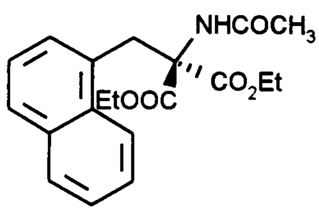

***Stade A***

**Mode opératoire :**

Le sodium (28 mmol ; 0,64g) est introduit dans 50 $cm^3$ d'éthanol absolu, sous atmosphère inerte. A 0°C, on additionne le 2-acétamidomalonate de diéthyle (28 mmol ; 6,08g) en solution dans 35 $cm^3$ d'éthanol absolu, puis au goutte à goutte à la seringue le chlorure de (napht-1-yl)méthyle (28 mmol ; 4,1 $cm^3$). Le mélange est porté à reflux jusqu'à disparition du produit de départ (6h puis la nuit à température ambiante puis 2h à reflux). Le milieu réactionnel est versé sur un mélange $KHSO_4$ (0,05M ; 240 $cm^3$) / hexane (120 $cm^3$) et agité vivement. On récupère un précipité blanc qui est le dérivé malonique attendu.

Rendement : 79 %
RMN $^1H$ ($CDCl_3$) $\delta$ (ppm) : 1,29 (t, 6H, J=7,1Hz, 2 $CH_2\underline{CH_3}$) ; 1,87 (s, 3H, $COCH_3$) ; 4,13 (s, 2H, $CH_2$) ; 4,17-4,31 (m, 4H, 2 $CH_3\underline{CH_2}$) ; 6,41 (s, 1H, NH) ; 7,16 (d, 1H, $H_{ar}$) ; 7,32-7,41 (m, 3H, $H_{ar}$) ; 7,73-7,82 (m, 2H, Har) ; 7,96-8,01 (m, 1H, $H_{ar}$).

STADE B : ACIDE 2-ACETAMIDO-3-(NAPHT-1-YL)PROPANOIQUE

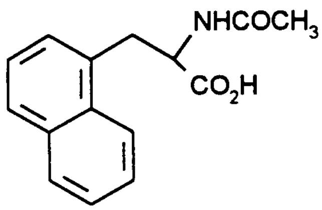

***Stade B***

**Mode opératoire :**

Le dérivé malonique obtenu au stade précédent (7mmol ; 2,5g) est mis en solution dans 13 $cm^3$ d'éthanol. On ajoute la solution de soude 2N (18 mmol ; 9 $cm^3$ ; 2,5 eq.) et on porte à reflux durant 3 h 30 min. Le milieu est acidifié jusqu'à pH=1 avec une solution $KHSO_4$ (1N). Un précipité se forme, on le filtre et isole le composé du titre qui est recristallisé dans un mélange acétone/éther de pétrole.

Rendement : 89 %
Chromatographie Couche Mince (CCM) (silice) : Rf = 0,18 (AcOH:MeOH:$CH_2Cl_2$ 0,5:4,5:95)
F (température de fusion) = 171-173°C
RMN $^1H$ ($CD_3OD$) $\delta$ (ppm) : 1,68 (s, 3H, $COCH_3$) ; 3,08-3,19 (q, 1H, AB d'un système ABX CH$\underline{CH_2}$) ; 3,53-3,63 (q, 1H, AB d'un système ABX CH$\underline{CH_2}$) ; 4,6-4,65 (m, 1H, CH) ; 7,19-7,38 (m, 4H, $H_{ar}$) ; 7,58 (t, 1H, $H_{ar}$) ; 7,68 (dd, 1H, $H_{ar}$) ; 7,98 (d, 1H, J=8,3 Hz, $H_{ar}$).

PREPARATION 2 : (2,3-DIHYDRO-1H-PHENALEN-2-YL)AMINE

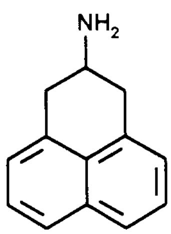

**Mode opératoire :**

Le N-(2,3-dihydro-1H-phénalèn-2-yl)acétamide (0,97mmol ; 0,22g) est solubilisé dans 2 $cm^3$ de dioxane et on ajoute 9 $cm^3$ d'HCl à 10 %. On porte à 100°C pendant 3h puis à 90°C toute la nuit. Après refroidissement on ajoute NaOH 10 % jusqu'à pH basique et l'amine est extraite avec du $CH_2Cl_2$. La phase organique est séchée sur $MgSO_4$ et évaporée sous pression réduite. On sole ainsi l'amine désirée sans autre purification.

Rendement : 90 %
RMN $^1H$ ($CDCl_3$) $\delta$ (ppm) : 1,7 (sl, 2H, $NH_2$) ; 2,85-3,0 (q, 2H, AB d'un système ABX $CHCH_2$) ; 3,2-3,4 (q, 2H, AB d'un système ABX CH$\underline{CH_2}$) ; 3,42-3,6 (m, 1H, CH) ; 7,25 (d, 2H, $H_{ar}$) ; 7,35 (t, 2H, $H_{ar}$) ; 7,68 (d, 2H, $H_{ar}$).

**PREPARATION 3 : ACIDE 2-ACETAMIDO-3-(2-METHOXY-NAPHT-1-YL) PROPANOIQUE**

**STADE A : CHLORURE DE (2-METHOXY-NAPHT-1-YL)METHYLE**

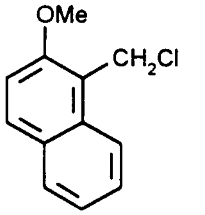

***Stade A***

**Mode opératoire :**

A une suspension d'hydrure d'aluminium et de lithium (96,66 mmol ; 3,69g ; 3eq.) dans le tétrahydrofurane (THF) anhydre on ajoute à 0°C, sous argon et au goutte à goutte la solution de (2-méthoxy-napht-1-yl)carbaldéhyde (32,22 mmol ; 6g) dans 30 $cm^3$ du même solvant. L'agitation est maintenue toute la nuit à température ambiante. On hydrolyse en ajoutant lentement à 0°C 4 $cm^3$ d'eau puis 4 $cm^3$ d'une solution NaOH à 15 % et enfin 12 $cm^3$ d'eau. Le précipité formé est filtré ; le filtrat est séché sur $MgSO_4$ puis évaporé. On sole le 2-(2-méthoxy-napht-1-yl)méthanol pur par RMN.

L'alcool ainsi obtenu (30,28 mmol ; 5,7 g) est mis en solution dans 80 $cm^3$ de toluène anhydre en présence de pyridine (30,28 mmol ; 2,5 $cm^3$). A 0°C on ajoute au goutte à goutte sous atmosphère d'argon, le chlorure de thionyle (72,67 mmol ; 5,3 $cm^3$ ; 2,4 eq.) en solution dans 6 $cm^3$ de toluène anhydre. L'agitation est maintenue toute la nuit à température ambiante puis le milieu réactionnel est versé sur de la glace et agité durant 1h. La phase organique est lavée avec une solution saturée de $NaHCO_3$, une solution saturée de NaCl puis une fois à l'eau. Elle est séchée sur $MgSO_4$ puis évaporée. On isole le composé du titre pur par RMN.

Rendement : 82 %
CCM (silice) : $R_f$ = 0,8 ($CH_2Cl_2$ : pentane)
RMN $^1H$ ($CDCl_3$) $\delta$ (ppm) : 4,0 (s, 3H, $OCH_3$) ; 5,2 (s, 2H, $ClCH_2$) ; 7,25 (d, 1H, $H_{ar}$) ; 7,43 (t, 1H, $H_{ar}$) ; 7,6 (t, 1H, $H_{ar}$) ; 7,88 (2d, 2H, $H_{ar}$) ; 8,08 (d, 1H, $H_{ar}$).

**STADE B : 2-[(2-METHOXY-NAPHT-1-YL)-METHYL]-2-ACETAMIDO MALONATE DE DIETHYLE**

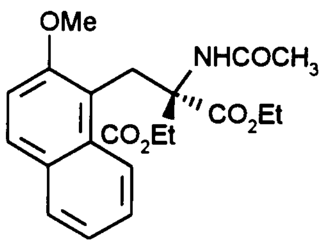

***Stade B***

**Mode opératoire :**

L'acétamidomalonate de diéthyle (44,5 mmol ; 9,65 g ; 1,2 eq.) est mis en solution dans 60 $cm^3$ de diméthyl-formamide (DMF) anhydre préalablement distillé. A 0°C sous argon, on ajoute l'hydrure de sodium (NaH) (44,5 mmol ; 1,78 g d'une solution 60 % dans l'huile ; 1,2 eq.). Le chlorure obtenu au stade précédent (37 mmol ; 7,66 g) en solution

dans 30 $cm^3$ de DMF anhydre est ensuite additionné au goutte à goutte puis on porte à reflux durant 20h. Le DMF est alors évaporé et le résidu, repris au dichlorométhane, est lavé plusieurs fois à l'eau. La phase organique est séchée sur $MgSO_4$ puis évaporée sous pression réduite. La poudre de couleur marron clair obtenue est lavée à l'éther et filtrée. On isole ainsi le composé du titre.

Rendement : 84 %
CCM (silice) : $R_f$ = 0,15 ($CH_2Cl_2$)
F = 110°C
RMN $^1H$ ($CDCl_3$) δ (ppm) : 1,22 (t, 6H, 2x $CH_2\underline{CH_3}$) ; 1,63 (s, 3H, $COCH_3$) ; 3,78 (s, 3H, $OCH_3$) ; 4,05 (s, 2H, $CH_2$) ; 4,16 (q, 4H, 2x $\underline{CH_2}CH_3$) ; 6,19, (s, 1H, NH) ; 7,16 (d, 1H, $H_{ar}$) ; 7,24 (t, 1H, $H_{ar}$) ; 7,36 (t, 1H, $H_{ar}$) ; 7,69 (2d, 2H, $H_{ar}$) ; 7,89 (d, 1H, $H_{ar}$).

**STADE C : ACIDE 2-(ACETAMIDO)-3-(2-METHOXY-NAPHT-1-YL)PROPANOIQUE**

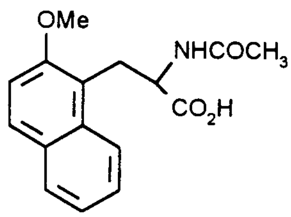

***Stade C***

**Mode opératoire :**

Le composé obtenu au stade précédent (21,38 mmol ; 8,1 g) est solubilisé dans 44 $cm^3$ d'éthanol auxquels on ajoute de la soude 2N (31 $cm^3$). Le milieu réactionnel est porté à reflux durant 6h puis le solvant est évaporé de moitié. On extrait 1 fois avec de l'éther puis la phase aqueuse est acidifiée jusqu'à pH 1 avec une solution $KHSO_4$ 1N. Un dépôt brun apparait que l'on extrait avec de l'acétate d'éthyle. La phase organique lavée à l'eau est ensuite séchée sur $MgSO_4$ puis évaporée sous pression réduite. On isole le composé du titre pur sous forme d'une poudre blanche.

Rendement : 87 %
F = 201°C
RMN $^1H$ ($CD_3OD$) δ (ppm) : 1,88 (s, 3H, $COCH_3$) ; 3,52-3,77 (m, 2H, AB d'un système ABX $\underline{CH_2}CH$) ; 4,04 (s, 3H, $OCH_3$) ; 4,74-4,78 (q, 1H, CH) ; 7,36-7,45 (m, 2H, $H_{ar}$) ; 7,56 (t, 1H, $H_{ar}$) ; 7,87 (2d, 2H, $H_{ar}$) ; 8,12 (d, 1H, $H_{ar}$).

**PREPARATION 4 : (4-METHOXY-2,3-DIHYDRO-1H-PHENALEN-2-YL)AMINE**

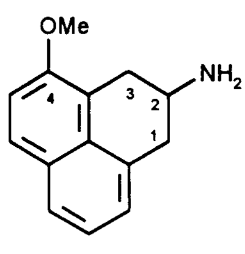

**Mode opératoire :**

Le N-(4-méthoxy-2,3-dihydro-1H-phénalèn-2-yl)acétamide (5,95 mmol ; 1,52 g) est solubilisé dans 20 $cm^3$

d'éthanol auxquels on ajoute 54 $cm^3$ d'HCl à 10 %. On porte à 90°C pendant 20h. Après refroidissement on ajoute une solution de NaOH à 10 % jusqu'à pH basique et l'amine est extraite avec du $CH_2Cl_2$. La phase organique est séchée sur $MgSO_4$ et évaporée sous pression réduite.
On isole ainsi l'amine du titre sans autre purification.

Rendement : 79 %
RMN $^1H$ ($CDCl_3$) $\delta$ (ppm) : 1,6 (s, 2H, $NH_2$) ; 2,6-2,95 (m, 2H, AB d'un système ABX $\underline{CH_2}CH$) ; 3,05-3,45 (m, 3H, X et AB d'un système ABX $CH_2CH$) ; 3,93 (s, 3H, $OCH_3$) ; 7,1-7,23 (m, 3H, $H_{ar}$) ; 7,55 (dd, 2H, $H_{ar}$) ; 7,65 (d, 1H, $H_{ar}$).

Le chlorhydrate correspondant de l'amine du titre est obtenu par addition d'éther à une solution de l'amine du titre dans un minimum d'éthanol en présence d'éthanol chlorhydrique.

Rendement : 80%
RMN $^1H$ ($CD_3OD$) $\delta$ (ppm) : 3,06-3,7 (m, 4H, 2 AB d'un système ABX $\underline{CH_2}CH$) ; 3,83-3,9 (m, $(CH_2)_2CH$) ; 4,06 (s, 3H, $OCH_3$) ; 7,36-7,39 (2d, 2H, $H_{ar}$) ; 7,49 (d, 1H, $H_{ar}$) ; 7,79 (t, 1H, $H_{ar}$) ; 7,9 (d, 1H, $H_{ar}$).

| **Analyse élémentaire :** Formule : $C_{14}H_{16}NOCl$, M=249,77 | | | |
|---|---|---|---|
| Calculé : | C : 67,31 % | H : 6,45 % | N : 5,60 % |
| Trouvé : | C : 67,12 % | H : 6,54 % | N : 5,66 % |

PREPARATIONS 5 A 32 :

En procédant comme dans la préparation 1, mais en utilisant au stade A le malonate de diéthyle convenablement substitué, on obtient les préparations suivantes :

PREPARATION 5 : ACIDE 2-PROPIONAMIDO-3-(NAPHT-1-YL)PROPANOIQUE

PREPARATION 6 : ACIDE 2-BUTYRAMIDO-3-(NAPHT-1-YL)PROPANOIQUE

PREPARATION 7 : ACIDE 2-PENTANAMIDO-3-(NAPHT-1-YL)PROPANOIQUE

PREPARATION 8 : ACIDE 2-HEXANAMIDO-3-(NAPHT-1-YL)PROPANOIQUE

PREPARATION 9 : ACIDE 2-HEPTANAMIDO-3-(NAPHT-1-YL)PROPANOIQUE

PREPARATION 10 : ACIDE 2-(2-IODOACETAMIDO)-3-(NAPHT-1-YL)PROPANOIQUE

PREPARATION 11 : ACIDE 2-(2-BROMOACETAMIDO)-3-(NAPHT-1-YL)PROPANOIQUE

PREPARATION 12 : ACIDE 2-TRIFLUOROACETAMIDO-3-(NAPHT-1-YL)PROPANOIQUE

PREPARATION 13 : ACIDE 2-ISOPENTANAMIDO-3-(NAPHT-1-YL)PROPANOIQUE

PREPARATION 14 : ACIDE 2-ISOBUTYRAMIDO-3-(NAPHT-1-YL)PROPANOIQUE

PREPARATION 15 : ACIDE 2-CYCLOPROPYLCARBOXAMIDO-3-(NAPHT-1-YL)PROPANOIQUE

PREPARATION 16 : ACIDE 2-CYCLOBUTYLCARBOXAMIDO-3-(NAPHT-1-YL)PROPANOIQUE

PREPARATION 17 : ACIDE 2-CYCLOHEXYLCARBOXAMIDO-3-(NAPHT-1-YL)PROPANOIQUE

PREPARATION 18 : ACIDE 2-CYCLOPROPYLACETAMIDO-3-(NAPHT-1-YL)PROPANOIQUE

PREPARATION 19 : ACIDE 2-BENZYLCARBOXAMIDO-3-(NAPHT-1-YL)PROPANOIQUE

PREPARATION 20 : ACIDE 2-PHENYLCARBOXAMIDO-3-(NAPHT-1-YL)PROPANOIQUE

PREPARATION 21 : ACIDE 2-ACETAMIDO-3-(INDOL-4-YL)PROPANOIQUE

PREPARATION 22 : ACIDE 2-ACETAMIDO-3-(5-METHOXY-INDOL-4-YL)PROPANOIQUE

PREPARATION 23 : ACIDE 2-ACETAMIDO-3-(5-BROMO-INDOL-4-YL)PROPANOIQUE

PREPARATION 24 : ACIDE 2-ACETAMIDO-3-(5-ETHYL-INDOL-4-YL)PROPANOIQUE

PREPARATION 25 : ACIDE 2-ACETAMIDO-3-(BENZOFURAN-4-YL)PROPANOIQUE

PREPARATION 26 : ACIDE 2-ACETAMIDO-3-(5-METHOXY-BENZOFURAN-4-YL)PROPANOIQUE

PREPARATION 27 : ACIDE 2-ACETAMIDO-3-(5-BROMO-BENZOFURAN-4-YL)PROPANOIQUE

PREPARATION 28 : ACIDE 2-ACETAMIDO-3-(5-ETHYL-BENZOFURAN-4-YL)PROPANOIQUE

PREPARATION 29 : ACIDE 2-ACETAMIDO-3-(BENZOTHIOPHEN-4-YL)PROPANOIQUE

PREPARATION 30 : ACIDE 2-ACETAMIDO-3-(5-METHOXYBENZOTHIOPHEN-4-YL)PROPANOIQUE

PREPARATION 31 : ACIDE 2-ACETAMIDO-3-(5-BROMOBENZOTHIOPHEN-4-YL)PROPANOIQUE

PREPARATION 32 : ACIDE 2-ACETAMIDO-3-(5-ETHYLBENZOTHIOPHEN-4-YL)PROPANOIQUE

PREPARATION 33 : ACIDE 2-ACETAMIDO-3-(2,7-DIMETHOXY-NAPHT-1-YL)PROPANOIQUE

*Stade A : Chlorure de (2,7-diméthoxy-napht-1-yl)-méthyle 5 :*

**Mode opératoire :**

Le 2,7-dihydroxynaphtalène 1 (62 mmol ; 10g) et le diméthylsulfate (126 mmol ; 11,9 $cm^3$) sont mis en solution dans 320 $cm^3$ d'acétone anhydre en présence de $K_2CO_3$ anhydre (304 mmol ; 42 g). Le milieu réactionnel est porté au reflux du solvant pendant 6h puis à température ambiante toute la nuit. Après addition de 7,4 $cm^3$ d'eau, l'agitation est encore maintenue à température ambiante durant 2h, puis le carbonate est filtré. Le filtrat est séché sur $MgSO_4$ et évaporé sous pression réduite. Une cristallisation dans un mélange $CH_2Cl_2$ : EP permet d'isoler 10,7g de 2,7-diméthoxy-naphtalène 2 attendu.

Le 2,7-diméthoxynaphtalène 2 (65 mmol ; 12,26g) est dissous dans 150 $cm^3$ de dichlorométhane anhydre sous atmosphère inerte. A la seringue on ajoute à 0°C le $TiCl_4$ (91 mmol ; 10 $cm^3$) puis au goutte à goutte à l'aide d'une ampoule d'addition l'$\alpha$-$\alpha$-dichlorométhyl méthyléther (99 mmol ; 9 $cm^3$) en solution dans 40 $cm^3$ du même solvant. L'agitation est maintenue à 0°C pendant 1h puis à température ambiante durant 4h30. Le milieu réactionnel est versé sur un mélange glace-HCl 3N (250 $cm^3$), agité vivement puis extrait au $CH_2Cl_2$. La phase organique est lavée à l'eau puis avec une solution saturée de $NaHCO_3$. La phase organique est séchée sur $MgSO_4$ et évaporée. Le résidu est repris par de l'éther, la solution est filtrée et le filtrat évaporé à sec. Le 2,7-diméthoxynaphtaldéhyde 3 est isolé pur sous forme d'une poudre beige.

A une suspension de LAH (46 mmol ; 1,75g) dans 100 $cm^3$ de THF anhydre est ajoutée à 0°C, sous argon et au goutte à goutte la solution de 2,7 diméthoxynaphtaldéhyde 3 (19 mmol ; 4g) dans 30 $cm^3$ du même solvant. L'agitation est maintenue toute la nuit à température ambiante. Le milieu réactionnel est hydrolysé par addition lente à 0°C de 1,8 $cm^3$ d'eau puis 1,8 $cm^3$ d'une solution NaOH à 15 % et enfin 5,4 $cm^3$ d'eau. Le précipité formé est filtré ; le filtrat est séché sur $MgSO_4$ puis évaporé. On isole le (2,7-diméthoxynaphtyl)méthanol 4 pur par RMN.

L'alcool 4 (18 mmol; 4g) est mis en solution dans 80 $cm^3$ de toluène anhydre en présence de pyridine (1,5 $cm^3$). A 0°C on ajoute au goutte à goutte sous atmosphère d'argon, le chlorure de thionyle (45 mmol ; 3,3 $cm^3$ ; 2,4 eq.) en solution dans 3,8 $cm^3$ du même solvant. L'agitation est maintenue toute la nuit à température ambiante puis le milieu réactionnel est versé sur de la glace et agité durant 1h30. La phase organique est séparée et lavée avec une solution aqueuse saturée de $NaHCO_3$, une solution aqueuse saturée de NaCl puis une fois à l'eau. Elle est séchée sur $MgSO_4$ puis évaporée sous pression réduite. Le chlorure attendu isolé est suffisamment pur pour être directement remis en réaction.

**Données physico-chimiques :**

2 :

Rendement = 91 %
CCM (silice) : Rf = 0,83 ($CH_2Cl_2$)
F=142°C
RMN $^1H$ ($CDCl_3$) $\delta$ (ppm) : 3,83 (s, 6H, 2x$OCH_3$) ; 6,90 (dd, 2H, J=9 et 3Hz, $H_3$ et $H_6$) ; 7,00 (d, 2H, J=3Hz, $H_1$ et $H_8$) ; 7,59 (d, 2H, J=9Hz, $H_4$ et $H_5$).

3 :

Rendement = 76 %
CCM (silice) : Rf = 0,91 (MeOH:$CH_2Cl_2$ 5:95)
F=98°C
RMN $^1H$ ($CDCl_3$) $\delta$ (ppm) : 3,91 (s, 3H, $OCH_3$) ; 3,95 (s, 3H, $OCH_3$) ; 6,99 (dd, 1H, J=9 et 3Hz, $H_6$) ; 7,03 (d, 1H, J=9Hz, $H_3$) ; 7,55 (d, 1H, J=9Hz, $H_5$) ; 7,87 (d, 1H, J=9Hz, $H_4$) ; 8,77 (d, 1H, J=3hz, $H_8$) ; 10.81 (s, 1H, CHO).

4 :

Rendement = 94 %
F=107°C
RMN $^1H$ ($CDCl_3$) $\delta$ (ppm) : 1,97-2,02 (s, 1H, OH) ; 3,95 (s, 3H, $OCH_3$) ; 3,97 (s, 3H, $OCH_3$) ; 5,16 (s, 2H, $HOC\underline{H_2}$) ; 7,05 (dd, 1H, J=11 et 3Hz, $H_6$) ; 7,13 (d, 1H, J=11Hz, $H_3$) ; 7,39 (d, 1H, J=3Hz, $H_8$) ; 7,71 (d, 1H,

J=11Hz, $H_4$) ; 7,76 (d, 1H, J=11Hz, $H_5$).

<u>**5**</u> :

Rendement = 94 %
RMN $^1H$ ($CDCl_3$) δ (ppm) : 3,97 (s, 3H, $OCH_3$) ; 3,99 (s, 3H, $OCH_3$) ; 5,16 (s, 2H, $ClCH_2$) ; 7,05 (dd, 1H, J=9 et 3Hz, $H_6$) ; 7,11 (d, 1H, J=9Hz, $H_3$) ; 7,28 (d, 1H, J=3Hz, $H_8$) ; 7,70 (d, 1H, J=9Hz, $H_4$) ; 7,77 (d, 1H, J=9Hz, $H_5$).

***Stade B : 2-[(2,7-diméthoxy-napht-1-yl)-méthyl]-2-acétamido malonate de diéthyle :***

**Mode opératoire :**

L'acétamido malonate de diéthyle (21 mmol ; 4,5g) est mis en solution dans 30 $cm^3$ de DMF anhydre préalablement distillé. A 0°C sous argon, on ajoute le NaH (21 mmol ; 0,5 g suspension à 60 % dans l'huile). Le chlorure obtenu au stade précédent (17 mmol ; 4,08 g) en solution dans 16 $cm^3$ de DMF anhydre est ensuite additionné au goutte à goutte puis le milieu réactionnel est porté au reflux du solvant durant 20h. Le DMF est évaporé et le résidu repris au dichlorométhane est lavé plusieurs fois à l'eau. La phase organique est séchée sur $MgSO_4$ puis évaporée sous pression réduite. Le produit obtenu est lavé à l'éther et filtré. On isole ainsi le dérivé du stade B pur.

Rendement = 55%
CCM (silice) : Rf = 0,79 (MeOH :$CH_2Cl_2$ 2:98)
RMN $^1H$ ($CDCl_3$) δ (ppm) : 1,29 (t, 6H J=7Hz, 2x $CH_2\underline{CH_3}$) ; 1,65 (s, 3H, $COCH_3$) ; 3,82 (s, 3H, $OCH_3$) ; 3,94 (s, 3H, $OCH_3$) ; 4,07 (s, 2H, $CH_2$) ; 4,17-4,38 (m, 4H, 2x $\underline{CH_2}CH_3$) ; 6,27 (s, 1H, NH) ; 6,96 (dd, 1H, J=9 et 2,3Hz, $H_6$) ; 7,04 (d, 1H, J= 9Hz, $H_3$) ; 7,27 (d, 1H, J= 2,3Hz, $H_8$) ; 7,63 (d, 1H, J= 9Hz, $H_4$) ; 7,69 (d, 1H, J= 9Hz, $H_5$).

***Stade C : acide 2-acétamido-3-(2,7-diméthoxy-napht-1-yl)propanoïque***

**Mode opératoire :**

Le composé obtenu au stade précédent (10 mmol ; 4,01 g) est solubilisé dans 30 $cm^3$ d'éthanol auxquels on ajoute de la soude 2N (15 $cm^3$). Le milieu réactionnel est porté au reflux du solvant durant 3h et la nuit à température ambiante. On extrait 1 fois avec de l'éther puis la phase aqueuse est acidifée jusqu'à pH 1 avec une solution $KHSO_4$ (1N). Le précipité formé est extrait avec de l'acétate d'éthyle. La phase organique lavée à l'eau est ensuite séchée sur $MgSO_4$ puis évaporée sous pression réduite. Le composé attendu est isolé pur sous forme d'une poudre blanche.

Rendement = 76%
RMN $^1H$ ($CD_3OD$) δ (ppm) : 1,83 (s, 3H, $COCH_3$) ; 3,40-3,67 (m, 2H, $\underline{CH_2}$) ; 3,9 (s, 6H, $2xOCH_3$) ; 4,63-4,71 (t, 1H, CH) ; 6,78-6,84 (dd, 1H, J=9Hz, $H_{ar}$) ; 7,13 (d, 1H, $H_{ar}$) ; 7,32 (d, 1H, $H_{ar}$) ; 7,63 (d, 1H, $H_{ar}$) ; 7,68 (d, 1H, $H_{ar}$).

PREPARATION 34 : ACIDE 2-PROPIONAMIDO-3-(2,7-DIMETHOXY-NAPHT-1-YL)PROPANOIQUE

PREPARATION 35 : ACIDE 2-CYCLOPROPYLCARBOXAMIDO-3-(2,7-DIMETHOXYNAPHT-1-YL)PROPANOIQUE

PREPARATION 36 : N-ETHYL N-(4-METHYL-2,3-DIHYDRO-1H-PHENALEN-2-YL)AMINE (J. Chem. Soc 1971, (9), pp 1607-1609)

PREPARATION 37 : N-METHYL N-(2,3-DIHYDRO-1H-PHENALEN-2-YL)AMINE (Chim. Ther. 1971, 6 (3), pp 196-202)

PREPARATION 38 : (5-METHOXY-2,3-DIHYDRO-1H-PHENALEN-2-YL)AMINE (Tetrahedron Lett. 1988, 29(16), pp 1883-1886)

PREPARATION 39 : 10-METHOXY-2-AMINO-1,2,3,4-TETRAHYDRO-PHENANTHRENE

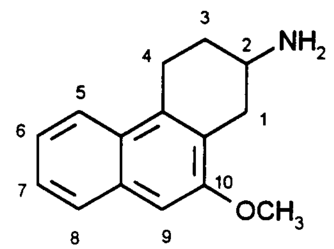

***Stade A : 2-méthoxy-3-naphtoate de méthyle***

L'acide 2-hydroxy-3-naphtoïque (18.8g ; 100 mmol), le diméthylsulfate (1 equiv) et du $K_2CO_3$ anhydre (4,8 equiv.) dans 500 $cm^3$ d'acétone anhydre sont portés au reflux pendant 6 h. 25 $cm^3$ d'eau sont ajoutés au mélange refroidi qui est agité 2h à température ambiante. Après filtration, de l'éther est ajouté à la solution qui est séchée sur $MgSO_4$. Le solvant est évaporé pour donner le composé qui est purifié par chromatographie flash sur gel de silice (Eluant : éther / éther de pétrole) Huile.

Rendement : 96 %
RMN $^1H$ (200 MHz, $CDCl_3$) $\delta$ 8,2 (s, 1H), 7,6-7,65 (2d, J=8,1 Hz, 2H), 7,2-7,35 (2td, J= 8,1, 6,9 et 1,1 Hz, 2H), 7,05 (s, 1H) ; 3,85-3,9 (2s, 6H) ; RMN $^{13}C$ (200 MHz, $CDCl_3$) ; $\delta$ 166,4, 155,4, 135,8, 132,5, 128,4, 128,1, 127,2, 126,2, 124,6, 121,4, 106,5, 55,6, 51,9.

***Stade B : (2-méthoxy-napht-3-yl)méthanol***

Une solution du composé obtenu au stade précédent (10, 8 g, 50 mmol) est ajouté lentement à une suspension sous agitation de $LiAlH_4$ (28,9 mmol) dans 50 $cm^3$ de THF anhydre, refroidie à 0°C par un bain de glace. Le mélange est porté à température ambiante puis à reflux pendant 18h sous agitation. L'eau (1 $cm^3$), une solution de NaOH 15 % (1 $cm^3$) puis de l'eau (3 $cm^3$) sont ajoutés goutte à goutte au mélange refroidi jusqu'à ce qu'aucune émission gazeuse ne soit plus observée. La solution est filtrée sur $MgSO_4$ et les solvants sont éliminés sous vide. Le résidu est purifié par chromatographie-flash (éluant : 2 % Méthanol / $CH_2$ $Cl_2$).

Rendement : 85,3 %
F = 70,5°C
RMN $^1H$ (200 MHz, $CDCl_3$) $\delta$ 7,55 (m, 3H), 7,1-7,3 (m, 2H), 6,9 (s, 1H), 4,6 (s, 2H) ; 3,7 (s, 3H) ; 2,8 (s, 1H) RMN $^{13}C$ : (200 MHz, $CDCl_3$) ; $\delta$ 155,7, 133,9, 130,5, 128,6, 127,6, 127,1, 126,3, 126,1, 123,8, 104,9, 61,9, 55,1.

***Stade C : 2-méthoxy-3-chlorométhyl-naphtalene***

Une solution de chlorure de thionyl (1,5 equiv.) et de pyridine (1 equiv). dans du toluène anhydre (360 $cm^3$)

est refroidie à 0°C dans un bain de glace. Le composé obtenu au stade précédent est ajouté lentement sous agitation forte.

Le milieu est mis sous agitation à température ambiante durant une nuit puis verser sur de la glace. La solution est mise sous agitation pendant 1 h.

La phase organique est séparée puis lavée 2 fois avec de l''eau, puis saturée par une solution aqueuse de $NaHCO_3$ et la saumure. Après séchage sur $MgSO_4$, le solvant est évaporée pour donner le composé attendu.

Rendement : 98,3 %
Poudre jaune pâle
F = 134°C
RMN $^1H$ (200 MHz, $CDCl_3$) δ 7,7 (m, 2H), 7,6 (m, 2H), 7,25-7,35 (m, 2H), 7,0 (s, 1H); 4,7 (s, 2H) ; 3,8 (s, 3H) ; RMN $^{13}C$ (200 MHz, $CDCl_3$) ; δ 155,2, 134,4, 129,7, 128,1, 127,5, 127,1, 126,6, 126,2, 123,8, 105,3, 55,3, 41,9.

*Stade D : Acide (2-méthoxy-3-méthylnaphtyl)succinique*

Du sodium (265 mmol) est ajouté par petits morceaux dans une solution d'acétyl succinate de diéthyle (278 mmol) dans 200 $cm^3$ de toluène anhydre et chauffée à 80°C. Le composé obtenu au stade précédent (316 mmol) est ajouté lentement et le milieu réactionnel est porté au reflux pendant 18h. La solution refroidie est acidifiée avec de l'acide acétique jusqu'à pH 7 et évaporée.

De l'eau est ajoutée et la solution est extraite avec de l'éther. L'ensemble des phases organiques est séché sur $MgSO_4$ et le solvant est éliminé pour donner un résidu qui est hydrolysé par ébullition dans 1400 $cm^3$ de NaOH 2N pendant 18h. La solution alcaline refroidie est lavée avec de l'éther et acidifié avec de l'HCl concentré pour obtenir le composé du titre sous forme d'huile qui est extrait par l'acétate d'éthyl. La phase organique est séchée sur $MgSO_4$ et le solvant évaporé. Recristallisation dans l'acétone/toluène.

Rendement :16 %
F = 159°C
RMN $^1H$ (200 MHz, $CD_3OD$) δ 7,75-7,8 (m, 2H), 7,6 (s, 1H), 7,3-7,5 (m, 2H) ; 7,3 (s, 1H), 4.0 (s, 3H), 3,2-3,4 (m, 2H), 3,0 (m, 1H), 2,4-2,75 (m, 2H) ; RMN $^{13}C$ (200 MHz, $CD_3OD$) ; δ 178,6, 175,9, 157,8, 135,6, 130,9, 130,2, 129,9, 128,3, 127,6, 127,1, 34,2, 124,8, 106,3, 55,9, 42,9, 36,4.

*Stade E : 10-méthoxy-2-carboxy-1,2,3,4-tétrahydrophénanthren-4-one*

Le composé obtenu au stade précédent (33,6 mmol) est converti en anhydride par reflux pendant 2h avec l'anhydride acétique (80 g). Le solvant est éliminé sous vide pour donner une huile brune qui sera utilisée sans autre purification.

L'anhydride ainsi obtenu (25,5 mmol) est dissous dans 60 $cm^3$ de nitrobenzène et ajouté lentement à une solution à 0°C d'$AlCl_3$ (75,5 mmol) dans 60 $cm^3$ de nitrobenzène. Le mélange réactionnel est agité pendant 5 min et une solution de glace avec 40 $cm^3$ d'HCl concentré est ajoutée. On laisse reposer une nuit puis le nitrobenzène est éliminé par entrainement à la vapeur. La solution refroidie est extraite avec de l'acétate d'éthyle et la phase organique est séchée sur $MgSO_4$ puis le solvant est évaporé. Le produit du titre est purifié par chromatographie-flash sur gel de silice (éluant : 5 % Méthanol/$CH_2Cl_2$)

Rendement : 58 %
F = 196°C
RMN $^1H$ (200 MHz, $CD_3OD$) δ 9,1 (m, 1H), 7,6 (m, 1H), 7,35 (m, 2H) ; 7,2 (s, 1H), 3,8 (s, 3H), 2,8-3,4 (m, 5H) ; RMN $^{13}C$ (200 MHz, $CD_3OD$) ; δ 200,6, 177,0, 155,7, 138,8, 135,2, 129,4, 128,9, 128,4, 127,7, 127,5, 127,2, 112,7, 56,4, 43,4, 40,2, 27,7.

*Stade F : 10-méthoxy-2-carboxy-1,2,3,4-tétrahydrophénanthrene*

Le composé obtenu au stade précédent (11,1 mmol) est réduit avec $H_2$ sous pression atmosphérique en utilisant du palladium sur charbon activé comme catalyseur. La réaction est réalisée durant une nuit avec 10 % en poids de Pd/C 10 % dans l'AcOH à 70°C.

Rendement : 83 %
F = 235°C
RMN $^1H$ (200 MHz, $CDCl_3$) δ 7,8 (dd, J=7,3, 2,2 Hz, 1H), 7,6 (dd, J=7, 2,2 Hz, 1H), 7,3 (m, 2H) 6,9 (s, 1H), 3,8 (s,

3H), 2,5-3,3 (m, 5H), 2,25 (m, 1H), 1,8 (m, 1H); RMN $^{13}C$ (200 MHz, $CDCl_3$) ; δ 178,4, 155,6, 132,8, 132,0, 127,4, 126,9, 125,3, 125,2, 123,3, 122,5, 102,5, 54,9, 39,0, 26,2, 25,2, 25,1.

***Stade G : 10-méthoxy-2-amino-1,2,3,4-tétrahydrophénanthrene***

Une solution du composé obtenu au stade précédent (7,5 mmol), du diphénylphosphorylazide (1 équiv) et de triéthylamine (1,1 equiv) dans 35 $cm^3$ de terbutanol est portée à reflux pendant 18h. Le mélange réactionnel est évaporé 220 $cm^3$ toluène sont ajoutés et la solution est successivement lavée avec une solution d'acide citrique 5 % (20 $cm^3$), de l'eau (20 $cm^3$) une solution aqueuse saturée de $NaHCO_3$ (40 $cm^3$) et de la saumure (20 $cm^3$). Le solvant est éliminé sous vide pour produire le dérivé carbamate correspondant, qui est utilisé sans autre purification.
A une solution du carbamate (1,1 mmol) dans 10 $cm^3$ d'acétate d'éthyle sont ajoutés 10 $cm^3$ d'une solution d'acétate d'éthyle chlorhydrique dans l'acétate d'éthyle anhydre. Le milieu réactionnel est mis sous agitation pendant 3h puis évaporé pour donner le composé du titre qui est purifié par dissolution dans l'éthanol puis précipitation dans le diéthyléther. Le précipité est filtré et séché pour donner le composé du titre sous forme de chlorhydrate.

Rendement : 77 %
F = 272°C (chlorhydrate)
RMN $^1H$ (200 MHz, $CD_3OD$) δ 7,6 (d, J=7,3 Hz, 1H), 7,5 (J= 8,7 Hz, 1H), 7,2-7,1 (m, 2H), 6,9 (s, 1H), 3,7 (s, 3H), 3,3 (m, 1H) 3,2-3,0 (m, 2H) 3,0-2,8 (m, 1H), 2,5 (m, 1H), 2,15 (m, 1H), 1,8-1,7 (m, 1H); RMN $^{13}C$ (200 MHz, $CD_3OD$) ; δ 156,5, 134,7, 132,3, 128,5, 128,2, 126,8, 124,8, 123,6, 123,5, 104,4, 55,7, 48,9, 29,3, 27,7, 25,0.

<u>PREPARATION 40</u> : 10-METHOXY-3-AMINO-1,2,3,4-TETRAHYDRO-PHENANTHRENE

En procédant comme dans la préparation 39 mais en partant de l'acide naphtoïque convenablement substitué, on obtient le produit du titre.

<u>PREPARATION 41</u> : 9-METHOXY-2-AMINO-1,2,3,4-TETRAHYDRO-ANTHRACENE

En procédant comme dans la préparation 39 mais en partant de l'acide 1-hydroxy-2-naphtoïque, on obtient le produit du titre.

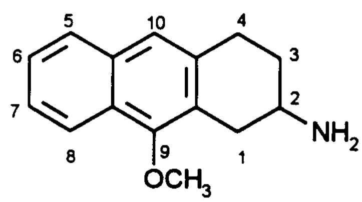

***Stade A : 1-méthoxy-naphtoate de méthyle***

Rendement : 95%
huile.

***Stade B : 1-méthoxy-2-hydroxyméthyl-naphtalene***

Rendement : 84,3 %
F : 68°C

***Stade C : 1-méthoxy-2-chlorométhyl-naphtalene***

Rendement : 98 %
F : 134°C

***Stade D : acide (1-méthoxy-2-méthylnaphtyl)succinique***

Rendement : 17 %
F : 161°C

***Stade E : 10-méthoxy-3-carboxy-1,2,3,4-tétrahydroanthracèn-1-one***

Rendement : 46,3 %
F : 202°C

***Stade F : 9-méthoxy-2-carboxy-1,2,3,4-tétrahydro-anthracene***

NB : La réduction est réalisée dans le méthanol en présence d'HCl. Après filtration, l'huile doit être hydrolysée par ébulition avec NaOH 2N pendant 30 min puis acidifiée avec HCl concentré jusqu'à pH 1 pour donner le produit du titre.
Rendement : 61 %
F : 192°C

***Stade G : 9-méthoxy-2-amino-1,2,3,4-tétrahydro-anthracene***

Rendement : 45 %
F : 285°C (chlorhydrate)

**EXEMPLE 1 : N-(2,3-DIHYDRO-1H-PHENALEN-2-YL)ACETAMIDE**

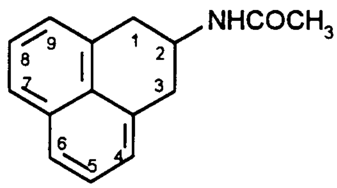

***Stade A : 2-méthyl-4-[(napht-1-yl)méthyl]-oxazolin-5-one***

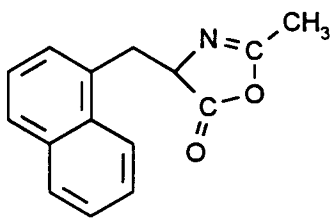

***Stade A***

**Mode opératoire :**

Le composé obtenu dans la préparation 1 (28,76 mmol ; 7,4 g) est solubilisé dans 10eq. d'$Ac_2O$ (287,6 mmol ; 27,18 $cm^3$). On porte à reflux pendant 40 min et on évapore l'acide acétique à sec. Le résidu est purifié par distillation au four à boules pour donner une huile jaune correspondant au composé du titre.

Rendement : 89 %
CCM (silice) : $R_f$ = 0,85 (MeOH : $CH_2Cl_2$ 2:98)
Eb = 180-190°C/5mmHg
RMN $^1H$ ($CDCl_3$) $\delta$ (ppm) : 1,42 (s, 3H, $CH_3$) ; 3,32-3,45 (q, 1H, AB d'un système ABX CH$\underline{CH_2}$) ; 3,72-3,85 (q, 1H, AB d'un système ABX CH$\underline{CH_2}$) ; 3,9-4,12 (m, 1H, CH) ; 7,4-7,58 (m, 5H, $H_{ar}$) ; 7,72-7,9 (m, 2H, $H_{ar}$) ; 8,1 (d, 1H, $H_{ar}$)

*Stade B : N-(1-oxo-2,3-dihydro-1-h-phénalen-2-yl)acétamide*

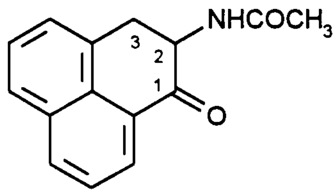

***Stade B***

**Mode opératoire :**

A une solution d'$AlCl_3$ (45,75 mmol ; 6,1 g ; 3 eq) dans 75 $cm^3$ de $Cl_2CHCHCl_2$, on ajoute au goutte à goutte le composé obtenu au stade précédent (15,25 mmol ; 3,65 g) en solution dans 150 $cm^3$ du même solvant. On porte 2h à 60°C et on laisse revenir à température ambiante. Le milieu réactionnel est versé sur un mélange glace (25 g) / HCl conc. (1,5 $cm^3$) et agité pendant 1h. La phase aqueuse est extraite deux fois au chloroforme puis les phases organiques réunies sont séchées sur $MgSO_4$ et évaporées sous pression réduite. Le résidu est chromatographié sur colonne de gel de silice (granulométrie 60-200 µm), éluant: $CH_2Cl_2$ puis $CH_2Cl_2$ : MeOH,98 : 2,v/v.
On isole ainsi le produit du titre.

Rendement : 54 %
CCM (silice) : $R_f$ = 0,68 (MeOH : $CH_2Cl_2$,5:95)
F = 185-186°C ($CH_2Cl_2$/EP)
RMN $^1H$ ($CDCl_3$) δ (ppm) : 2,14 (s, 3H, $COCH_3$) ; 3,09-3,23 (q, 1H, $J_{AX}$=13Hz, $J_{AB}$= -15Hz, AB d'un système ABX CH$\underline{CH_2}$) ; 4,02-4,13 (q, 1H,$J_{BX}$=5,5Hz, $J_{AB}$=15Hz, AB d'un système ABX CH$\underline{CH_2}$) ; 4,99-5,05 (m, 1H, CH) ; 6,48 (sl, 1H, NH) ; 7,5-7,65 (m, 3H, $H_{ar}$) ; 7,80-7,84 (m, 1H, $H_{ar}$) ; 8,1-8,17 (d et t, 2H, $H_{ar}$).
RMN $^{13}C$ ($CDCl_3$) δ (ppm) : 23,24 ($COCH_3$) ; 35,19 ($CH_2$) ; 55,47 ($\underline{CH}$NH) ; 125,52 ($C_{ar}$) ; 126,49 ($C_{ar}$) ; 126,62 ($C_{ar}$) ; 128,96 ($C_{ar}$) ; 131,04 ($C_{ar}$) ; 133 ($C_{ar}$) ; 134,54 ($C_{ar}$) ; 170,29 ($COCH_3$) ; 195 (CO)

| **Analyse élémentaire :** Formule : $C_{15}H_{13}NO_2$,M=239,25 | | | |
|---|---|---|---|
| Calculé : | C : 75,29 % | H : 5,47 % | N : 5,85 % |
| Trouvé : | C : 75,03 % | H : 5,73 % | N : 5,79 % |

*Stade C : N-(2,3-dihydro-1-H-phénalèn-2-yl)acétamide*

**Mode opératoire :**

La cétone obtenue au stade précédent (2,08 mmol, 0,5 g) est solubilisée dans 35 $cm^3$ d'acide acétique et après plusieurs purges à l'argon, on ajoute le Pd/C 10 % (0,25 g) et on place le milieu réactionnel sous atmosphère d'$H_2$. On maintient l'agitation 5h à température ambiante et le palladium est filtré sur célite. L'acide acétique est évaporé à sec et le résidu chromatographié sur colonne de gel de silice (granulométrie 60-200 µm) ; éluant : $CH_2Cl_2$ : MeOH 98:2 v/v. On isole ainsi le composé du titre ainsi que le N-(1-hydroxy-2,3-dihydro-1H-phénalèn-2-yl)acétamide.

- N-(2,3-dihydro-1H-phénalèn-2-yl)acétamide

Rendement : 30% (Le rendement est de 76 % après 21h de réaction dans les mêmes conditions)
CCM (silice) : $R_f$ = 0,73 (MeOH : $CH_2Cl_2$ 5 : 95)

F = 180-181°C ($CH_2Cl_2$/éther de pétrole)

RMN $^1H$ ($CDCl_3$) δ (ppm) : 1,86 (s, 3H, $COCH_3$) ; 3,06-3,16 (q, 1H, $J_{AX}$=6Hz, $J_{AB}$=-16Hz, AB d'un système ABX $CH\underline{CH_2}$) ; 3,32-3,42 (q, 1H, $J_{BX}$=3,5Hz, $J_{AB}$=-15,9 Hz, AB d'un système ABX $CH\underline{CH_2}$) ; 4,63-4,73 (m, 1H, CH) ; 5,6 (sl, 1H, NH) ; 7,24 (d, 2H, J=7Hz, $H_{ar}$) ; 7,41 (t, 2H, J = 7Hz, $H_{ar}$) ; 7,72 (d, 2H, J=8,3 Hz, $H_{ar}$).

RMN $^{13}C$ ($CDCl_3$) δ (ppm) : 23,28 ($COCH_3$) ; 36,31 (2 $CH_2$) ; 43,95 ($\underline{CH}NH$) ; 125,27 ($C_{ar}$) ; 125,68 ($C_{ar}$) ; 126,34 ($C_{ar}$) ; 132,41 ($C_{ar}$) ; 169,68 ($COCH_3$).

| Analyse élémentaire : Formule : $C_{15}H_{15}NO$, M=225,27 | | | |
|---|---|---|---|
| Calculé : | C : 79,9 % | H : 6,71 % | N : 6,21 % |
| Trouvé : | C : 78,54% | H : 6,85% | N : 6,12% |

- N-(1-hydroxy-2,3-dihydro-1H-phenalèn-2-yl)acétamide

Rendement : 55 %
CCM (silice) : Rf : 0,43 (MeOH : $CH_2Cl_2$ 5 : 95)
F: 205-206°C

**EXEMPLE 2 : N-(4-METHOXY-2,3-DIHYDRO-1H-PHENALEN-2-YL)ACETAMIDE**

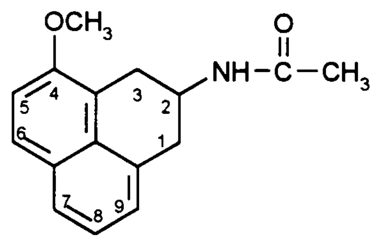

***Stade A : 2-méthyl-4-[(2-méthoxy-napht-1-yl)méthyl]-oxazolin-5-one***

**Mode opératoire :**

Le composé obtenu dans la préparation 3 (11,11 mmol ; 4g) est solubilisé dans 14 $cm^3$ d'anhydride acétique et le milieu réactionnel est porté à reflux durant 40 min. L'excès d'anhydride est évaporé à sec à la pompe à palette afin d'isoler le composé du titre qui sera utilisé sans autre purification.

RMN $^1H$ ($CDCl_3$) δ (ppm) : 2,1 (s, 3H, $CH_3$) ; 3,55-3,60 (m, 2H, AB d'un système ABX $\underline{CH_2}CH$) ; 3,88 (s, 3H, $OCH_3$) ; 4,42-4,51 (q, 1H, CH) ; 7,18-7,30 (m, 2H, $H_{ar}$) ; 7,42 (t, 1H, $H_{ar}$) ; 7,72 (2 d, 2H, $H_{ar}$) ; 7,83 (d, 1H, $H_{ar}$).

***Stade B : N-(4-méthoxy-1-oxo-2,3-dihydro-1H-phénalen-2-yl) acétamide***

**Mode opératoire :**

Dans un tricol placé sous atmosphère inerte, on introduit 50 $cm^3$ de $Cl_2CHCHCl_2$, le chlorure d'aluminium (42,35 mmol ; 6g) puis au goutte à goutte le composé obtenu au stade précédent dissous dans 90 $cm^3$ du même solvant. On porte à 60°C durant 2h puis le milieu réactionnel est versé sur un mélange eau (17 g)/HCl conc. (1,1 $cm^3$) et

agité durant 1h. La phase organique est récupérée et la phase aqueuse extraite plusieurs fois au $CH_2Cl_2$. Les phases organiques réunies sont séchées sur $MgSO_4$ puis évaporées. Le composé du titre est purifié par chromatographie sur colonne de gel de silice (granulométrie 60-200 μm) ; éluant : EP:$CH_2Cl_2$ 60:30, $CH_2Cl_2$ puis $CH_2Cl_2$:MeOH 99:1 v/v.

Rendement global : 25 %
CCM (silice) : $R_f$ = 0,8 ($CH_2Cl_2$ : MeOH 95 : 5)
RMN $^1H$ ($CDCl_3$) δ (ppm) : 2,07 (s, 3H, $COCH_3$) ; 2,61-2,76 (q, 1H, J $_{AX}$=13,2Hz, J $_{AB}$=16Hz, AB d'un système ABX <u>$CH_2$</u>CH) ; 3,9 (s, 3H, $OCH_3$) ; 4,2-4,32 (q, 1H, J $_{BX}$=7Hz, J $_{AB}$=16Hz, AB d'un système ABX <u>$CH_2$</u>CH) ; 4,84-4,97 (m, 1H CH) ; 6,75 (d, 1H, NH) ; 7,25 (d, 1H, J=9Hz, $H_{ar}$) ; 7,35 (t, 1H, J=8Hz, $H_{ar}$); 7,75 (2 d, 2H, J = 9Hz, $H_{ar}$) ; 8,02 (d, 1H, J=7Hz, $H_{ar}$).

**OPTIMISATION : VOIE DU CHLORURE D'ACIDE**

L'acétamido acide obtenu au stade C de la préparation 3 (5,22 mmol ; 1,5 g) est mis en suspension dans 60 $cm^3$ de dichlorométhane anhydre. On ajoute du chlorure d'oxalyle (5,22 mmol ; 0,455 $cm^3$) à -10°C puis 3 à 4 gouttes de DMF. Le produit se solubilise et la solution jaunit. La température est maintenue 2h à -10°C. On ajoute rapidement $AlCl_3$ (4 éq. ; 0,88 mmol ; 2,78 g) et la solution devient rouge foncé. L'agitation est maintenue strictement à -10°C durant 3h30. Le milieu réactionnel est versé sur un mélange eau/glace/HCl con. (0,5 $cm^3$) et agité vigoureusement. Les phases sont séparées et la phase organique est lavée 1 fois à l'eau puis avec une solution NaOH (1N). La phase aqueuse est lavée plusieurs fois avec du dichlorométhane puis les phases organiques réunies sont séchées sur $MgSO_4$ et évaporées. La poudre orangée isolée est reprise dans l'acétone et filtrée (le filtrat peut être évaporé, repris avec un minimum d'acétone et de l'éther pour optimiser le rendement en produit attendu). Le produit est utilisé sans autre purification.

***Stade C : N-(4-méthoxy-2,3-dihydro-1H-phénalen-2-yl)acétamide***

**Mode opératoire :**

Le composé obtenu au stade précédent (3,71 mmol ; 1g) est solubilisée dans 85 $cm^3$ d'acide acétique et après plusieurs purges à l'argon on ajoute le Pd/C 10 % (0,74 g) et on place le milieu réactionnel sous atmosphère d'$H_2$. On maintient l'agitation 28h à température ambiante et on filtre le palladium sur célite. L'acide acétique est évaporé à sec et le résidu purifié par chromatographie sur colonne de gel de silice (granulométrie 60-200 μ m) ; éluant : $CH_2Cl_2$:MeOH 99:1 v/v. On isole le composé du titre pur sous forme d'une poudre blanche.

Rendement : 76 %
CCM (silice) : $R_f$ = 0,74 ($CH_2Cl_2$ : MeOH 95 : 5)
F = 196-197°C
RMN $^1H$ ($CDCl_3$) δ (ppm) : 1,86 (s, 3H, $COCH_3$) ; 3,01-3,34 (m, 4H, 2 AB des systèmes ABX <u>$CH_2$</u>CH) ; 3,93 (s, 3H, $OCH_3$) ; 4,62-4,71 (m, 1H, CH) ; 5,5 (d, 1H, NH) ; 7,19-7,3 (m, 3H, $H_{ar}$) ; 7,65 (d d, 2H, J=7Hz, $H_{ar}$) ; 7,73 (d, 1H, J=9Hz, $H_{ar}$).

| **Analyse élémentaire :** Formule : $C_{16}H_{17}NO_2$,M=225,3 | | | |
|---|---|---|---|
| Calculé : | C : 75,26 % | H : 6,71 % | N : 5,48 % |
| Trouvé : | C : 75,14% | H : 6,72% | N : 5,53% |

**EXEMPLE 3 : N-(4-METHOXY-2,3-DIHYDRO-1H-PHENALEN-2-YL)PROPIONAMIDE**

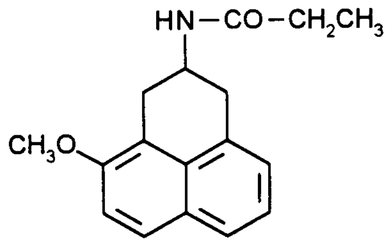

**Mode opératoire :**

Le composé obtenu lors de la préparation 4 (0,56 mmol ; 0,120 g) est dissous dans 5 $cm^3$ de dichlorométhane en présence de triéthylamine (0,84 mmol ; 0,118 $cm^3$ ; 1,5 eq.). et de chlorure de propanoyle. L'agitation est maintenue 40 min à température ambiante. Le milieu est lavé 2 fois à l'eau puis la phase organique est séchée sur $MgSO_4$ et évaporée. Le résidu est chromatographié sur colonne de gel de silice (granulométrie 60-200 μm) ; (éluant : $CH_2Cl_2$ puis $CH_2Cl_2$ : MeOH 95:5 v/v) pour donner le composé du titre pur.

Rendement : 66 %
CCM (silice) : $R_f$ = 0,81 (MeOH : $CH_2Cl_2$ 5:95)
F=184-186°C ($CH_2Cl_2/Et_2O$)
RMN $^1H$ ($CDCl_3$) δ (ppm) : 1,07 (t, 3H, J=7,6, $\underline{CH_3}CH_2$) ; 2,03 (q, 2H, J=7,6 Hz, $\underline{CH_2}CH_3$) ; 3,03-3,35 (m, 4H, 2 AB d'un système ABX $\underline{CH_2}CH$) ; 3,93 (s, 3H, $OCH_3$) ; 4,65-4,68 (m, 1H, CH) ; 5,45 (d, 1H, NH) ; 7,18-7,29 (m, 3H, $H_{ar}$) ; 7,65 (d, 1H, J=7,5 Hz, $H_{ar}$) ; 7,73 (d, 1H, J=9Hz, $H_{ar}$).
RMN $^{13}C$ ($CDCl_3$) δ (ppm) : 9,79 $\underline{C}H_3CH_2$) ; 29,66 et 29,74 ($\underline{C}H_2CH_3$ et $C_1$) 36,12 ($C_3$) ; 43,28 ($C_2$) ; 56,16 ($OCH_3$) ; 112,91, 117,24, 123,44 125,72, 126,26, 127,28, 128,44, 130,2, 131,52, 153,78 ($C_{ar}$), 177,22 (CO).

| **Analyse élémentaire :** Formule: $C_{17}H_{19}NO_2$,M=269,32 | | | |
|---|---|---|---|
| Calculé : | C : 75,8 % | H : 7,11 % | N : 5,19 % |
| Trouvé : | C : 75,72 % | H : 7,06 % | N : 5,14 % |

**<u>EXEMPLE 4 A 19</u> :**

En procédant comme dans l'exemple 1, mais en partant des préparations 5 à 20, on obtient les composés des exemples suivants :

**EXEMPLE 4 : N-(2,3-DIHYDRO-1H-PHENALEN-2-YL)PROPIONAMIDE**

**Deuxième mode opératoire :**

A une solution d'amine de la préparation 2 (0,7 mmol, 140 mg) dans 7 $cm^3$ de $CH_2Cl_2$ anhydre en présence de $Et_3N$ (1 mmol ; 0,16 $cm^3$) est ajouté à 0°C le chlorure de propionyle (0,8 mmol ; 0,07 $cm^3$). L'agitation est maintenue 1h à température ambiante puis le milieu est lavé deux fois à l'eau. La phase organique est séchée sur $MgSO_4$ puis évaporée. Le résidu est lavé à l'éther et le précipité est filtré pour donner 70 mg du produit pur attendu.

Rendement : 39 %
CCM (silice) : Rf=0,72 (MeOH:$CH_2Cl_2$ 5:95)

F = 148°C

RMN $^1H$ ($CDCl_3$) $\delta$ (ppm) : 1,06 (t, 3H, $CH_2CH_3$) ; 2,06 (q, 2H, $CH_2CH_3$) ; 3,05-3,16 (dd, 2H, J AX=6Hz, J AB = -16Hz, AB des systèmes ABX) ; 3,33-3,42 (dd, 2H, J=8Hz, $H_4$ et $H_9$) ; 7,39 (t, 2H, J = 9Hz, $H_8$ et $H_5$) ; 7,71 (d, 2H, J=9Hz, $H_6$ et $H_7$).

| Analyse élémentaire<br>Formule : $C_{16}H_{17}NO$<br>M = 239,41 | | | |
|---|---|---|---|
| | C % | H % | N % |
| Calculé | 80,29 | 7,16 | 5,85 |
| Trouvé | 72,17 | 6,88 | 5,53 |

**EXEMPLE 5 : N-(2,3-DIHYDRO-1H-PHENALEN-2-YL)BUTYRAMIDE**

**EXEMPLE 6: N-(2,3-DIHYDRO-1H-PHENALEN-2-YL)PENTANAMIDE**

**EXEMPLE 7 : N-(2,3-DIHYDRO-1H-PHENALEN-2-YL)HEXANAMIDE**

**EXEMPLE 8 : N-(2,3-DIHYDRO-1H-PHENALEN-2-YL)HEPTANAMIDE**

**EXEMPLE 9: N-(2,3-DIHYDRO-1H-PHENALEN-2-YL)-2-IODOACETAMIDE**

**EXEMPLE 10 : N-(2,3-DIHYDRO-1H-PHENALEN-2-YL)-2-BROMOACETAMIDE**

**EXEMPLE 11: N-(2,3-DIHYDRO-1H-PHENALEN-2-YL)TRIFLUOROACETAMIDE**

**EXEMPLE 12 : N-(2,3-DIHYDRO-1H-PHENALEN-2-YL)ISOPENTANAMIDE**

**EXEMPLE 13 : N-(2,3-DIHYDRO-1H-PHENALEN-2-YL)ISOBUTYRAMIDE**

**EXEMPLE 14 : N-(2,3-DIHYDRO-1H-PHENALEN-2-YL)CYCLOPROPYLCARBOXAMIDE**

**EXEMPLE 15 : N-(2,3-DIHYDRO-1H-PHENALEN-2-YL)CYCLOBUTYLCARBOXAMIDE**

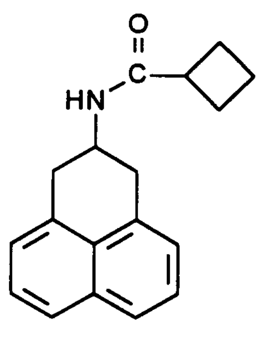

**Deuxième mode opératoire :**

A une solution d'amine de la préparation 2 (2 mmol, 350 mg) dans 7 $cm^3$ de $CH_2Cl_2$ anhydre en présence de $Et_3N$ (0,4 $cm^3$ ; 1,5 eq) est ajouté à 0°C le chlorure de l'acide cyclobutylcarboxylique (2 mmol ; 0,24 $cm^3$). L'agitation

est maintenue 1h à température ambiante puis le milieu est lavé deux fois à l'eau. La phase organique est séchée sur $MgSO_4$ puis évaporée. Le résidu est purifié par chromatographie sur colonne de gel de silice (granulométrie 60 200 μm) ; éluant $CH_2Cl_2$:MeOH 99:1. Le composé attendu est isolé et recristallisé dans un mélange $CH_2Cl_2$:Ether de pétrole pour donner 200 mg de l'amide pur attendu.

Rendement : 40%
CCM (silice) : Rf=0,89 (MeOH:$CH_2Cl_2$ 5:95)
F = 173°C
RMN $^1H$ ($CDCl_3$) δ (ppm) : 1,79-2,25 (m, 6H, 3x$CH_2$) ; 2,77-2,86 (m, 1H, CH) ; 3,03-3,14 (dd, 2H, $J_{AX}$=6Hz, $J_{AB}$=-16Hz, AB d'un système ABX) ; 3,33-3,42 (dd, 2H, $J_{BX}$=4Hz, $J_{AB}$=-16Hz, AB d'un système ABX) ; 4,65-4,69 (m, 1H CH) ; 5,29-5,42 (s, 1H, NH) ; 7,24 (d, 2H, J = 7Hz, $H_4$ et $H_9$) ; 7,4 (t, 2H, J=9Hz, $H_5$ et $H_8$) ; 7,71 (d, 2H, J=9Hz, $H_6$ et $H_7$).

| Analyse élémentaire<br>Formule : $C_{18}H_{19}NO$<br>M = 265,34 | | | |
|---|---|---|---|
| | C % | H % | N % |
| Calculé | 81,47 | 7,22 | 5,28 |
| Trouvé | 79,35 | 7,35 | 5,09 |

**EXEMPLE 16 : N-(2,3-DIHYDRO-1H-PHENALEN-2-YL)CYCLOHEXYLCARBOXAMIDE**

**EXEMPLE 17 : N-(2,3-DIHYDRO-1H-PHENALEN-2-YL)CYCLOPROPYLACETAMIDE**

**EXEMPLE 18 : N-(2,3-DIHYDRO-1H-PHENALEN-2YL)BENZYLCARBOXAMIDE**

**EXEMPLE 19 : N-(2,3-DIHYDRO-1H-PHENALEN-2-YL)PHENYLCARBOXAMIDE**

**EXEMPLES 20 A 25 :**

En procédant comme dans l'exemple 3, mais en utilisant le chlorure d'acyle approprié, on obtient les composés des exemples suivants :

**EXEMPLE 20 : N-(4-METHOXY-2,3-DIHYDRO-1H-PHENALEN-2-YL)ISOBUTYRAMIDE**

**EXEMPLE 21 : N-(4-METHOXY-2,3-DIHYDRO-1H-PHENALEN-2-YL)BUTYRAMIDE**

**EXEMPLE 22 : N-(4-METHOXY-2,3-DIHYDRO-1H-PHENALEN-2-YL)HEXANAMIDE**

**EXEMPLE 23 : N-(4-METHOXY-2,3-DIHYDRO-1H-PHENALEN-2-YL)TRIFLUOROACETAMIDE**

**EXEMPLE 24 : N-(4-METHOXY-2,3-DIHYDRO-1H-PHENALEN-2-YL)CYCLOBUTYLCARBOXAMIDE**

**EXEMPLE 25 : N-(4-METHOXY-2,3-DIHYDRO-1H-PHENALEN-2-YL)CYCLOPROPYLCARBOXAMIDE**

**EXEMPLES 26 A 36 :**

En procédant comme dans l'exemple 3, mais en remplaçant le chlorure de propanoyle par l'isocyanate ou l'isothiocyanate approprié, on obtient les composés des exemples suivants :

**EXEMPLE 26 : N-(4-METHOXY-2,3-DIHYDRO-1H-PHENALEN-2-YL)N'-METHYLUREE**

**EXEMPLE 27 : N-(4-METHOXY-2,3-DIHYDRO-1H-PHENALEN-2-YL)N'-ETHYLUREE**

**EXEMPLE 28 : N-(4-METHOXY-2,3-DIHYDRO-1H-PHENALEN-2-YL)N'-PROPYLUREE**

**EXEMPLE 29 : N-(4-METHOXY-2,3-DIHYDRO-1H-PHENALEN-2-YL)N'-BUTYLUREE**

**EXEMPLE 30 : N-(4-METHOXY-2,3-DIHYDRO-1H-PHENALEN-2-YL)N'-HEXYLUREE**

**EXEMPLE 31 : N-(4-METHOXY-2,3-DIHYDRO-1H-PHENALEN-2-YL)N'-CYCLOPROPYLUREE**

**EXEMPLE 32 : N-(4-METHOXY-2,3-DIHYDRO-1H-PHENALEN-2-YL)N'-CYCLOBUTYLUREE**

**EXEMPLE 33 : N-(4-METHOXY-2,3-DIHYDRO-1H-PHENALEN-2-YL)N'-CYCLOHEXYLUREE**

**EXEMPLE 34 : N-(4-METHOXY-2,3-DIHYDRO-1H-PHENALEN-2-YL)N'-METHYLTHIOUREE**

**EXEMPLE 35 : N-(4-METHOXY-2,3-DIHYDRO-1H-PHENALEN-2-YL)N'-PROPYLTHIOUREE**

**EXEMPLE 36 : N-(4-METHOXY-2,3-DIHYDRO-1H-PHENALEN-2-YL)N'-CYCLOPROPYL THIOUREE**

**EXEMPLE 37 : N-(2,3-DIHYDRO-1H-PHENALEN-2-YL)PROPIONAMIDE**

(2ème procédé)

En procédant comme dans l'exemple 3, mais en partant de la préparation 2, on obtient le composé du titre.

**<u>EXEMPLES 38 A 46</u> :**

En procédant comme dans l'exemple 3, mais en partant des préparations 36 à 38 et en utilisant les chlorures d'acyle appropriés, on obtient les composés suivants :

**EXEMPLE 38 : N-ETHYL N-(4-METHYL-2,3-DIHYDRO-1H-PHENALEN-2-YL)ACETAMIDE**

**EXEMPLE 39 : N-ETHYL N-(4-METHYL-2,3-DIHYDRO-1H-PHENALEN-2-YL)PROPIONAMIDE**

**EXEMPLE 40 : N-ETHYL N-(4-METHYL-2,3-DIHYDRO-1H-PHENALEN-2-YL)CYCLOPROPYL CARBOXAMIDE**

**EXEMPLE 41 : N-METHYL N-(2,3-DIHYDRO-1H-PHENALEN-2-YL)ACETAMIDE**

**EXEMPLE 42 : N-METHYL N-(2,3-DIHYDRO-1H-PHENALEN-2-YL)PROPIONAMIDE**

**EXEMPLE 43 : N-METHYL N-(2,3-DIHYDRO-1H-PHENALEN-2-YL)CYCLOPROPYL CARBOXAMIDE**

**EXEMPLE 44 : N-(5-METHOXY-2,3-DIHYDRO-1H-PHENALEN-2-YL)ACETAMIDE**

**EXEMPLE 45 : N-(5-METHOXY-2,3-DIHYDRO-1H-PHENALEN-2-YL)PROPIONAMIDE**

**EXEMPLE 46 : N-(5-METHOXY-2,3-DIHYDRO-1H-PHENALEN-2-YL)CYCLOPROPYL CARBOXAMIDE**

**EXEMPLE 47 : N-(4,9-DIMETHOXY-2,3-DIHYDRO-1H-PHENALEN-2-YL)ACETAMIDE**

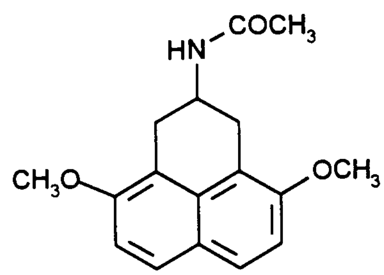

***Stade A : N-(1-oxo-4,9-diméthoxy-2,3-dihydro-1H-phénalen-2-yl)acétamide***

Le composé obtenu à la préparation 33 (5 mmol ; 1,65 g) est solubilisé dans 10 $cm^3$ d'anhyride acétique et le milieu réactionnel est porté au reflux du solvant durant 40 min. L'excès d'anhydride est évaporé à sec à la pompe à palette afin d'isoler la 2-méthyl-4-[(2,7-diméthoxynapht-1-yl)méthyl]-oxazolin-5-one utilisée sans autre purification. Dans un tricol placé sous atmosphère inerte on introduit 30 $cm^3$ de $CH_2Cl_2$ anhydre, le chlorure d'aluminium (18 mmol ; 2,37g) puis au goutte à goutte la 2-méthyl-4-[(2,7-diméthoxynapht-1-yl)méthyl]-oxazolin-5-one (6 mmol, 1,78g) dissoute dans 40 $cm^3$ du même solvant. On porte à 60°C durant 2h puis après refroidissement le milieu réactionnel est versé sur un mélange glace/HCl concentré et agité durant 1h. La phase organique est récupérée et la phase aqueuse extraite plusieurs fois au $CH_2Cl_2$. Les phases organiques réunies sont séchées sur $MgSO_4$ puis évaporées. Le résidu est purifié par chromatographie sur colonne de gel de silice (granulométrie 60-200 μm) ; éluant : $CH_2Cl_2$ puis $CH_2Cl_2$:MeOH 99:1 v/v. On obtient, isolés, le N-(1-oxo-4,9-diméthoxy-2,3-dihydro-1H-phénalèn-2-yl)acétamide (α) et le N-(1-oxo-9-hydroxy-4-méthoxy-2,3-dihydro-1H-phénalène-2-yl)acétamide (β).

<u>α</u> :

Rendement : 10 %
F > 260°C
RMN $^1H$ ($CDCl_3$) δ (ppm) : 2,11 (s, 3H, $COCH_3$) ; 2,59-2,74 (m, 1H, AB d'un système ABX <u>$CH_2$</u>CH) ; 3,94 (s, 3H, $OCH_3$) ; 4,04 (s, 3H, $OCH_3$) ; 4,19-4,30 (dd, 1H, $J_{BX}$=7Hz, $J_{AB}$=16Hz, AB d'un système ABX <u>$CH_2$</u>CH) ; 4,88 (m, 1H, CH) ; 6,97 (s, 1H, NH) ; 7,12-7,18 (2d, 2H, J=9Hz, $H_{ar}$) ; 7,71 (d 1H, J=9Hz, $H_{ar}$) ; 7,96 (d, 1H, J=9Hz, $H_{ar}$).

<u>β</u> :

Rendement : 12 %

F > 260°C

RMN $^1$H ($CDCl_3$) δ (ppm) : 2,15 (s, 3H, $COCH_3$) ; 2,67-2,74 (dd, 1H, AB d'un système ABX $\underline{CH_2}CH$) ; 3,94 (s, 3H, $OCH_3$) ; 4,17-4,22 (dd, 1H, $J_{BX}$=8Hz, $J_{AB}$=16Hz, AB d'un système ABX $\underline{CH_2}CH$) ; 4,94-4,97 (m, 1H, CH) ; 6,52 (sl, 1H, NH) ; 6,97 (d, 1H, J=9Hz, $H_8$) ; 7,12 (d 1H, J=9Hz, $H_5$) ; 7,67 (d, 1H, J=9Hz, $H_7$) ; 7,90 (d, 1H, J=9Hz, $H_6$) ; 12,35 (s, 1H, OH).

***Stade B : N-(4,9-diméthoxy-2,3-dihydro-1H-phénalén-2-yl)acétamide***

**Mode opératoire :**

Le N-(1-oxo-4,9-diméthoxy-2,3-dihydro-1H-phénalèn-2-yl)acétamide obtenu au stade précédent (0,6 mmol ; 0,17 g) est solubilisé dans 25 $cm^3$ d'acide acétique et après plusieurs purges à l'argon, le Pd/C 10 % (85 mg) est ajouté et le milieu réactionnel placé sous atmosphère d'$H_2$. L'agitation est maintenue 28h à température ambiante et le palladium est filtré sur célite. L'acide acétique est évaporé à sec et le résidu purifié par chromatographie sur colonne de gel de silice (granulométrie 60-200 µm) ; éluant $CH_2Cl_2$:MeOH 99:1 v/v. pour donner le produit attendu.

Rendement : 43 %

CCM (silice) : Rf=0,35 ($CH_2Cl_2$:MeOH 95:5)

F = 254°C

RMN $^1$H (DMSO- $d_6$) δ (ppm) : 1,83 (s, 3H, $COCH_3$) ; 2,53-2,60 (dd, 2H, $J_{AX}$=10Hz, $J_{AB}$=16Hz, AB d'un système ABX $\underline{CH_2}CH$) ; 3,23-3,28 (dd, 2H, $J_{BX}$=4Hz, $J_{AB}$=16Hz, AB d'un système ABX $\underline{CH_2}CH$) ; 3,86 (s, 6H, $2xOCH_3$) ; 3,89-3,95 (m, 1H, CH) ; 7,22 (d, 1H, J=9Hz, $H_5$ et $H_8$) ; 7,71 (d, 2H, J=9Hz, $H_6$ et $H_7$) ; 7,99 (d, 1H, NH).

RMN $^{13}$C (DMSO-$d_6$) δ (ppm) : 22,77 ($COCH_3$) ; 29,02 (2 $CH_2$) ; 43,64 ($\underline{CH}NH$) ; 55,58 (2 $OCH_3$) ; 111,12 ($C_{ar}$) ; 116,74 ($C_{ar}$) ; 123,7 ($C_{ar}$) ; 127,05 ($C_{ar}$) ; 130,7 ($C_{ar}$) ; 153,07 ($C_{ar}$) ; 168,83 ($COCH_3$).

| Analyse élémentaire<br>Formule : $C_{17}H_{19}NO_3$<br>M = 255,3 | | | |
|---|---|---|---|
| | C % | H % | N % |
| Calcul | 71,55 | 6,71 | 4,91 |
| Trouvé | 70,59 | 6,79 | 4,80 |

**EXEMPLES 48 ET 49 :**

En procédant comme dans l'exemple 47 mais utilisant les réactifs d'acylation appropriés, on obtient les composés des exemples suivants :

**EXEMPLE 48 : N-(4,9-DIMETHOXY-2,3-DIHYDRO-1H-PHENALEN-2-YL)PROPIONAMIDE**

**EXEMPLE 49 : N-(4,9-DIMETHOXY-2,3-DIHYDRO-1H-PHENALEN-2-YL)CYCLOPROPYLCARBOXAMIDE**

**EXEMPLES 50 A 61 :**

En procédant comme dans l'exemple 1 mais en partant des préparations 21 à 32, on obtient les composés des exemples suivants :

**EXEMPLE 50 : N-(1, 3, 4, 5-TETRAHYDRO-BENZO[cd]INDOL-4-YL)ACETAMIDE**

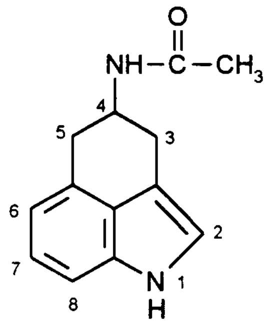

**Exemple 50**

**EXEMPLE 51 : N-(1, 3, 4, 5-TETRAHYDRO-6-METHOXY-BENZO[cd]INDOL-4-YL)ACETAMIDE**

**EXEMPLE 52 : N-(1, 3, 4, 5-TETRAHYDRO-6-BROMO-BENZO[cd]INDOL-4-YL)ACETAMIDE**

**EXEMPLE 53 : N-(1, 3, 4, 5-TETRAHYDRO-6-ETHYL-BENZO[cd]INDOL-4-YL)ACETAMIDE**

**EXEMPLE 54 : N-(3, 4, 5-TRIHYDRO-BENZO[cd]BENZOFURAN-4-YL)ACETAMIDE**

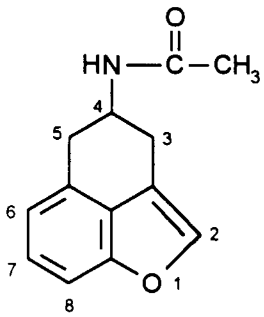

**Exemple 54**

**EXEMPLE 55 : N-(3, 4, 5-TRIHYDRO-6-METHOXY-BENZO[cd]BENZOFURAN-4-YL)ACETAMIDE**

**EXEMPLE 56 : N-(3, 4, 5-TRIHYDRO-6-BROMO-BENZO[cd]BENZOFURAN-4-YL)ACETAMIDE**

**EXEMPLE 57 : N-(3, 4, 5-TRIHYDRO-6-ETHYL-BENZO[cd]BENZOFURAN-4-YL)ACETAMIDE**

**EXEMPLE 58 : N-(3, 4, 5-TRIHYDRO-BENZO[cd[BENZOTHIOPHEN-4-YL)ACETAMIDE**

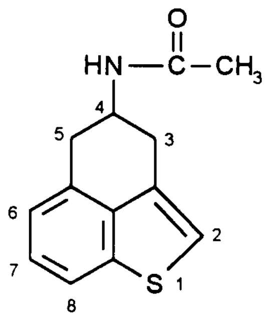

**Exemple 58**

**EXEMPLE 59 : N-(3, 4, 5-TRIHYDRO-6-METHOXY-BENZO[cd]BENZOTHIOPHEN-4-YL)ACETAMIDE**

**EXEMPLE 60 : N-(3, 4, 5-TRIHYDRO-6-BROMO-BENZO[cd]BENOTHIOPHEN-4-YL)ACETAMIDE**

**EXEMPLE 61 : N-(3, 4, 5-TRIHYDRO-6-ETHYL-BENZO[cd]BENOTHIOPHEN-4-YL)ACETAMIDE**

**EXEMPLE 62 : N-(4-METHOXY-9-HYDROXY-2,3-DIHYDRO-1H-PHENALEN-2-YL) ACETAMIDE**

**Mode opératoire :**

A partir de 0,7 mmol (200 mg) de N-(1-oxo-9-hydroxy-4-méthoxy-2,3-dihydro-1H-phénalèn-2-yl)acétamide obtenu au stade A de l'exemple 47 et de 100 mg de Pd/C 10 % dans 12 $cm^3$ d'acide acétique on obtient, dans les mêmes conditions que dans le stade B de l'exemple 47 le composé du titre qui est purifé par simple cristallisation dans le dichlorométhane.

Rendement : 37 %
CCM (silice) : Rf=0,18 ($CH_2Cl_2$:MeOH 95:5)
F > 254°C
RMN $^1H$ ($CD_3COCD_3$) δ (ppm) : 1,86 (s, 3H, $COCH_3$) ; 267-2,79 (dd, 2H, AB d'un système ABX $\underline{CH_2}$CHCH) ; 2,99 (sl, 1H, OH) ; 3,33-3,41 (dd, 2H, AB d'un système ABX $\underline{CH_2}$CH) ; 3,89 (s, 3H, $OCH_3$) : 4,18-4,32 (m, 1H, CH) ; 7,01 (d, 1H, J=9Hz, $H_8$) ; 7,12 (d, 1H, J=9Hz, $H_5$); 7,25 (sl, 1H, NH) ; 7,50 (d, 1H, J=9Hz, $H_7$) ; 7,62 (d, 1H, J=9Hz, $H_6$).
RMN $^{13}C$ (DMSO- $d_6$) δ (ppm) : 22,79 ($COCH_3$) ; 29,16 (2 $CH_2$) ; 43,67 ($\underline{CH}$NH) ; 55,90 ($OCH_3$) ; 109,94 ($C_{ar}$) ; 111,12 ($C_{ar}$) ; 113,78 ($C_{ar}$) ; 116,55 ($C_{ar}$) ; 123,0 ($C_{ar}$) ; 126,47 ($C_{ar}$) ; 126,58 ($C_{ar}$) ; 130,1 ($C_{ar}$) ; 131,07 ($C_{ar}$) ; 151,29 ($C_{ar}$) ; 152,96 ($C_{ar}$) ; 168,74 ($COCH_3$).

**EXEMPLE 63 : 10-METHOXY-2-ACETAMIDO-1,2,3,4-TETRAHYDROPHENANTHRENE**

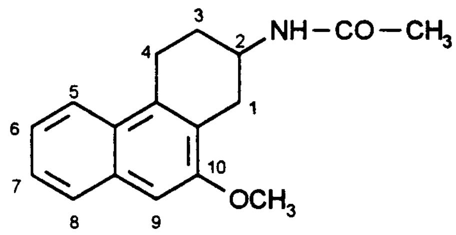

Le composé obtenu dans la préparation 39 (0, 11g, 0,5 mmol) est dissous dans le dichlorométhane en présence de triéthylamine (1,2 equiv.). Le chlorure d'acétyle est ajouté à 0°C et le milieu réactionnel est mis sous agitation pendant 1h à température ambiante. Le composé attendu est purifié par chromatographie sur colonne de silice (éluant : 5 % méthanol : $CH_2Cl_2$).

Rendement : 56 %
RMN $^1H$ (200 MHz, $CDCl_3$) : δ 7,81 (d, 1H), 7.64 (d, 1H), 7,38-7,26 (m, 2H), 6,9 (s, 1H), 5,34 (d, 1H), 4,33-4,2 (m, 1H), 3,83 (s, 3H), 3,1-3,04 (m, 2H), 2,67-2,44 (m, 2H), 2,11-2,01 (m, 1H), 1,99-1,7 (m, 1H), 1,90 (s, 3H), 1,83-1,69 (m, 1H)

**EXEMPLES 64 A 67 :**

En procédant comme dans l'exemple 63 mais en utilisant les réactifs d'acylation appropriés, on obtient les composés des exemples suivants :

**EXEMPLE 64 : 10-METHOXY-2-PROPIONAMIDO-1,2,3,4-TETRAHYDRO-PHENANTHRENE**

**EXEMPLE 65 : 10-METHOXY-2-TRIFLUOROACETAMIDO-1,2,3,4-TETRAHYDROPHENANTHRENE**

**EXEMPLE 66 : 10-METHOXY-2-CYCLOPROPYLCARBOXAMIDO-1,2,3,4-TETRAHYDROPHENANTHRENE**

**EXEMPLE 67 : 10-METHOXY-2-CYCLOBUTYLCARBOXAMIDO-1,2,3,4-TETRAHYDROPHENANTHRENE**

**EXEMPLE 68 : 10-METHOXY-3-ACETAMIDO-1,2,3,4-TETRAHYDROPHENANTHRENE**

En procédant comme dans l'exemple 63 mais en partant de la préparation 40, on obtient le composé du titre.

**EXEMPLE 69 : 9-METHOXY-2-ACETAMIDO-1,2,3,4-TETRAHYDROANTHRACENE**

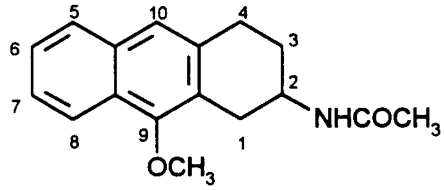

En procédant comme dans l'exemple 63 mais en partant de la préparation 41, on obtient le composé du titre.

**EXEMPLE 70 A 73 :**

En procédant comme dans l'exemple 69, mais en utilisant les réactifs d'acylation appropriés, on obtient les composés des exemples suivants :

**EXEMPLE 70 : 9-METHOXY-2-PROPIONAMIDO-1,2,3,4-TETRAHYDROANTHRACENE**

**EXEMPLE 71 : 9-METHOXY-2-TRIFLUOROACETAMIDO-1,2,3,4-TETRAHYDROANTHRACENE**

**EXEMPLE 72 : 9-METHOXY-2-CYCLOPROPYLCARBOXAMIDO-1,2,3,4-TETRAHYDROANTHRACENE**

**EXEMPLE 73 : 9-METHOXY-2-CYCLOBUTYLCARBOXAMIDO-1,2,3,4-TETRAHYDROANTHRACENE**

**EXEMPLE 74 : N-(1-METHOXY-2,3-DIHYDRO-1H-PHENALEN-2-YL-)ACETAMIDE**

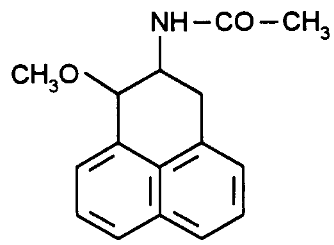

Le N-(1-hydroxy-2,3-dihydro-1H-phénalèn-2-yl)acétamide obtenu au stade C de l'exemple 1 (1 mmol, 290 mg) est mis en solution dans 10 $cm^3$ de DMF anhydre. Sous atmosphère inerte le NaH (1 mmol, 0,05 g suspension à 60 % dans l'huile), puis l'iodure de méthyle (0,09 $cm^3$, 1,2 eq.) sont ajoutés au milieu réactionnel. L'agitation est maintenue 4h30 à température ambiante puis le solvant est évaporé. Le résidu est repris dans du $CH_2Cl_2$ et la solution est filtrée. Le filtrat est évaporé et purifié par chromatographie sur colonne de gel de silice (granulométrie 60-200 µm) : éluant : $CH_2Cl_2$ : MeOH 99:1 v/v. On isole le composé du titre qui est un produit amorphe, mélange de diastéréoisomères.

CCM (silice) : Rf=0,64 (MeOH : $CH_2Cl_2$ 5:95)
RMN $^1H$ ($CDCl_3$) $\delta$ (ppm) : 1,78 (s, 3H, $COCH_3$) 2,93-3,03 (m, 1H, AB d'un système ABX $CH\underline{CH_2}$) ; 3,42 (s, 3H, $OCH_3$) ; 3,66-3,77 (m, 1H, AB d'un système ABX $CH\underline{CH_2}$) ; 4,48 (d, 1H, CHOMe) ; 4,78-4,90 (m, 1H, CH) ; 5,18 (s, 1H, NH) ; 7,32-7,55 (m, 4H, $H_{ar}$) ; 7,79 (d, 1H, $H_{ar}$) ; 7,88 (d, 1H, $H_{ar}$).

**ETUDE PHARMACOLOGIQUE**

**EXEMPLE A : ETUDE DE LA TOXICITE AIGUE**

La toxicité aigüe a été appréciée après administration orale à des lots de 8 souris (26 ± 2 grammes). Les animaux ont été observés à intervalles réguliers au cours de la première journée et quotidiennement pendant les deux semaines suivant le traitement. La $DL_{50}$, entraînant la mort de 50 % des animaux, a été évaluée.
La $DL_{50}$ des produits testés est supérieure à 1 000 $mg.kg^{-1}$ pour la plupart des composés étudiés, ce qui indique la faible toxicité des composés de l'invention.

**EXEMPLE B : ETUDE DE LIAISON AUX RECEPTEURS DE LA MELATONINE**

B1) Etude sur des cellules de la pars tuberalis de mouton

Les études de liaison aux récepteurs de la mélatonine des composés de l'invention ont été réalisées selon les techniques classiques sur les cellules de la pars tuberalis de mouton. La pars tuberalis de l'adénohypophyse est en effet caractérisée, chez les mammifères, par une haute densité en récepteurs de la mélatonine (Journal of Neuroendocrinology 1989, vol. (1), pp 1-4).

PROTOCOLE

1) Les membranes de pars tuberalis de mouton sont préparées et utilisées comme tissu cible dans des expériences de saturation pour déterminer les capacités et affinités de liaison pour la 2-[$^{125}$I]-iodomélatonine.
2) Les membranes de pars tuberalis de mouton sont utilisées comme tissu cible, avec les différents composés à tester, dans des expériences de liaison compétitive par rapport à la iodo-mélatonine.

Chaque expérience est réalisée en triple et une gamme de concentrations différentes est testée pour chaque composé.
Les résultats permettent de déterminer, après traitement statistique, les affinités de liaison du composé testé.

RESULTATS

Il apparaît que les composés de l'invention possèdent une puissante affinité pour les récepteurs de la mélatonine supérieure à celle de la mélatonine elle-même.

B2) Etude sur des membranes de cellules du cerveau de poulet (Gallus domesticus)

Les animaux utilisés sont des poulets (Gallus domesticus) âgés de 12 jours. Ils sont sacrifiés entre 13 et 17 heures le jour de leur arrivée. Les cerveaux sont rapidement prélevés et congelés à -200°C puis conservés à -80°C. Les membranes sont préparées selon la méthode décrite par Yuan et Pang (Journal of Endocrinology 1991, 128, pp 475-482). La 2-[$^{125}$I]-iodomélatonine est incubée en présence des membranes dans une solution tamponnée à pH 7.4 pendant 60 min à 25°C. A l'issue de cette période, la suspension membranaire est filtrée (Whatman GF/C). La radioactivité retenue sur le filtre est déterminée à l'aide d'un compteur à scintillation liquide Beckman ® LS 6000.
Les produits utilisés sont :

- 2-[$^{125}$I-iodomélatonine
- mélatonine
- produits courants
- composés testés

En screening primaire, les molécules sont testées à 2 concentrations ($10^{-7}$ et $10^{-5}$M). Chaque résultat est la moyenne de n=3 mesures indépendantes. Les composés testés font l'objet d'une détermination quantitative de leur efficacité ($IC_{50}$). Ils sont utilisés à 10 concentrations différentes.
Ainsi, les valeurs d'$IC_{50}$ trouvées pour les composés préférés de l'invention, qui correspondent aux valeurs de l'affinité, montrent que la liaison des composés testés est très puissante.
Il s'avère également que les composés de l'invention présentent un profil d'activité original puisque ils apparaissent comme antagonistes sur le modèle de la pars tuberalis d'ovin (Exemple B-1) et agonistes sur le modèle d'aggrégation des mélanophores. (Exemple B-2).

**EXEMPLE B-1 :**

***PROTOCOLE EXPERIMENTAL***

Cette étdude est réalisée sur une culture de cellules de pars tuberalis d'ovin et permet de caractériser l'activité des composés testés sur la production d'AMPc induite par la forskoline. (Morgan et al. J. Molecular Endocrinol 1989, 3, pp R5-R8).
Les composés ($1.10^{-5}$ M) sont testés seuls et sont comparés à la mélatonine ($1.10^{-9}$ M) pour détecter leur capacité à inhiber la production d'AMPc stimulée par la forskolin (1.10-6 M) ou, en combinaison avec la mélatonine, pour détecter une activité antagoniste. Ces expériences sont réalisées en triplicate. Deux indexes de AMPc sont alors déterminés, l'un pour le composé testé seul (AMPc [D]) et l'autre pour le composé testé en combinaison avec la mélatonine (AMPc [D/M]). Ils sont calculés de la façon suivante :

$$AMPc [D] = ([F] - [F/D]) / ([F] - [F/M])$$

AMPc [D/M] = ([F] - [F/D/M]) / ([F] - [F/M])

où

[F] : niveau cellulaire d'AMPc après stimulation par 1 μM de forskoline
[F/D] : niveau cellulaire d'AMPc après stimulation par 1 μM de forskoline en présence du composé à tester (1 ou 10 nM).
[F/M] : niveau cellulaire d'AMPc après stimulation par 1 μM de forskoline en présence de la mélatonine (1 ou 10 μM).
[F/D/M] : niveau cellulaire d'AMPc après stimulation par 1 μM de forskoline en présence de la mélatonine (1 ou 10 nM) et du composé à tester (1 ou 10 μM).

Un composé qui est agoniste pur a un AMPc [D] et un AMPc [D/M] eGaux à 1.

**EXEMPLE B-2 :**

***PROTOCOLE EXPERIMENTAL***

Les crêtes neurales de 20 embryons de Xenopus laevis sont disséquées et dispersées en petits aggregats dans des boîtes de Petri contenant du milieu de culture (L-15 dilué avec 10 % de sérum de veau foetal). Après 7 jours, un certain nombre (20-200) de mélanophores peuvent être visualisés dans la boîte de Petri parmi de nombreuses autres cellules (nerveuses et musculaires par exemple) qui se différencient.

Au repos, les mélanophores sont remplis de façon homogène de granules de pigment lorsque la mélatonine est ajoutée au milieu, les granules de pigment se condensent autour du noyau. La modification de la surface cellulaire occupée par les pigments est quantifiée par imagerie en fonction de la concentration de mélatonine ajoutée. La mélatonine a un $EC_{50}$ (concentration efficace de 50 %) de 10 pM environ. Les composés sont testés pour leur capacité à mimer ou à abolir cette condensation pigmentaire induite par la mélatonine (10 nM).

**EXEMPLE C : TEST DES QUATRE PLAQUES**

Les produits de l'invention sont administrés par voie oesophagienne à des lots de dix souris. Un lot reçoit du sirop de gomme. 30 min après administration des produits à étudier, les animaux sont placés dans des habitacles dont le plancher comprend quatre plaques métalliques. Chaque fois que l'animal passe d'une plaque à l'autre, il reçoit une légère décharge électrique (0,35 mA). Le nombre de passage est enregistré pendant une minute. Après administration, les composés de l'invention augmentent de façon significative le nombre de passages ce qui montre l'activité anxiolytique des dérivés de l'invention.

**EXEMPLE D : COMPOSES DE L'INVENTION SUR LES RYTHMES CIRCADIENS D'ACTIVITE LOCOMOTRICE DU RAT**

L'implication de la mélatonine dans l'entraînement, par l'alternance jour/nuit, de la plupart des rythmes circadiens physiologiques, biochimiques et comportementaux a permis d'établir un modèle pharmacologique pour la recherche de ligands mélatoninergiques.

Les effets des molécules sont testés sur de nombreux paramètres et, en particulier, sur les rythmes circadiens d'activité locomotrice qui représentent un marqueur fiable de l'activité de l'horloge circadienne endogène.

Dans cette étude, on évalue les effets de telles molécules sur un modèle expérimental particulier, à savoir, le rat placé en isolement temporel (obscurité permanente).

<u>PROTOCOLE EXPERIMENTAL</u>

Des rats mâles Long Evans âgés de un mois sont soumis dès leur arrivée au laboratoire, à un cycle lumineux de 12 h de lumière par 24 h (LD 12 : 12).

Après 2 à 3 semaines d'adaptation, ils sont placés dans des cages équipées d'une roue reliée à un système d'enregistrement afin de détecter les phases d'activité locomotrice et de suivre ainsi les rythmes nycthéméraux (LD) ou circadiens (DD).

Dès que les rythmes enregistrés témoignent d'un entraînement stable par le cycle lumineux LD 12 : 12, les rats sont mis en obscurité permanente (DD).

Deux à trois semaines plus tard, lorsque le libre-cours (rythme reflétant celui de l'horloge endogène) est clairement établi, les rats reçoivent une administration quotidienne de la molécule à tester.

Les observations sont réalisées grâce à la visualisation des rythmes d'activité :

- entraînement des rythmes d'activité grâce au rythme lumineux,
- disparition de l'entraînement des rythmes en obscurité permanente,
- entraînement par l'administration quotidienne de la molécule ; effet transitoire ou durable.

Un logiciel permet :

- de mesurer la durée et l'intensité de l'activité, la période du rythme chez les animaux en libre-cours et pendant le traitement,
- de mettre éventuellement en évidence, par analyse spectrale, l'existence de composants circadiens et non circadiens.

RESULTATS :

Il apparaît clairement que les composés de l'invention permettent d'agir de façon puissante sur le rythme circadien via le système mélatoninergique.

**EXEMPLE E : ACTIVITE DES PRODUITS DE L'INVENTION SUR LA MICROCIRCULATION ISCHEMIQUE**

L'étude expérimentale a été réalisée sur les muscles crémasters de rats mâles (Sprague-Dawley) après ligature de l'artère iliaque commune.

Les muscles ont été placés dans une chambre transparente, perfusés par une solution de tampon bicarbonate équilibrée par un mélange gazeux $CO_2/N_2$ 5/95 %. La vélocité des globules rouges et le diamètre des artérioles de premier ou second ordre irriguant le crémaster ont été mesurés, le flux sanguin artériolaire a été calculé. Des informations identiques ont été obtenues pour quatre types de veinules.

On a effectué le même type de mesure simultanément :

- sur le crémaster perfusé normalement,
- sur le crémaster sous ligature, c'est à dire le crémaster ischémié 2, 7, 14 et 21 jours après ligature.

Deux groupes d'animaux ont été étudiés :

- un groupe témoin sans traitement,
- un groupe traité per os par un produit de l'invention, à raison de 0,1 $mg.kg^{-1}$ par jour.

On n'a constaté aucune différence dans la vélocité des globules ni dans le diamètre des vaisseaux dans les muscles crémasters normalement irrigués chez les animaux traités par rapport aux témoins.

Par contre, au niveau du muscle crémaster ischémié, le diamètre moyen des artérioles était amélioré chez les animaux traités par rapport aux témoins. La vélocité des globules rouges était normalisée par un traitement de 21 jours.

En fait, chez les animaux traités, la vélocité des globules rouges et le débit sanguin mesurés 7 jours après la ligature, ne présentent pas de différence significative avec les valeurs obtenues dans le crémaster non ischémié. Ces résultats sont obtenus sans modification de la pression artérielle.

Ces résultats indiquent que le traitement chronique par un composé de l'invention améliore la microcirculation et l'irrigation sanguine des territoires ischémiés.

**EXEMPLE F : STIMULATION DES REPONSES IMMUNITAIRES**

A des groupes de six souris, on a administré des globules rouges de mouton. Ces groupes de souris ont ensuite été traités par voie sous cutanée par les composés de l'invention pendant six jours et un groupe témoin a été traité par un placébo. Les souris sont ensuite laissées au repos pendant quatre semaines puis ont ensuite reçu une injection de rappel de globules rouges de mouton sans recevoir de nouvelles administrations de produit de l'invention. La réponse immunitaire a été évaluée 3 jours après l'injection de rappel. Elle est statistiquement accrue dans le groupe traité par les composés de l'invention.

**EXEMPLE G : INHIBITION DE L'OVULATION**

On utilise des rates femelles adultes avec des cycles réguliers de quatre jours.
Des frottis vaginaux quotidiens ont été réalisés et des rates ont été sélectionnées après qu'elles ont montré au moins deux cycles consécutifs de quatre jours.
Chaque cycle est constitué de deux jours de dioestrus, un jour de proestrus et un jour d'oestrus.
L'après-midi du jour de proestrus, l'hormone lutéinisante est libérée dans le sang par l'hypophyse. Cette hormone induit l'ovulation qui se traduit par la présence d'oeufs au niveau de l'oviducte le jour de l'oestrus. Les composés de l'invention sont administrés par voie orale à midi le jour de l'oestrus. Les rates traitées et témoins sont sacrifiées le jour de l'oestrus. Les oviductes sont examinés. On remarque un pourcentage significatif de diminution du nombre des oeufs dans les oviductes de rates traitées.

**EXEMPLE H : ACTIVITE ANTIARRYTHMIQUE**

PROTOCOLE (ref: Lawson J.N. et al. J. Pharmacol. Exper. Therap. 1968, 160, pp 22-31)

La substance testée est administrée en intrapéritonéal à un groupe de 3 souris 30 min avant l'exposition à une anesthésie par le chloroforme. Les animaux sont ensuite observés pendant 15 min. L'absence d'enregistrement d'arrythmies et de fréquences cardiaques supérieures à 200 battements/min (témoin : 400-480 battements/min) chez les animaux au moins indique une protection significative.

**EXEMPLE I : COMPOSITION PHARMACEUTIQUE : COMPOSES**

1000 comprimés dosés à 5 mg de N-(2,3-dihydro-1H-phénalèn-2-yl)acétamide

| | |
|---|---|
| N-(2,3-dihydro-1H-phénalèn-2-yl)acétamide | 5 g |
| Amidon de blé | 20 g |
| Amidon de maïs | 20 g |
| Lactose | 30 g |
| Stéarate de magnésium | 2 g |
| Silice | 1 g |
| Hydroxypropylcellulose | 2 g |

## Revendications

**1.** Composés de formule (I) :

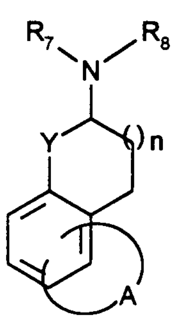

**(I)**

dans laquelle A forme avec le groupement auquel il est lié un groupement tricyclique choisi parmi ($A_1$) à ($A_4$) :

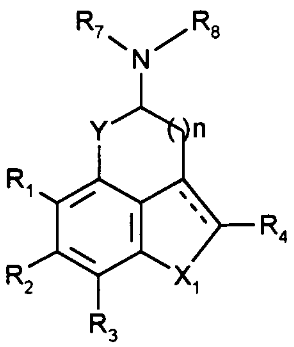

(A1)

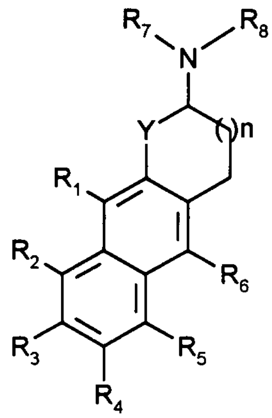

(A2)

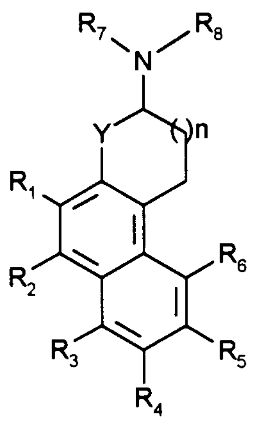

(A3)

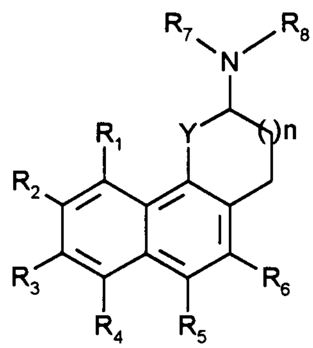

(A4)

dans lesquels :

- $R_1$, $R_2$, $R_3$, $R_4$, $R_5$ et $R_6$ représentent chacun indépendamment l'un de l'autre un hydrogène ou un radical choisi parmi halogène, hydroxy, Ra et -O-Ra ; avec Ra représentant un radical choisi parmi alkyl, alkyl substitué par un ou plusieurs halogènes, alcényl, alcynyl, cycloalkyl, cycloalkylalkyl, aryl, aryl substitué, arylalkyl, et arylalkyl substitué ;
- $X_1$ représente un groupement choisi parmi soufre, oxygène, $-C(R_5)=C(R_6)-$, $-C(R_5)(R_5)-C(R_6)(R_6')-$, $-C(R_5)(R_5')-C(R_6)=$, $-C(R_5)(R_5')-$ et $-N(R_{14})-$ où $R_5$ et $R_6$ sont tels que définis précédemment et $R_5'$ et $R_6'$ sont choisis parmi les mêmes significations que celles de $R_1$, $R_2$, $R_3$, $R_4$, $R_5$ et $R_6$, telles que définies précédemment, et $R_{14}$ représente un hydrogène ou un radical choisi parmi alkyl, aryl, arylalkyl, arylsubstitué et arylalkylsubstitué,
- la liaison --- signifie que cette liaison peut être simple ou double,
- Y représente un groupement $-C(R_9)(R_{10})-$ dans lequel $R_9$ et $R_{10}$ représentent, chacun indépendamment l'un de l'autre un hydrogène, un alkyl ou un alkoxy,
- n représente un nombre entier de 1 à 3 ;
- $R_7$ représente un hydrogène ou un radical $R_7'$ choisi parmi alkyl, aryl, arylalkyl, aryl substitué et arylalkyl substitué
- et $R_8$ représente :

  . un groupement de formule $-CO-R_{11}$ dans laquelle $R_{11}$ représente un radical $R_{12}$ avec $R_{12}$ représentant un hydrogène ou un radical choisi parmi alkyle, alkyle substitué par un ou plusieurs halogènes, alcényle, alcynyle, cycloalkyl, cycloalkylalkyl, aryl, aryl substitué, arylalkyl, arylalkyl substitué, ou $R_{11}$ représente un radical -NH-$R_{13}$, avec $R_{13}$ représentant un atome d'hydrogène ou un radical choisi parmi alkyl, alkyl substitué par un ou plusieurs halogènes, alcényl, alcynyl, cycloalkyl, cycloalkylalkyl, aryl, aryl substitué, arylalkyl et arylalkyl substitué ;

. ou un groupement de formule -CS-$R_{11}$ dans lequel $R_{11}$ est tel que défini précédemment

avec la réserve que le composé de formule (I) ne peut représenter le N-(4-méthyl-1H-2,3-dihydro-phénalèn-2-yl)acétamide, et que si $X_1$ représente un groupement -NH- ou -N($CH_3$)- alors $R_1$ ne peut pas représenter un hydrogène, un halogène ou un groupement méthoxy,

leurs énantiomères et diastéréoisomères
et leurs sels d'addition à une base pharmaceutiquement acceptable,
étant entendu que :

- les termes "alkyl" et "alkoxy" désignent des radicaux linéaires ou ramifiés de 1 à 6 atomes de carbone,
- les termes "alcényl" et "alcynyl" représentent des radicaux insaturés, linéaires ou ramifiés, de 2 à 6 atomes de carbone,
- le terme "cycloalkyl" désigne un groupement cyclique de 3 à 8 atomes de carbone,
- le terme "aryl" désigne un naphtyl, un phényl ou un pyridyl,
- l'expression "substitué" associée aux termes "aryl" et "arylalkyl" signifie que ces groupements sont substitués par un ou plusieurs radicaux choisis parmi halogène, alkyl, alkyl substitué par un ou plusieurs halogènes, alkoxy et hydroxy.

**2.** Composé selon la revendication 1 qui est le N-(2,3-DIHYDRO-1H-PHENALEN-2-YL)ACETAMIDE.

**3.** Composé selon la revendication 1 qui est le N-(4-METHOXY-2,3-DIHYDRO-1H-PHENALEN-2-YL)ACETAMIDE.

**4.** Composé selon la revendication 1 qui est le N-(4,9-DIMETHOXY-2,3-DIHYDRO-1H-PHENALEN-2-YL)-ACETAMIDE.

**5.** Procédé de préparation des composés de formule ($A_1$) telle que définie dans la formule (I) selon la revendication 1, caractérisé en ce que on procède à la cyclisation d'un composé de formule (II) :

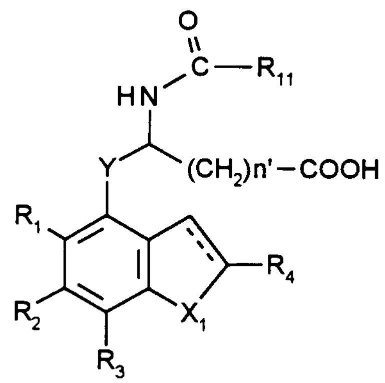

**(II)**

dans laquelle $R_1$, $R_2$, $R_3$, $R_4$, $R_{11}$, $X_1$ et Y sont tels que définis dans la revendication 1, et n' représente 0, 1 ou 2, pour obtenir un composé de formule (III) :

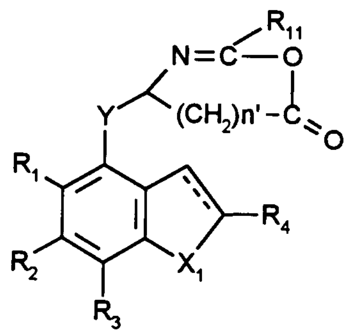

**(III)**

dans laquelle $R_1$, $R_2$, $R_3$, $R_4$, $R_{11}$, n', $X_1$ et Y sont tels que définis précédemment, qui est ensuite mis en réaction avec un acide de Lewis pour obtenir un composé de formule (IV) :

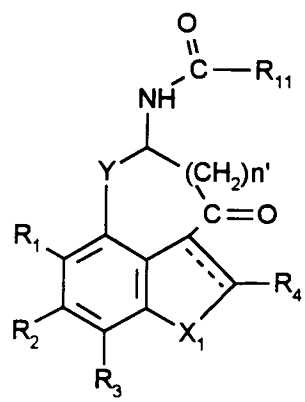

**(IV)**

dans laquelle $R_1$, $R_2$, $R_3$, $R_4$, $R_{11}$, n', $X_1$ et Y sont tels que définis précédemment, qui est alors réduit pour obtenir le composé de formule (I/a) :

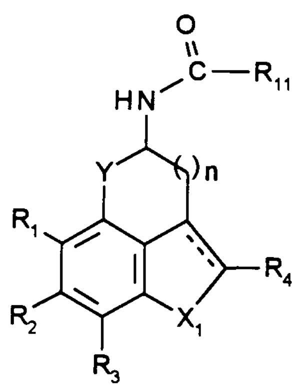

**(I/a)**

dans laquelle $R_1$, $R_2$, $R_3$, $R_4$, $R_{11}$, n, $X_1$ et Y sont tels que définis précédemment et n est tel que défini dans la revendication 1,
composé de formule (I/a) qui est soumis à un agent de thionation, tel que le réactif de Lawesson, pour obtenir le composé de formule (I/b) :

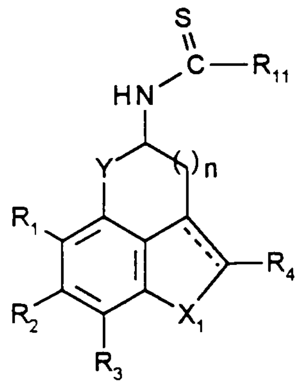

(I/b)

dans laquelle $R_1$, $R_2$, $R_3$, $R_4$, $R_{11}$, n, $X_1$ et Y sont tels que définis précédemment, le composé de formule (I/a) ou (I/b) pouvant être mis en réaction avec un composé de formule (V) :

$$R_7'\text{—Hal} \qquad (V)$$

dans laquelle $R_7'$ est tel que défini dans la revendication 1 et Hal représente un atome d'halogène, pour obtenir le composé correspondant de formule (I/c) :

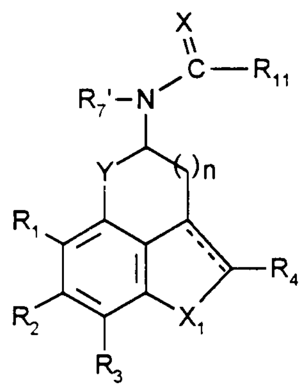

(I/c)

dans laquelle $R_1$, $R_2$, $R_3$, $R_4$, $R_{11}$, $R_7'$, n, $X_1$ et Y sont tels que définis précédemment, et X représente un oxygène ou un soufre, les composés de formule (I) pouvant être :

- purifiés suivant une ou plusieurs méthodes de purification choisies parmi la cristallisation, la chromatographie, l'extraction, la filtration, et le passage sur charbon ou résine,
- séparés, sous forme pure ou sous forme de mélange, en leurs éventuels énantiomères ou diastéréoisomères,
- et salifiés par une base pharmaceutiquement acceptable.

6. Procédé de préparation des composés de formule (I) selon la revendication 1 caractérisé en ce que on fait réagir un composé de formule (g-VI) :

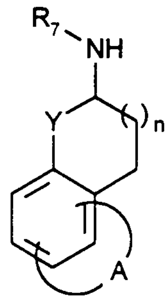

**(g-VI)**

dans laquelle A, $R_7$, n et Y sont tels que définis dans la revendication 1,

- soit avec un composé de formule (VII) ou (VIIa) :

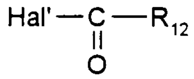

**(VII)**

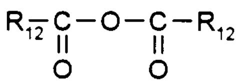

**(VIIa)**

dans laquelle $R_{12}$ est tel que défini dans la revendication 1, l'anhydride de formule (VIIa) étant mixte ou symétrique, et Hal' représente un halogène,
pour obtenir un composé de formule (g-I/e) :

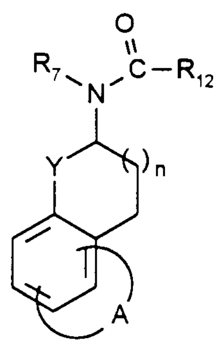

**(g-I/e)**

dans laquelle A, $R_7$, $R_{12}$, n et Y sont tels que définis précédemment,
qui est ensuite soumis à un réactif de thionation tel que le réactif de Lawesson pour obtenir un composé de formule (g-I/e') :

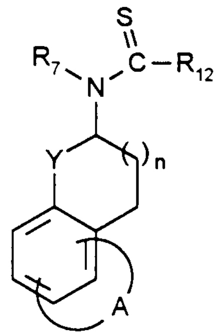

**(g-I/e')**

dans laquelle A, $R_7$, $R_{12}$, Y et n sont tels que définis précédemment,

- soit avec un composé de formule (VIII) :

$$X{=}C{=}N{-}R_{13} \quad \text{(VIII)}$$

dans laquelle $R_{13}$ est tel que défini dans la revendication 1 et X représente un oxygène ou un soufre, pour obtenir un composé de formule (g-I/f) :

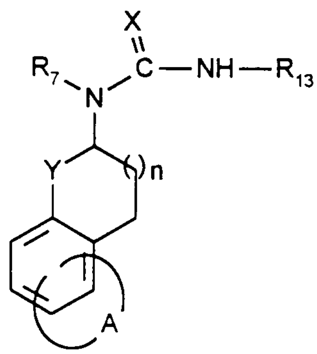

**(g-I/f)**

dans laquelle A, $R_7$, $R_{13}$, n, X et Y sont tels que définis précédemment,
les composés de formule (g-I/e), (g-I/e') et (g-I/f) formant l'ensemble des composés de formule (I),
les composés de formule (I) ainsi obtenus pouvant être :

- purifiés suivant une ou plusieurs méthodes de purification choisies parmi la cristallisation, la chromatographie, l'extraction, la filtration, et le passage sur charbon ou résine,
- séparés, sous forme pure ou sous forme de mélange, en leurs éventuels énantiomères ou diastéréoisomères,
- et salifiés par une base pharmaceutiquement acceptable.

**7.** Procédé de préparation des composés de formule (A/1), cas particulier des composés de formule (I) selon la revendication 1 caractérisé en ce que on fait réagir un composé de formule (VI) :

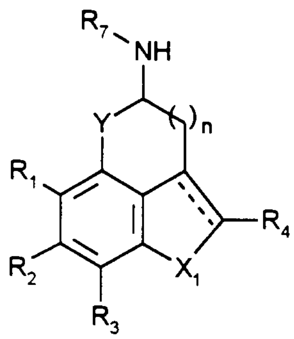

(VI)

dans laquelle $R_1$ $R_2$, $R_3$, $R_4$, $R_7$, n, $X_1$ et Y sont tels que définis dans la revendication 1,

- soit avec un composé de formule (VII) ou (VIIa) :

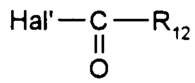

(VII)

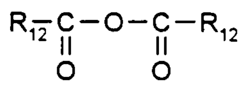

(VIIa)

dans laquelle $R_{12}$ est tel que défini dans la revendication 1, l'anhydride de formule (VIIa) étant mixte ou symétrique, et Hal' représente un halogène,
pour obtenir un composé de formule (I/e) :

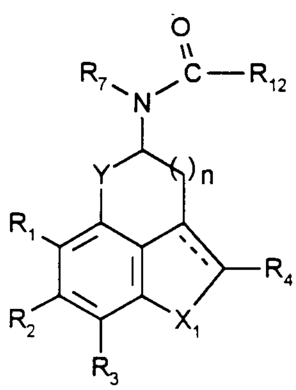

(I/e)

dans laquelle $R_1$, $R_2$, $R_3$, $R_4$, $R_7$, $R_{12}$, n, $X_1$ et Y sont tels que définis précédemment,
qui est ensuite soumis à un réactif de thionation, tel que le réactif de Lawesson, pour obtenir un composé de formule (I/e') :

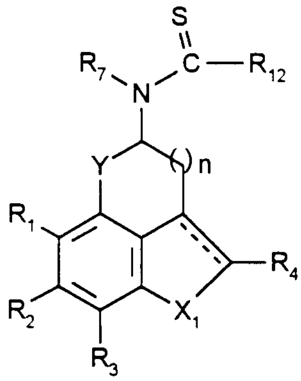

**(I/e')**

dans laquelle $R_1$, $R_2$, $R_3$, $R_4$, $R_7$, $R_{12}$, $X_1$, Y et n sont tels que définis précédemment,

- soit avec un composé de formule (VIII) :

$$X{=}C{=}N{-}R_{13} \qquad (VIII)$$

dans laquelle $R_{13}$, est tel que défini dans la revendication 1 et X représente un oxygène ou un soufre, pour obtenir un composé de formule (I/f) :

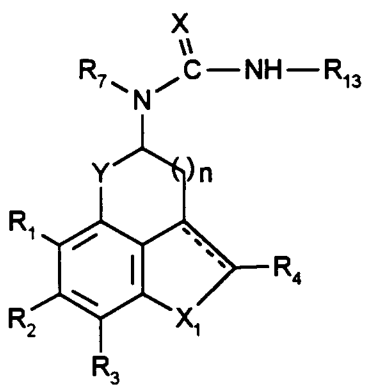

**(I/f)**

dans laquelle $R_1$, $R_2$, $R_3$, $R_4$, $R_7$, $R_{13}$, n, $X_1$, X et Y sont tels que définis précédemment, les composés de formule (I) ainsi obtenus pouvant être :

- purifiés suivant une ou plusieurs méthodes de purification choisies parmi la cristallisation, la chromatographie, l'extraction, la filtration, et le passage sur charbon ou résine,
- séparés, sous forme pure ou sous forme de mélange, en leurs éventuels énantiomères ou diastéréoisomères,
- et salifiés par une base pharmaceutiquement acceptable.

**8.** Procédé de préparation des composés de formule (I/i), cas particulier des composés de formule (I) selon la revendication 1 :

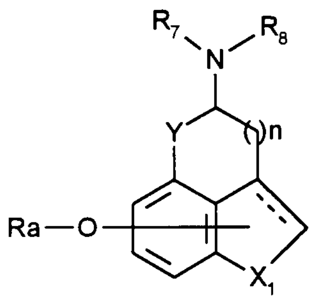

**(I/i)**

dans laquelle $R_7$, $R_8$, Ra, n, $X_1$ et Y sont tels que définis dans la revendication 1 par greffage d'un radical Ra sur un composé de formule (I/j) :

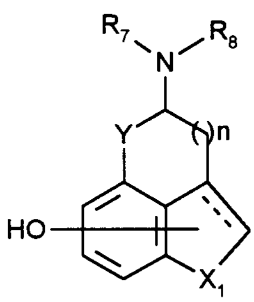

**(I/j)**

dans laquelle $R_7$, $R_8$, $X_1$, Y et n sont tels que définis précédemment,
les composés de formule (I/i) ainsi obtenus pouvant être :

- purifiés suivant une ou plusieurs méthodes de purification choisies parmi la cristallisation, la chromatographie sur colonne de silice, l'extraction, la filtration, et le passage sur charbon ou résine,
- et séparés, sous forme pure ou sous forme de mélange, en leurs éventuels énantiomères ou diastéréoisomères,

**9.** Compositions pharmaceutiques contenant un composé de formule (I) selon la revendication 1 en combinaison avec un ou plusieurs excipients pharmaceutiquement acceptables.

**10.** Compositions selon la revendication 9 pour l'utilisation dans le traitement des troubles du système mélatoninergique et des troubles liés au système mélatoninergique.

**11.** Compositions selon l'une des revendications 9 ou 10 pour l'utilisation dans les traitements des dépressions saisonnières, des troubles du sommeil, des pathologies cardiovasculaires, des insomnies et fatigues dues aux décalages horaires, des troubles de l'appétit et de l'obésité.

## Claims

**1.** Compounds of formula (I):

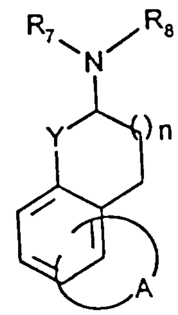

(I)

wherein A forms with the grouping to which it is bonded a tricyclic grouping selected from $(A_1)$ to $(A_4)$:

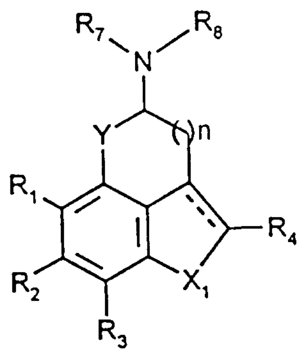

$(A_1)$

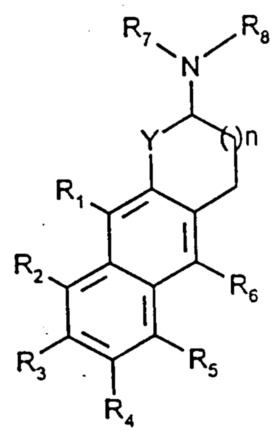

$(A_2)$

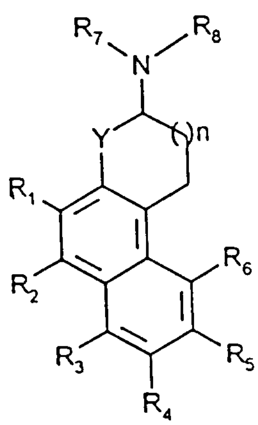

$(A_3)$

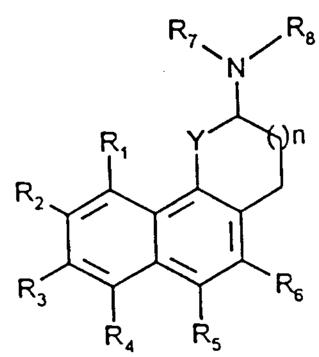

$(A_4)$

wherein:

- $R_1$, $R_2$, $R_3$, $R_4$, $R_5$ and $R_6$ each independently of the others represent a hydrogen atom or a radical selected from halogen, hydroxy, Ra and -O-Ra; with Ra representing a radical selected from alkyl, alkyl substituted by one or more halogen atoms, alkenyl, alkynyl, cycloalkyl, cycloalkylalkyl, aryl, substituted aryl, arylalkyl and substituted arylalkyl;
- $X_1$ represents a grouping selected from sulphur, oxygen, $-C(R_5)=C(R_6)-$, $-C(R_5)(R_5)-C(R_6)(R_6')-$, $-C(R_5)(R_5')-$

$C(R_6)=$, $-C(R_5)(R_5')-$ and $-N(R_{14})-$ wherein $R_5$ and $R_6$ have the definitions given hereinbefore and $R_5'$ and $R_6'$ are selected from the same definitions as those for $R_1$, $R_2$, $R_3$, $R_4$, $R_5$ and $R_6$ as defined hereinbefore, and $R_{14}$ represents a hydrogen atom or a radical selected from alkyl, aryl, arylalkyl, substituted aryl and substituted arylalkyl,

- the bond ---- denotes that the bond may be single or double,
- Y represents a grouping $-C(R_9)(R_{10})-$ wherein $R_9$ and $R_{10}$ each independently of the other represent a hydrogen atom, alkyl or alkoxy,
- n is an integer from 1 to 3;
- $R_7$ represents a hydrogen atom or a radical $R_7'$ selected from alkyl, aryl, arylalkyl, substituted aryl and substituted arylalkyl; and
- $R_8$ represents:

  - a group of formula $-CO-R_{11}$ wherein $R_{11}$ represents a radical $R_{12}$ with $R_{12}$ representing a hydrogen atom or a radical selected from alkyl, alkyl substituted by one or more halogen atoms, alkenyl, alkynyl, cycloalkyl, cycloalkylalkyl, aryl, substituted aryl, arylalkyl and substituted arylalkyl; or $R_{11}$ represents a radical $-NH-R_{13}$ with $R_{13}$ representing a hydrogen atom or a radical selected from alkyl, alkyl substituted by one or more halogen atoms, alkenyl, alkynyl, cycloalkyl, cycloalkylalkyl, aryl, substituted aryl, arylalkyl and substituted arylalkyl;
  - or a group of formula $-CS-R_{11}$ wherein $R_{11}$ is as defined hereinbefore,

with the proviso that the compound of formula (I) cannot represent N-(4-methyl-1H-2,3-dihydro-phenalen-2-yl)acetamide,
and that if $X_1$ represents a group -NH- or $-N(CH_3)-$, $R_1$ cannot represent a hydrogen atom, a halogen atom or a methoxy group,

their enantiomers and diastereoisomers,
and addition salts thereof with a pharmaceutically acceptable base,
it being understood that:

- the terms "alkyl" and "alkoxy" denote linear or branched radicals having from 1 to 6 carbon atoms,
- the terms "alkenyl" and "alkynyl" represent unsaturated, linear or branched radicals having from 2 to 6 carbon atoms,
- the term "cycloalkyl" denotes a cyclic group having from 3 to 8 carbon atoms,
- the term "aryl" denotes naphthyl, phenyl or pyridyl,
- the term "substituted" associated with the terms "aryl" and "arylalkyl" denotes that those groups are substituted by one or more radicals selected from halogen, alkyl, alkyl substituted by one or more halogen atoms, alkoxy and hydroxy.

**2.** Compound according to claim 1 which is N-(2,3-dihydro-1H-phenalen-2-yl)acetamide.

**3.** Compound according to claim 1 which is N-(4-methoxy-2,3-dihydro-1H-phenalen-2-yl)acetamide.

**4.** Compound according to claim 1 which is N-(4,9-dimethoxy-2,3-dihydro-1H-phenalen-2-yl)acetamide.

**5.** Process for the preparation of compounds of formula ($A_1$) as defined for formula (I) according to claim 1, characterised in that a compound of formula (II):

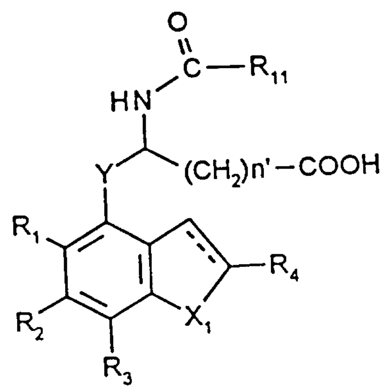

(II)

wherein $R_1$, $R_2$, $R_3$, $R_4$, $R_{11}$, $X_1$ and Y are as defined in claim 1, and n' is 0, 1 or 2, is cyclised to obtain a compound of formula (III):

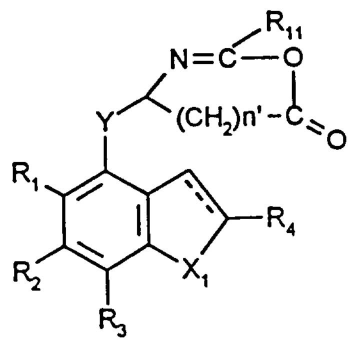

(III)

wherein $R_1$, $R_2$, $R_3$, $R_4$, $R_{11}$, n', $X_1$ and Y are as defined hereinbefore, which is then reacted with a Lewis acid to obtain a compound of formula (IV):

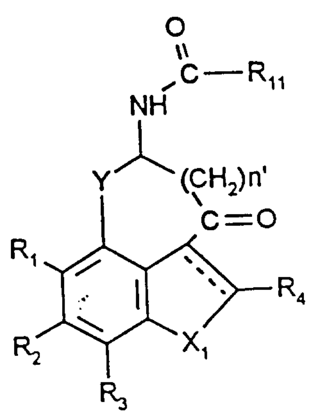

(IV)

wherein $R_1$, $R_2$, $R_3$, $R_4$, $R_{11}$, n', $X_1$ and Y are as defined hereinbefore, which is then reduced to obtain a compound of formula (I/a):

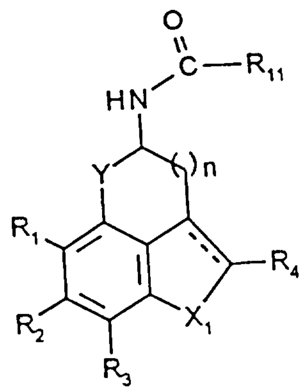

(I/a)

wherein $R_1$, $R_2$, $R_3$, $R_4$, $R_{11}$, n, $X_1$ and Y are as defined hereinbefore and n is as defined in claim 1, which compound of formula (I/a) is subjected to the action of a thionisation agent, such as Lawesson's reagent, to obtain a compound of formula (I/b):

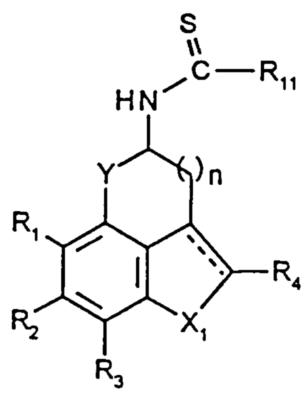

(I/b)

wherein $R_1$, $R_2$, $R_3$, $R_4$, $R_{11}$, n, $X_1$ and Y are as defined hereinbefore,
it being possible for the compound of formula(I/a) or (I/b) to be reacted with a compound of formula (V):

$$R_7'\text{—Hal} \qquad (V)$$

wherein $R_7'$ is as defined in claim 1 and Hal represents a halogen atom,
to obtain the corresponding compound of formula (I/c):

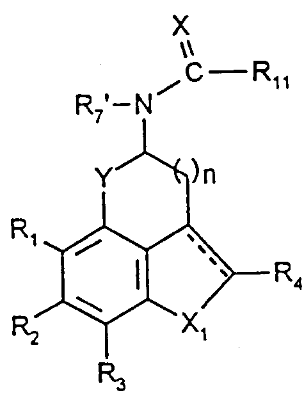

(I/c)

wherein $R_1$, $R_2$, $R_3$, $R_4$, $R_{11}$, $R_7'$, n, $X_1$ and Y are as defined hereinbefore,
and X represents an oxygen or sulphur atom,
wherein the compounds of formula (I) may be:

- purified according to one or more purification methods selected from crystallisation, chromatography, extraction, filtration and passage over charcoal or resin,
- separated, in pure form or in the form of a mixture, into their possible enantiomers or diastereoisomers, and
- converted into a salt by a pharmaceutically acceptable base.

**6.** Process for the preparation of compounds of formula (I) according to claim 1, characterised in that a compound of formula (g-VI):

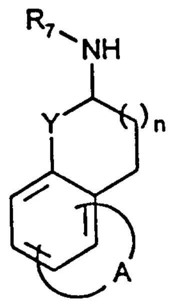

(g-VI)

wherein A, $R_7$, n and Y are as defined in claim 1, is reacted with

- a compound of formula (VII) or (VIIa):

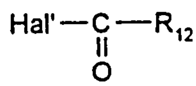

(VII)

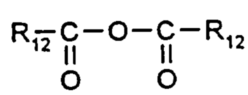

(VIIa)

wherein $R_{12}$ is as defined in claim 1, the anhydride of formula (VIIa) being mixed or symmetrical, and Hal' represents a halogen atom,
to obtain a compound of formula (g-I/e):

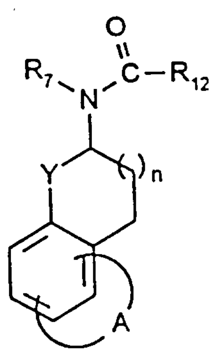

(g-I/e)

wherein A, $R_7$, $R_{12}$, n and Y are as defined hereinbefore,
which is then subjected to the action of a thionisation reagent, such as Lawesson's reagent, to obtain a compound of formula (g-I/e'):

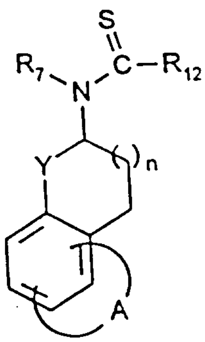

(g-I/e')

wherein A, $R_7$, $R_{12}$, Y and n are as defined hereinbefore,

- or with a compound of formula (VIII):

$$X=C=N—R_{13} \qquad (VIII)$$

wherein $R_{13}$ is as defined in claim 1 and X represents an oxygen or sulphur atom, to obtain a compound of formula (g-I/f):

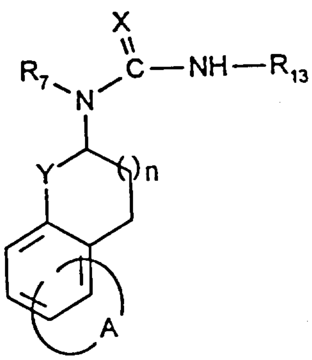

(g-I/f)

wherein A, $R_7$, $R_{13}$, n, X and Y are as defined hereinbefore, the compounds of formulae (g-I/e), (g-I/e') and (g-I/f) constituting the totality of the compounds of formula (I), wherein the compounds of formula (I) so obtained may be:

- purified according to one or more purification methods selected from crystallisation, chromatography, extraction, filtration and passage over charcoal or resin,
- separated, in pure form or in the form of a mixture, into their possible enantiomers or diastereoisomers, and
- converted into a salt by a pharmaceutically acceptable base.

**7.** Process for the preparation of compounds of formula (A/1), a particular case of the compounds of formula (I) according to claim 1, characterised in that a compound of formula (VI):

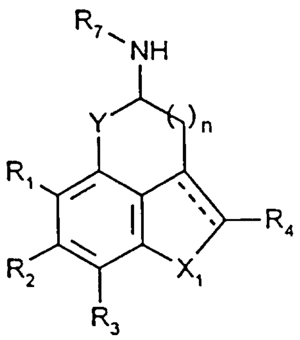

(VI)

wherein $R_1$, $R_2$, $R_3$, $R_4$, $R_7$, n, $X_1$ and Y are as defined in claim 1, is reacted with

- a compound of formula (VII) or (VIIa):

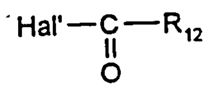

(VII)

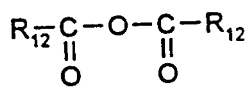

(VIIa)

wherein $R_{12}$ is as defined in claim 1, the anhydride of formula (VIIa) being mixed or symmetrical, and Hal' represents a halogen atom,
to obtain a compound of formula (I/e):

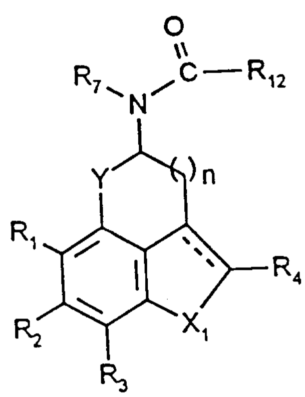

(I/e)

wherein $R_1$, $R_2$, $R_3$, $R_4$, $R_7$, $R_{12}$, n, $X_1$ and Y are as defined hereinbefore,
which is then subjected to the action of a thionisation reagent, such as Lawesson's reagent, to obtain a compound of formula (I/e'):

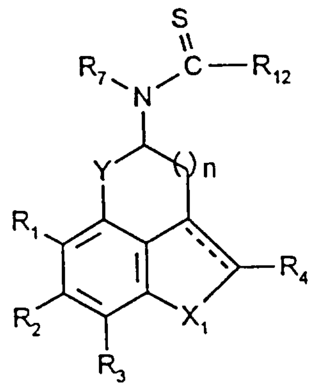

(I/e')

wherein $R_1$, $R_2$, $R_3$, $R_4$, $R_7$, $R_{12}$, $X_1$, Y and n are as defined hereinbefore,

- or with a compound of formula (VIII):

$$X{=}C{=}N{-}R_{13} \quad \text{(VIII)}$$

wherein $R_{13}$ is as defined in claim 1 and X represents an oxygen or sulphur atom, to obtain a compound of formula (I/f):

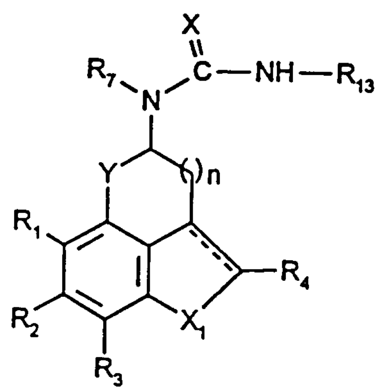

(I/f)

wherein $R_1$, $R_2$, $R_3$, $R_4$, $R_7$, $R_{13}$, n, $X_1$, X and Y are as defined hereinbefore, wherein the compounds of formula (I) so obtained may be:

- purified according to one or more purification methods selected from crystallisation, chromatography, extraction, filtration and passage over charcoal or resin,
- separated, in pure form or in the form of a mixture, into their possible enantiomers or diastereoisomers, and
- converted into a salt by a pharmaceutically acceptable base.

8. Process for the preparation of compounds of formula (I/i), a particular case of the compounds of formula (I) according to claim 1:

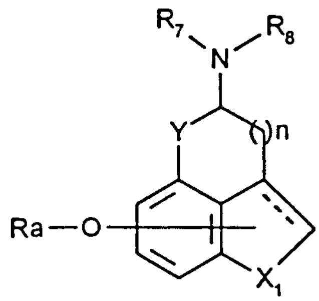

(I/i)

wherein $R_7$, $R_8$, Ra, n, $X_1$ and Y are as defined in claim 1, by the introduction of a radical Ra to a compound of formula (I/j):

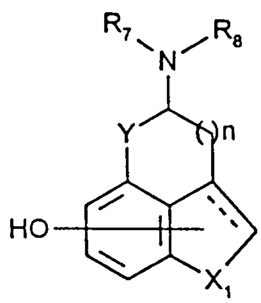

(I/j)

wherein $R_7$, $R_8$, $X_1$, Y and n are as defined hereinbefore,
wherein the compounds of formula (I/i) so obtained may be:

- purified according to one or more purification methods selected from crystallisation, chromatography on a silica column, extraction, filtration and passage over charcoal or resin, and
- separated, in pure form or in the form of a mixture, into their possible enantiomers or diastereoisomers.

**9.** Pharmaceutical compositions comprising a compound of formula (I) according to claim 1 in combination with one or more pharmaceutically acceptable excipients.

**10.** Compositions according to claim 9 for use in the treatment of disorders of the melatoninergic system and of disorders associated with the melatoninergic system.

**11.** Compositions according to claim 9 or claim 10 for use in the treatment of seasonal affective disorder, sleep disorders, cardiovascular pathologies, insomnia and fatigue due to jetlag, appetite disorders and obesity.

**Patentansprüche**

**1.** Verbindungen der Formel (I):

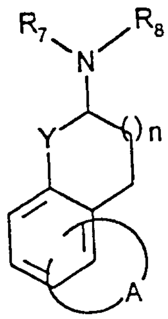

(I)

in der A zusammen mit der Gruppe, an die es gebunden eine tricyclische Gruppe ausgewählt aus ($A_1$) bis ($A_4$):

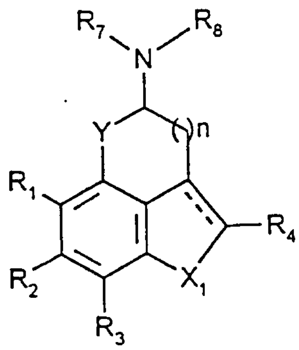

($A_1$)

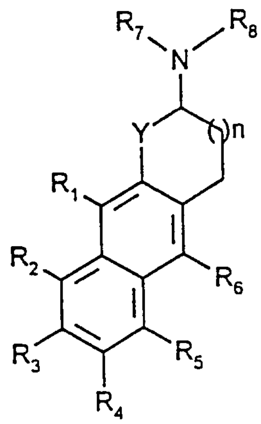

($A_2$)

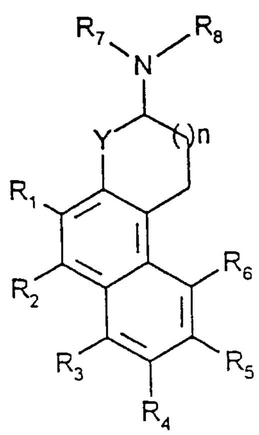

($A_3$)

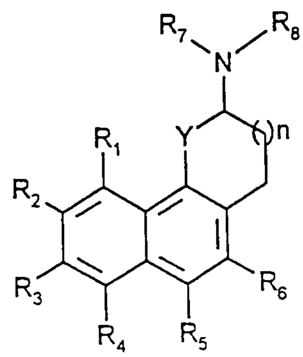

($A_4$)

bildet,
worin:

- $R_1$, $R_2$, $R_3$, $R_4$, $R_5$ und $R_6$ jeweils unabhängig voneinander ein Wasserstoffatom oder eine Gruppe ausgewählt aus Halogen, Hydroxy, Ra und -O-Ra darstellen; worin Ra eine Gruppe ausgewählt aus Alkyl, Alkyl substituiert durch ein oder mehrere Halogene, Alkenyl, Alkinyl, Cycloalkyl, Cycloalkylalkyl, Aryl, substituiertem Aryl, Arylalkyl und substituiertem Arylalkyl darstellen;

- $X_1$ eine Gruppe ausgewählt aus Schwefel, Sauerstoff, $-C(R_5)=C(R_6)-$, $-C(R_5)(R_5)-C(R_6)(R_6')-$, $-C(R_5)(R_5')-C(R_6)=$, $-C(R_5)(R_5')-$ und $-N(R_{14})-$, worin $R_5$ und $R_6$ die oben angegebenen Bedeutungen besitzen und $R_5'$ und $R_6'$ aus den gleichen Bedeutungen ausgewählt sind, wie die Gruppen $R_1$, $R_2$, $R_3$, $R_4$, $R_5$ und $R_6$, wie sie oben definiert worden sind, und $R_{14}$ ein Wasserstoffatom oder eine Gruppe ausgewählt aus Alkyl, Aryl, Arylalkyl, substituiertes Aryl und sustituiertes Arylalkyl darstellt,
- die Bindung ---- bedeutet, daß diese Bindung einfach oder doppelt sein kann,
- Y eine Gruppe $-C(R_9)(R_{10})-$, worin $R_9$ und $R_{10}$ jeweils unabhängig voneinander ein Wasserstoffatom, eine Alkylgruppe oder eine Alkoxygruppe darstellen, bedeutet,
- n eine ganze Zahl mit einem Wert von 1 bis 3 bedeutet;
- $R_7$ ein Wasserstoffatom oder eine Gruppe $R_7'$, ausgewählt aus Alkyl, Aryl, Arylalkyl, substituiertem Aryl und substituiertem Arylalkyl bedeutet und
- $R_8$:
  - eine Gruppe der Formel $-CO-R_{11}$, in der $R_{11}$ eine Gruppe $R_{12}$ darstellt, worin $R_{12}$ ein Wasserstoffatom oder eine Gruppe ausgewählt aus Alkyl, Alkyl substituiert durch ein oder mehrere Halogenatome, Alkenyl, Alkinyl, Cycloalkyl, Cycloalkylalkyl, Aryl, substituiertem Aryl, Arylalkyl, substituiertem Arylalkyl darstellt, oder $R_{11}$ eine Gruppe $-NH-R_{13}$ bedeutet, worin $R_{13}$ ein Wasserstoffatom oder eine Gruppe ausgewählt aus Alkyl, Alkyl substituiert durch ein oder mehrere Halogenatome, Alkenyl, Alkinyl, Cycloalkyl, Cycloalkylalkyl, Aryl, substituiertem Alkyl, Arylalkyl und substituiertem Arylalkyl darstellt;
  - oder eine Gruppe der Formel-$CS-R_{11}$ bedeutet, in der $R_{11}$ die oben angegebenen Bedeutungen besitzt,

mit der Maßgabe, daß die Verbindung der Formel (I) nicht N-(4-Methyl-1H-2,3-dihydro-phenalen-2-yl)acetamid darstellt,
und, wenn $X_1$ eine Grupe -NH- oder $-N(CH_3)-$ darstellt, $R_1$ nicht ein Wasserstoffatom, ein Halogenatom oder eine Methoxygruppe bedeutet,

deren Enantiomere und Diastereoisomere
und deren Additionssalze mit einer pharmazeutisch annehmbaren Base,
mit der Maßgabe, daß:

- die Begriffe "Alkyl" und "Alkoxy" für geradkettige oder verzweigte Gruppen mit 1 bis 6 Kohlenstoffatomen stehen,
- die Begriffe "Alkenyl" und "Alkinyl" für geradkettige oder verzweigte ungesättigte Gruppen mit 2 bis 6 Kohlenstoffatomen stehen,
- der Begriff "Cycloalkyl" für eine cyclische Gruppe mit 3 bis 8 Kohlenstoffatomen steht,
- der Begriff "Aryl" für eine Naphthylgruppe, Phenylgruppe oder Pyridylgruppe steht,
- der Begriff "substituiert", wie er für die Begriffe "Aryl" und "Arylalkyl" benutzt wird, bedeutet, daß diese Gruppen durch eine oder mehrere Gruppen ausgewählt aus Halogen, Alkyl, Alkyl das mit einem oder mehreren Halogenatomen substituiert ist, Alkoxy und Hydroxy substituiert sind.

2. Verbindung nach Anspruch 1, nämlich N-(2,3-DIHYDRO-1H-PHENALEN-2-YL)-ACETAMID.

3. Verbindung nach Anspruch 1, nämlich N-(4-METHOXY-2,3-DIHYDRO-1H-PHENALEN-2-YL)-ACETAMID.

4. Verbindung nach Anspruch 1, nämlich N-(4,9-DIMETHOXY-2,3-DIHYDRO-1H-PHENALEN-2-YL)-ACETAMID.

5. Verfahren zur Herstellung der Verbindungen der Formel ($A_1$), wie sie bezüglich der Formel (I) in Anspruch 1 definiert sind, dadurch gekennzeichnet, daß man eine Cyclisierung einer Verbindung der Formel (II):

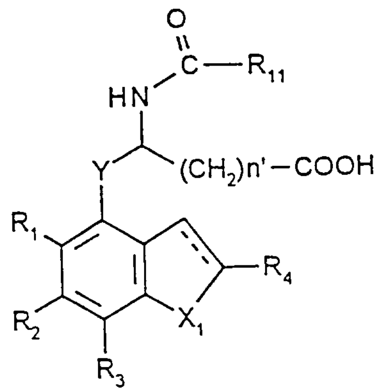

(II)

in der $R_1$, $R_2$, $R_3$, $R_4$, $R_{11}$, $X_1$ und Y die in Anspruch 1 angegebenen Bedeutungen besitzen und n' 0, 1 oder 2 darstellt, bewirkt zur Bildung einer Verbindung der Formel (III):

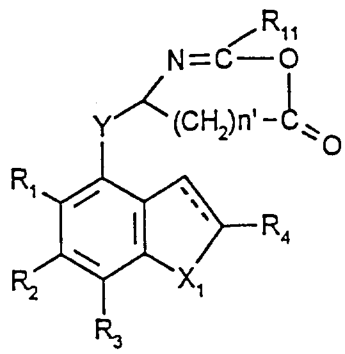

(III)

in der $R_1$, $R_2$, $R_3$, $R_4$, $R_{11}$, n', $X_1$ und Y die oben angegebenen Bedeutungen besitzen,
welche anschließend mit einer Lewis-Säure umgesetzt wird zur Bildung einer Verbindung der Formel (IV):

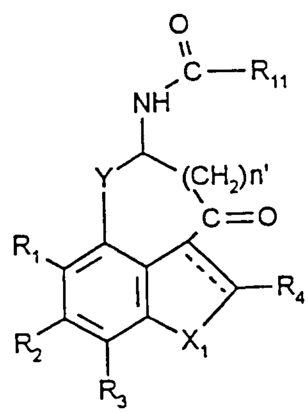

(IV)

in der $R_1$, $R_2$, $R_3$, $R_4$, $R_{11}$, n', $X_1$ und Y die oben angegebenen Bedeutungen besitzen,
welche anschließend reduziert wird zur Bildung der Verbindung der Formel (I/a):

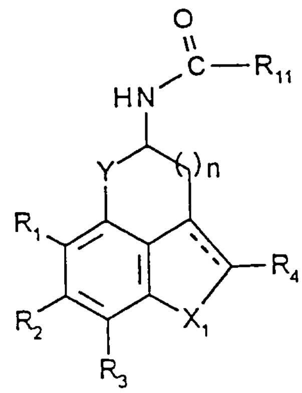

(I/a)

in der $R_1$, $R_2$, $R_3$, $R_4$, $R_{11}$, n, $X_1$ und Y die oben angegebenen Bedeutungen besitzen und n die in Anspruch 1 angegebenen Bedeutungen besitzt,
welche Verbindung der Formel (I/a) mit einem Thionierungsmittel behandelt wird, wie einem Lawesson-Reagens, zur Bildung der Verbindung der Formel (I/b):

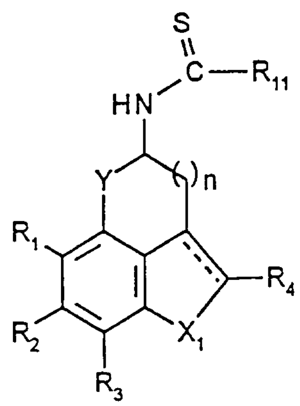

(I/b)

in der $R_1$, $R_2$, $R_3$, $R_4$, $R_{11}$, n, $X_1$ und Y die oben angegebenen Bedeutungen besitzen,
welche Verbindung der Formel (I/a) oder (I/b) mit einer Verbindung der Formel (V):

$$R_7'\text{—Hal} \qquad \text{(V)}$$

in der $R_7$' die in Anspruch 1 angegebenen Bedeutungen besitzt und Hal ein Halogenatom darstellt, umgesetzt werden kann
zur Bildung der entsprechenden Verbindung der Formel (I/c):

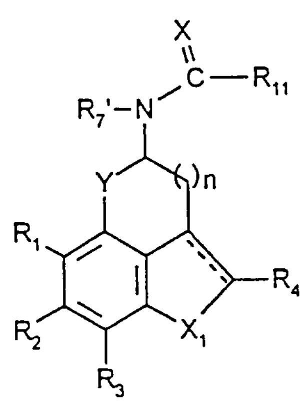

(I/c)

in der $R_1$, $R_2$, $R_3$, $R_4$, $R_{11}$, $R_7'$, n, $X_1$ und Y die oben angegebenen Bedeutungen besitzen und X Sauerstoff oder Schwefel bedeutet,
welche Verbindungen der Formel (I):

- mit Hilfe eines oder mehrerer Reinigungsverfahren, ausgewählt aus Kristallisation, Chromatographite, Extraktion, Filtration und Überführung über Aktivkohle oder ein Harz gereinigt werden können,
- in ihre eventuellen Enantiomeren oder Diastereoisomeren in reiner Form oder in Form einer Mischung getrennt werden können und
- mit einer pharmazeutisch annehmbaren Base in die Salze überführt werden können.

**6.** Verfahren zur Herstellung der Verbindungen der Formel (I) nach Anspruch 1, dadurch gekennzeichnet, daß man eine Verbindung der Formel (g-VI):

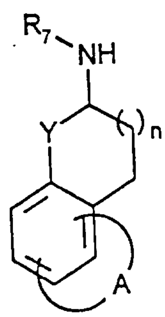

(g-VI)

in der A, $R_7$, n und Y die in Anspruch 1 angegebenen Bedeutungen besitzen,

- entweder mit einer Verbindung der Formel (VII) oder (VIIa):

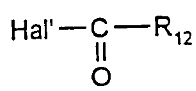

(VII)

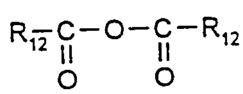

(VIIa)

worin $R_{12}$ die in Anspruch 1 angegebenen Bedeutungen besitzt und wobei das Anhydrid der Formel (VIIa) ein gemischtes oder symmetrisches Anhydrid sein kann und Hal' ein Halogenatom darstellt,
umsetzt zur Bildung einer Verbindung der Formel (g-I/e):

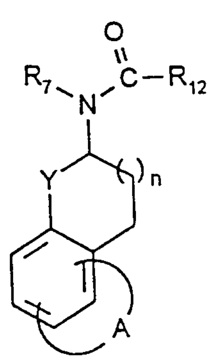

(g-I/e)

ind er A, $R_7$, $R_{12}$, n und Y die oben angegebenen Bedeutungen besitzen,
welches anschließend mit einem Thionierungsreagens behandelt wird, wie dem Lawesson-Reagens, zur Bildung einer Verbindung der Formel (g-I/e'):

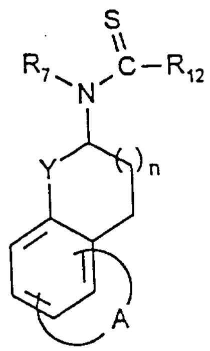

**(g-I/e')**

in der A, $R_7$, $R_{12}$, Y und n die oben angegebenen Bedeutungen besitzen,

- oder mit einer Verbindung der Formel (VIII):

$$X = C = N — R_{13} \qquad \text{(VIII)}$$

in der $R_{13}$ die in Anspruch 1 angegebenen Bedeutungen besitzt und X Sauerstoff oder Schwefel bedeutet, umsetzt zur Bildung einer Verbindung der Formel (g-I/f):

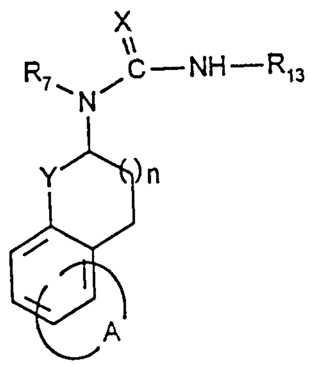

**(g-I/f)**

in der A, $R_7$, $R_{13}$, n, X und Y die oben angegebenen Bedeutungen besitzen,
welche Verbindungen der Formeln (g-I/e), (g-I/e') und (g-I/f) gemeinsam die Verbindungen der Formel (I) bilden, welche in dieser Weise erhaltenen Verbindungen der Formel (I):

- mit Hilfe eines oder mehrerer Reinigungsverfahren, ausgewählt aus Kristallisation, Chromatographie, Extraktion, Filtration und Überführung über Aktivkohle oder ein Harz gereinigt werden können,
- in ihre eventuellen Enantiomeren oder Diastereoisomeren in reiner Form oder in Form einer Mischung getrennt werden können und
- mit einer pharmazeutisch annehmbaren Base in ihre Salze überführt werden können.

**7.** Verfahren zur Herstellung der Verbindungen der Formel (A/1), einem Sonderfall der Verbindungen der Formel (I) nach Anspruch 1, dadurch gekennzeichnet, daß man eine Verbindung der Formel (VI):

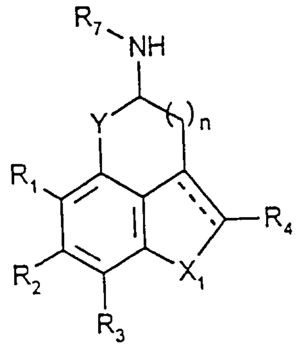

(VI)

in der $R_1$, $R_2$, $R_3$, $R_4$, $R_7$, n, $X_1$ und Y die in Anspruch 1 angegebenen Bedeutungen besitzen,

- entweder mit einer Verbindung der Formel (VII) oder (VIIa):

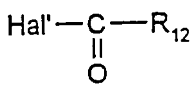

(VII)

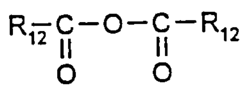

(VIIa)

In der $R_{12}$ die in Anspruch 1 angegebenen Bedeutungen besitzt, wobei das Anhydrid der Formel (VIIa) gemischt oder symmetrisch sein kann und Hal' ein Halogen bedeutet, umsetzt
zur Bildung einer Verbindung der Formel (I/e):

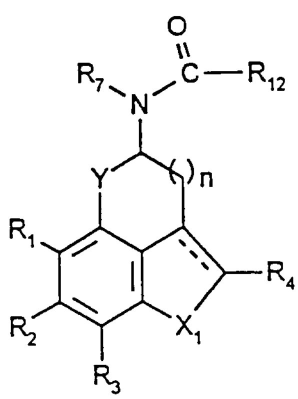

(I/e)

in der $R_1$, $R_2$, $R_3$, $R_4$, $R_7$, $R_{12}$, n, $X_1$ und Y die oben angegebenen Bedeutungen besitzen,
welche anschließend mit einem Thionierungsreagens, wie dem Lawesson-Reagens, behandelt wird zur Bildung einer Verbindung der Formel (I/e'):

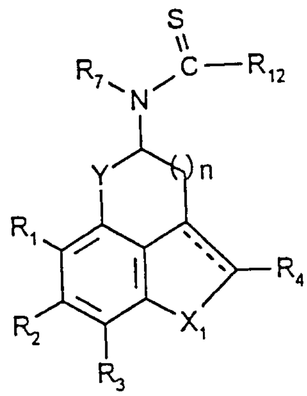

(I/e')

in der $R_1$, $R_2$, $R_3$, $R_4$, $R_7$, $R_{12}$, $X_1$, Y und n die oben angegebenen Bedeutungen besitzen,

- oder mit einer Verbindung der Formel (VIII):

$$X = C = N - R_{13} \qquad \text{(VIII)}$$

in der $R_{13}$ die in Anspruch 1 angegebenen Bedeutungen besitzt und X Sauerstoff oder Schwefel bedeutet, umsetzt zur Bildung einer Verbindung der Formel (I/f):

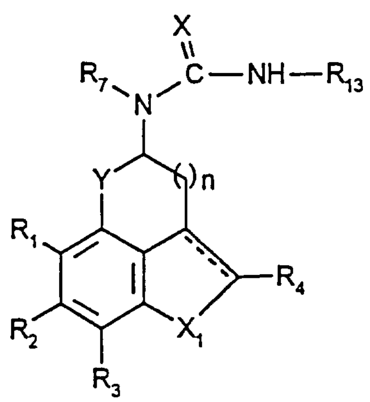

(I/f)

in der $R_1$, $R_2$, $R_3$, $R_4$, $R_7$, $R_{13}$, n, $X_1$, X und Y die oben angegebenen Bedeutungen besitzen, welche in dieser Weise erhaltenen Verbindungen der Formel (I):

- mit Hilfe eines oder mehrerer Reinigungsverfahren, ausgewählt aus Kristallisation, Chromatographie, Extraktion, Filtration und Überführung über Aktivkohle oder ein Harz gereinigt werden können,
- in ihre eventuellen Enantiomeren oder Diastereoisomeren in reiner Form oder in Form einer Mischung getrennt werden können und
- mit einer pharmazeutisch annehmbaren Base in ihre Salze überführt werden können.

**8.** Verfahren zur Herstellung der Verbindungen der Formel (I/i), einem Sonderfall der Verbindungen der Formel (I) nach Anspruch 1:

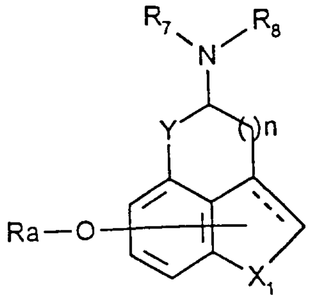

:

(I/i)

in der $R_7$, $R_8$, Ra, n, $X_1$ und Y die in Anspruch 1 angegebenen Bedeutungen besitzen, durch Aufpfropfen einer Gruppe Ra auf eine Verbindung der Formel (I/j):

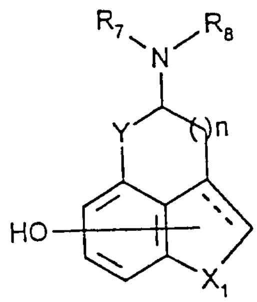

(I/j)

in der $R_7$, $R_8$, $X_1$, Y und n die oben angegebenen Bedeutungen besitzen,
welche in dieser Weise erhaltenen Verbindungen der Formel (I/i):

- mit Hilfe eines oder mehrerer Reinigungsverfahren, ausgewählt aus Kristallisation, Chromatographie über einer mit Siliciumdioxid beschicktem Säule, Extraktion, Filtration und Überführung über Aktivkohle oder ein Harz gereinigt werden können und
- in ihre eventuellen Enantiomeren oder Diastereoisomeren in reiner Form oder in Form einer Mischung getrennt werden können.

**9.** Pharmazeutische Zubereitungen enthaltend eine Verbindung der Formel (I) nach Anspruch 1 in Kombination mit einem oder mehreren pharmazeutisch annehmbaren Trägermaterialien.

**10.** Zubereitungen nach Anspruch 9 zur Verwendung bei der Behandlung von Störungen des melatoninergischen Systems und von Störungen, die mit dem melatoninergischen System verknüpft sind.

**11.** Zubereitungen nach einem der Ansprüche 9 oder 10 zur Verwendung bei der Behandlung von jahreszeitabhängigen Depressionen, Schlafstörungen, kardiovaskulären pathologischen Zuständen, Schlaflosigkeit und Müdigkeit als Folge von Zeitverschiebungen, Appetitstörungen und Fettsucht.